(19) Europäisches Patentamt
European Patent Office
Office européen des brevets

(11) EP 2 746 283 A1

(12) EUROPEAN PATENT APPLICATION
published in accordance with Art. 153(4) EPC

(43) Date of publication:
25.06.2014 Bulletin 2014/26

(21) Application number: 11867338.3

(22) Date of filing: 21.12.2011

(51) Int Cl.:
C07D 487/04 (2006.01)
A61K 31/5377 (2006.01)
A61K 31/541 (2006.01)
A61P 35/02 (2006.01)
A61K 31/519 (2006.01)
A61K 31/551 (2006.01)
A61P 35/00 (2006.01)

(86) International application number:
PCT/CN2011/002152

(87) International publication number:
WO 2012/167415 (13.12.2012 Gazette 2012/50)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 10.06.2011 CN 201110156399

(71) Applicant: Guangzhou Institute Of Biomedicine And Health,
Chinese Academy Of Sciences
Guangzhou, Guangdong 510530 (CN)

(72) Inventors:
- DING, Ke
Guangzhou
Guangdong 510530 (CN)
- CHANG, Shaohua
Guangzhou
Guangdong 510530 (CN)
- XU, Shilin
Guangzhou
Guangdong 510530 (CN)
- ZHANG, Lianwen
Guangzhou
Guangdong 510530 (CN)
- TU, Zhengchao
Guangzhou
Guangdong 510530 (CN)
- DING, Jian
Shanghai 201203 (CN)
- GENG, Meiyu
Shanghai 201203 (CN)
- CHEN, Yi
Shanghai 201203 (CN)

(74) Representative: Chapman, Paul William et al
Kilburn & Strode LLP
20 Red Lion Street
London
WC1R 4PJ (GB)

# (54) PYRIMIDOPYRIMIDONE DERIVATIVES, PHARMACEUTICAL COMPOSITIONS AND USES THEREOF

(57) A compound of formula (I) or (II) and use of the compound in the preparation of drugs for treating cancer are disclosed. The study shows that the compounds can inhibit the growth of many kinds of tumor cells, can be used for targeting epidermal growth factor receptor (EGFR), and particularly can inhibit tumor cells with single or multiple mutations of EGFR (T790M). Therefore, the compound can be used as EGFR inhibitor to treat cancer and has a relatively large application value.

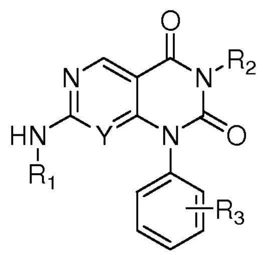

( I )

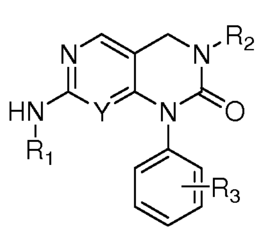

( II )

EP 2 746 283 A1

## Description

### FIELD OF THE INVENTION

The present invention belongs to chemical medicine field, and particularly relates to pyrimido[4,5-d] pyrimidineone compounds, or pharmaceutically acceptable salts, stereoisomers or prodrugs thereof; medical compositions containing the compounds; and use of the compounds and compositions in drug preparations.

### BACKGROUND OF THE INVENTION

Both in the world and China, the chronic diseases (non-infectious diseases) represented by malignancy tumor (cancer), cardiovascular disease and diabetes, are becoming the major long-term threat to human. On 19th May 2008, the world health organization (WHO) in its latest published report explicitly indicated the non-infectious disease was becoming the most lethal human "killer". Among of them, cancer is listed the first killer. In 2004, there were 7.4 million people died from cancer in the world, and the situation in china is more terrible. At the end of April in 2008, it was published in the third national death retrospective survey that the china's urban and rural death rate increased by more 80% in the last three decades, and one out of four or five people died from cancer. Every year the total population who die from cancer is closed to 2 million. Recently, the advance in therapeutic approach and drug offers hope to patients. However, there is still an urgent need to solve the bottleneck problems, such as side effects, poor response, recurrence and metastasis of tumor in traditional treatment. The individual therapy and targeted therapy are considered as the hope to break the bottleneck in lung cancer therapy by international medicine.

Tumor molecular targeted therapy is a therapeutic approach which selectively kills tumor cells through chemical or biological method based on key molecules closely related to tumor growth. The targeted therapy is characterized by high specificity, high selectivity and low toxicity. In addition, when it is used in combination with other drugs, it can strengthen effects of traditional chemotherapy and radiotherapy, and reduce recurrence after recovery. The targeted drugs represented by imatinib (STI571, Novartis, 2001), gefitinib (ZD1839, AstraZeneca, 2003), erlotinib (OSI774, Genentech and OSIP, 2004), sorafenib (Bay 43-9006, Bayer and Onyx, 2005), sunitinib (SU11248, Pfizer, 2006) and dasatinib (BMS-354825, Bristol-Myers Squibb, 2006) opened new age for tumor chemotherapy. Tumor targeted therapy developed rapidly in just a few years, and it had an impact on traditional concept and model of drug delivery. For example, since the targeted drug has low side effect, its dose in clinical I trails always does not reach to the maximum tolerated dose or leads to dosing-limiting toxicity. It is not need to use the maximum tolerated dose to reach the satisfactory effect. Therefore, the tumor targeted therapy is a hot topic and developing trend in tumor therapy.

Protein tyrosine kinases (PTKs) are protein enzymes capable of catalyzing the phosphorylation of phenolic hydroxyl group in tyrosine residues of various key proteins, resulting in activation of protein function. There are about 520 protein kinases in human, half of them are tyrosine kinases. They play an important role in cellular signal transductional pathway, and regulate a series of physiological process, such as cellular growth, differentiation and apoptosis. And dysfunction of protein tyrosine kinases can cause a series of diseases in human body. For instance, their overexpression would disturb the normal cell growth regulation, resulting in tumor. In addition, the abnomal expression of protein tyrosine kinases has been closely associated with tumor invasion, metastasis, angiogenesis and chemotherapy resistance. Therefore, the research of antitumor drug targeting tyrosine kinases has become an international hotspot, and attracts investment from national drug discovery organizations.

Epidermal growth factor receptor (EGFR) is a member of receptor tyrosine kinases, which regulates cell proliferation, survival, adhesion, metastasis and differentiation. Overexpression or constitutive activation of EGFR has been observed in many cancers, such as lung cancer, breast cancer and prostate cancer. EGFR is a kind of transmembrane protein and its family has four members: EGFR, HER-2, HER-3 and HER-4. The abnormal activation of EGFR and HER-2 plays a key role in tumor differentiation and growth, and blocking their activation has been validated in clinical tests as major targeted treatment for tumor cells. Taking lung cancer as an example, EGFR is expressed in 50% of NSCLC patients and is associated with poor prognosis. The two factors allow EGFR and its family members to be major candidate of targeted therapy. Two small molecules targeting EGFR, gefitinib and erlotinib, were rapidly approved for treatment of advanced NSCLC patients, who have not response to traditional chemotherapy.

Early clinical date indicated that 10% of NSCLC patients have response to getifinib and erlotinib. This significant clinical effect has been observed in special patients, including East Asian female non-smokers and carcinoma patients showing bronchioloalveolar pathology. Molecular evaluation showed that in most cases, the patients who have response to the drugs harbor special mutants in EGFR encoding gene. Of note are two particular mutations: deletion of amino acids 747-750 in exon 19 (del(746-750)) and leucine to arginine substitution at 858 in exon 21 (L858R) which together account for approximately 66% of all alterations. The activating mutations in EGFR kinase domain highly activate kinases, inducing an "addiction" of tumor to EGFR survival signal. Calculated prospective clinical studies demonstrated that the patients harboring activating mutations in EGFR have higher response rate than the NSCLC patients with wild type

EGFR, and their PFS and OS significantly prolong. But even so, the PFS of most patients with activating mutations is not more than 12-14 months, in other word, these patients suffer from drug resistance to TKI. And the mechanism of acquired drug resistance and its clinical coping strategies have become another research hotspot.

The drug resistance mechanisms of targeting EGFR inhibitor can be classified into two categories: resistance mutations and alternative signaling pathway. Drug resistance mechanism 1: A secondary EGFR mutation, T790M, is a point mutant in exon 20, and is thought to one of recognized drug resistance mechanisms. The exact resistance mechanism remains to be determined. It was first predicted that the T790M mutation sterically hindered the binding of TKIs to the EGFR kinase domain, by the introduction of a bulky Met residue, thus resulting in drug resistance. A recent report, however, directly showed that L858R/T790M mutant binds ATP more tightly than the L858R mutant and TKIs are ATP-competitive inhibitors, thus causing the binding affinity between TKIs and kinase domain decreased. Another one of controversial issue about T790M is whether this mutant is primary or acquired after treatment of TKIs. The T790M was first found in NSCLC patients who failed to response to TKIs, but then, it was identified in samples that did not receive any treatment. Therefore, it is now thought that this mutation also exists in tumor tissue that does not receive treatment of TKIs, but only in few cell clones, which were identified after treatment due to their resistance to TKIs. There are several drug resistance mutations that have similarities to T790M, such as D761Y, L747S, T854A, etc. These mutations are called "non-T790M acquired mutations", which account for less 5% of the total. Drug resistance mechanism 2: Amplification of MET is another EGFR-TKI acquired resistance that was identified in 2007. MET is a kind of transmembrane tyrosine kinase receptor. There is about 20% wild type MET gene amplification in EGFR mutant positive NSCLC patients who were resistance to TKIs, and most of whom did not harbor MET amplification before treatment. MET, together with ErbB family members, bypasses EGFR downstream signaling pathway mediated by AKT to boost tumor cell growth and inhibit cell apoptosis. In vivo trials, inhibiting MET signaling pathway through siRNA technology can recovery the sensitivity of drug-resistant patients to gefitinib. Combined inhibition of EGFR and MET is able to overcome TKI drug resistance induced by MET amplification. There are still several receptors that have similar effects to MET. A recent in vivo TKI resistance model showed that IGF-1R also can bypass EGFR to activate its downstream signaling pathway. However, for technique hindrance, it is difficult to detect activating IGF-1R in patients' samples. These drug resistance mechanisms through bypassing EGFR and its downstream signaling pathway are called "alternative signaling pathway". The drug resistance mechanism of about 30-40% of the EGFR mutation positive and TKI resistant patients who have neither primary mutation nor MET amplification remains to be discovered.

There are three clinical strategies for drug resistance. Strategy 1 is to continue adopting the cross-use of EGFR TKI, gefitinb and erlotinib. Despite of certain effect, the effect of continue use of TKI is limited. Strategy 2 is to discovery novel EGFR-TKI. Clinical study showed that EGFR irreversible inhibitors can in vivo inhibit T790M. Then, many EGFR irreversible inhibitors were discovered, called "second-generation EGFR TKI". To date, some of irreversible inhibitors have progressed into clinic from pre-clinic, such as neratinib, XL647, BIBW2992 and PF-00299804. Neratinib is a pan ErbB (EGFR, ErbB and ErbB3) irreversible inhibitor, which are under clinical trials. Based on clinical I study, it was studied to reveal whether neratinib (240 mg/d) can overcome T790M mutation or MET amplification-medicated TKI resistance in NSCLC patients after treatment of gefitinib or erlotinib. However, adverse results were showed in some clinical studies. For example, a PC-9 cell line with deletion of EGFR 19 exon developed drug resistance when treated with neratinib; In cell harboring L858R/T790M xenograft mouse model, single treatment of neratinib did not mitigate tumor growth. Therefore, the effect of neratinib on T790M patients remains to be determined. XL647 can irreversibly inhibit EGFR, HER2, VEGFR-2 and EphB4 and suppress the tumor growth in cell harboring L858R/T790M xenograft mouse model. In 2008, a clinical II study of XL647 demonstrated that there was only one patient achieving remission after treatment of XL647 (300 mg/d) in 34 NSCLC patients, who progressed again or harbored T790M mutation after tumor's remission for more than 3 months when treated with gefitinib or erlotinib. This patient was non-smoker, had deletion of 19 exon in EGFR and there is not T790M mutation in the blood of this patient, but no one with T790M mutation received remission and most patients progressed in 2 months. BIBW2992 is a dual EGFR and ErbB2 irreversible inhibitor. Clinical II study showed that BIBW2992 can mitigate tumor in patients with deletion of 19 exon, L858R, L861Q, or G719S/S768I. BIBW2992 is used to treat the patients who have received remission after treatment of gefitinib or erlotinib and failed to response to third-in-class chemotherapy, which is under clinical III study, and a study is conducted in randomized clinical IIb/III trials on BIBW2992 compared with placebo in these patients. These studies would help researchers to determine whether BIBW2992 is good for gefitinib or erlotinib resistant patients. PF-00299804 is a pan ErbB inhibitor, and a patient with T790M mutation received remission treated with it in a clinical I trial. A clinical II study is being conducted, in which PF-00299804 (45 mg/d) is used to treat the NSCLC patients with wild type KRAS who failed to chemotherapy or erlotinib. Strategy 3 is therapy for other targets. Since "alternative signaling pathway" plays a vital role in EGFR-TKI resistance, targeted drugs for these pathways are constantly emerging. MET-TKI probably plays a role in patient with MET amplification. Clinical study showed that the combination of EGFR-TKI and MET-TKI had effects on the cell line with both EGFR mutation and MET amplification, but single use was efficacious. Most important, there are about half of patients harboring both MET amplification and EGFR T790M mutation, then, MET-TKI probably need to be used in combination with T790M inhibitors. XL84 is a novel TKI, which has inhibition on MET, VEGFR-2 and RET.

Other MET inhibitors, such as ARQ197, PF-2341066 and SGX523 are under development. PF-2341066 is a selective c-MET and ALK TKI, which showed good effects on tumor growth, especially in the patients with ALK-EML4 fusion gene, in clinical I trials. And PF-2341066 is being under clinical II/III trials, which has become a new hotspot in targeted therapy area. Some drugs targeting other alternative signaling pathways, such as IGFR-1R inhibitors and HSP90 inhibitors, are also on the way.

In a word, current EGFR-TKIs do not still relieve clinical stress caused by drug resistance. In addition, most existing drugs are EGFR reversible or irreversible inhibitors based on 4-anilinoquinazolines scaffold, which display poor selectivity over wild type cell, resulting in side effects. Therefore, there is an urgent need to discovery novel structural compounds to overcome resistance and improve selectivity.

## SUMMARY OF THE INVENTION

In one aspect, the present invention provides a novel pyrimido[4,5-d]pyrimidinyl compound of formula (I) or (II):

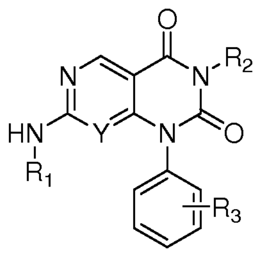

（I）

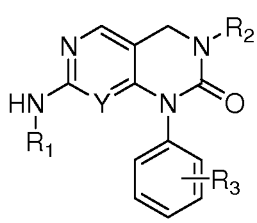

（II）

or a pharmaceutically acceptable salts, isomers or prodrugs thereof, wherein, Y is -CH or N;
$R_1$ is selected from:

1) H;
2) $C_1$-$C_5$alkyl;
3) $C_3$-$C_6$ cycloalkyl;
4) $C_1$-$C_5$ fluoroalkyl;
5) $((CH_2)_nX$, n is an integer of 0-6, X is OH, $NH_2$, $C_1$-$C_6$ heteroalkyl, or $C_3$-$C_7$ heterocycloalkyl;
6)

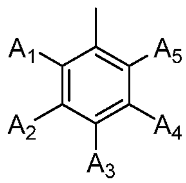

,

$A_1$, $A_2$, $A_3$, $A_4$, $A_5$ are each independently selected from:

a. H;
b. halo;
c. -CN;
d. $-NO_2$;
e. -OH;
f. $-NH_2$;
g. $C_1$-$C_6$ alkyl;
h. $C_3$-$C_6$ cycloalkyl;
i. $C_1$-$C_6$ fluoroalkyl;
j. $C_1$-$C_6$ heteroalkyl;
k. $C_1$-$C_7$ heterocycloalkyl;
1. the esters, amide, sulfone, sulfoxide, urea formed from the alkyl above; wherein, the heteroatom contained in the above heteroalkyl or heterocycloalkyl is O, N or S;

$R_2$ is selected from:

1) H;
2) $C_1$-$C_5$ alkyl;
3) $C_3$-$C_6$ cycloalkyl;
4) $C_1$-$C_5$ fluoroalkyl;
5) aryl;
6) heterocycloalkyl;
wherein, the heteroatom contained in the above heterocycloalkyl is O, N or S;

$R_3$ is selected from:

1) H;
2) halo;
3) $NH_2$, OH, CN, $NO_2$;
4) $C_1$-$C_5$ alkyl;
5) $C_3$-$C_6$ cycloalkyl;
6) aryl;

7) 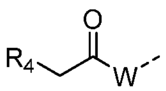 ; 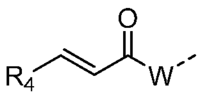 ; 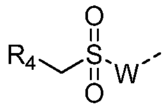 ; 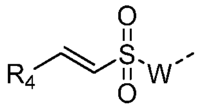 ; 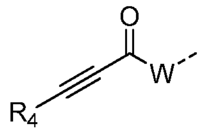 ;

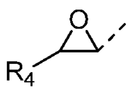 ; 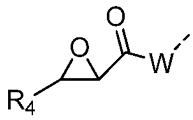 ; 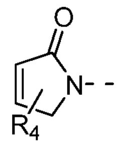 ; 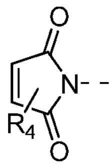 ; 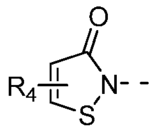 ; 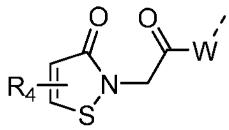 ;

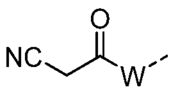 ; 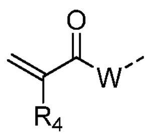 ; 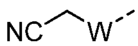 ; 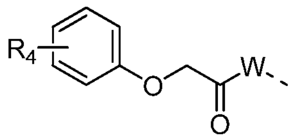 ; 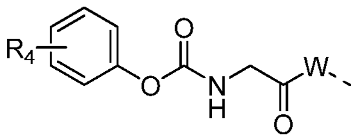 ,

wherein W is: a. $CH_2$; b. $CH_2CH_2$; c. O; d. S; e. NH; f. NR; R is $C_1$-$C_5$ alkyl or aryl;

$R_4$ is selected from:

1) H;
2) halo;
3) $C_1$-$C_5$ alkyl;
4) $C_3$-$C_6$ cycloalkyl;
5) aryl;
wherein, the alkyl, aryl described above are each independently substituted by 0, 1, 2 or 3 substituents selected from $R_5$;

$R_5$ is selected from:

1) H;
2) halo;
3) $C_1$-$C_3$ alkyl;
4) $C_3$-$C_6$ cycloalkyl;
5) $C_1$-$C_3$ alkoxy;
6) $C_1$-$C_3$ fluoroalkyl;

7) heterocycloalkyl;
8) $C_0$-$C_3$ alkylene heterocyclic;
9) phenyl;

wherein, the heteroatom contained in the above heterocycloalkyl or alkylene heterocyclic is O, N or S.

In another aspect, the present invention further provides a compound having the structure of formula (III):

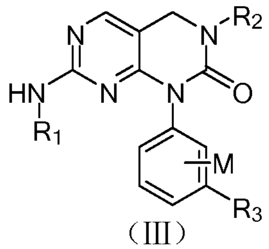

(III)

or the pharmaceutically acceptable salts, isomers or prodrugs thereof,
wherein $R_3$ is selected as above;
$R_1$ is selected from:

1) H;
2) methyl, ethyl, n-propyl, isopropyl, n-butyl, isobutyl, t-butyl, n-pentyl, isopentyl, neopentyl;
3) cyclopropyl, cyclobutyl, cyclopentyl, cyclohexyl;
4) $(CH_2)_nX$, n is an integer of 0-6, X is -OH, $-NH_2$, $-OCH_3$, $-OCH_2CH_3$, $-OCH_2CH_3OCH_3$, $-OCH_2CH_3OCH_2CH_3$, $-SCH_3$, $-N(CH_3)_2$, N-methylpiperazinyl, morpholinyl, thiomorpholinyl, piperdinyl, pyrrolidinyl, 4-N,N-dimethylpiperidinyl, 1-methyl-4-(piperidin-4-yl)piperazinyl, imidazole, 6-(4-methylpiperazine-1-yl)-3-pyridinyl;
5)

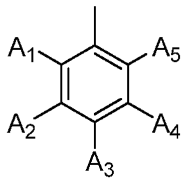

,

$A_1$, $A_2$, $A_3$, $A_4$, $A_5$ are each independently selected from:

a. H;
b. F, Cl, Br, I;
c. methyl, ethyl, n-propyl, isopropyl, n-butyl, isobutyl, t-butyl, n-pentyl, isopentyl, neopentyl;
d. $-OCH_3$, $-OCH_2CH_3$, $-OCH(CH_3)_2$, $-OC(CH_3)_3$, $-OCH_2CH_3OCH_3$, $-OCH_2CH_3OCH_2CH_3$;
e. $-CF_3$;
f. N,N-dimethylaminoethoxyl, N,N-dimethylaminopropoxyl, 2-(N-methylpiperazine)ethoxyl, 2-(N-acetylpiperazine)ethoxyl, 2-morpholinylethoxyl, 2-thiomorpholinylethoxyl, 2-pyridinylethoxyl, 2-pyrrolidinylethoxyl, N-methylpiperazinyl, morpholinyl, thiomorpholinyl, piperdinyl, pyrrolidinyl, imidazole, 3-N,N-dimethylpyrrolidinyl, 4-N,N-dimethylpyridinyl, 4-acetylpiperazinyl, 1-methyl-4-(piperazine-4-yl)pyridinyl, 4-(4-methylpiperazine-1-yl)methyl, (1-methylpiperidine-4-yl)amino, piperazine-2-one-4-yl, 1-methylpiperazine-2-one-4-yl;
g. the esters, amide, sulfone, sulfoxide, urea formed from the groups above;

$R_2$ is selected from:

1) H;
2) methyl, ethyl, n-propyl, isopropyl, n-butyl, isobutyl, t-butyl, n-pentyl, isopentyl, neopentyl;
3) cyclopropyl, cyclobutyl, cyclopentyl, cyclohexyl;
4) phenyl,4-methoxylphenyl, biphenyl, 4-phenoxylphenyl , 4-benzyloxylphenyl, 2,4-dichlorophenyl, 3-chloro-4-fluorophenyl, single or multiple substituted phenyl, benzyl, substituted benzyl, 1-naphthyl, 2-naphthy, pyridinyl;

The 2, 4, 5, 6-position of aromatic ring containing M is mono or multi-substituted by M, wherein M is selected from:

1) H;
2) halo;
3) -CN;
4) $-NO_2$;
5) -OH;
6) $-NH_2$;
7) $C_1$-$C_6$ alkyl;
8) $C_3$-$C_6$ cycloalkyl;
9) $C_1$-$C_6$ fluoroalkyl;
10) $C_1$-$C_6$ heteroalkyl;
11) $C_1$-$C_7$ heterocycloalkyl;
12) the esters, amide, sulfone, sulfoxide, urea formed from the groups above;
wherein, the heteroatom contained in the above heteroalkyl or heterocycloalkyl is O, N or S.

In another aspect, the present invention further provides a compound having the structure of formula (IV):

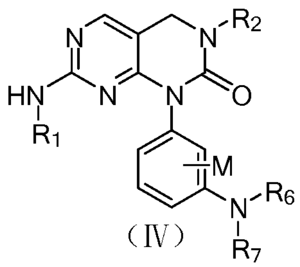

(IV)

or the pharmaceutically acceptable salts, isomers or prodrugs thereof,
wherein $R_3$, $R_2$, M are selected as above;
$R_6$, $R_7$ are each independently selected from:

1) H;
2) methyl, ethyl, n-propyl, isopropyl, n-butyl, isobutyl, t-butyl, n-pentyl, isopentyl, neopentyl;
3) cyclopropyl, cyclobutyl, cyclopentyl, cyclohexyl;
4) phenyl;

5) 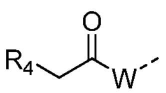 ; 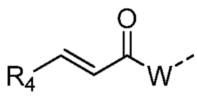 ; 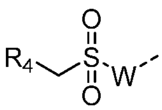 ; 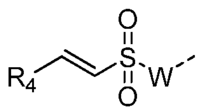 ; 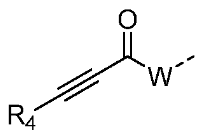 ; 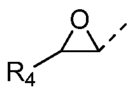 ;

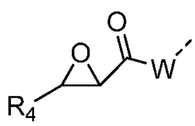 ; 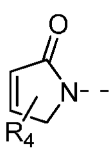 ; 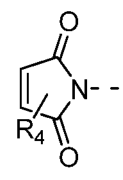 ; 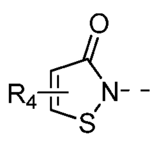 ; 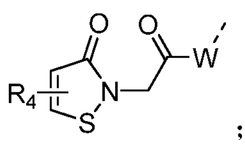 ; 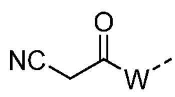 ;

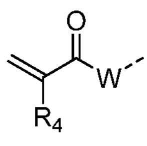 ; 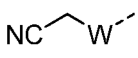 ; 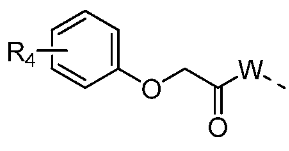 ; 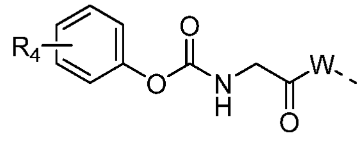 。

W is selected from: $-CH_2$, $-CH_2CH_2$, O, S, -NH, -NR; R is methyl, ethyl, or phenyl;

$R_4$ is selected from:

1) H;
2) F, Cl, Br, I;
3) methyl, ethyl, n-propyl, isopropyl, n-butyl, isobutyl, t-butyl;
4) cyclopropyl, cyclobutyl, cyclopentyl, cyclohexyl;
5) phenyl, mono- or multi- substituted phenyl.

Another objective of the present invention is to provide a pharmaceutical composition that is useful for treating cancer.

According to one embodiment of the present invention, a technical solution for achieving the above mentioned objective is as followings:

A pharmaceutical composition comprises of the above mentioned compound, i.e. 7-(substituted amino)- 3,4-dihydropyrimido[4,5-d]pyrimidin-(1H)-one or the pharmaceutically acceptable salts, prodrugs or stereoisomers thereof.

It is a further objective for the present invention to provide use of the above-mentioned compound.

According to one embodiment of the present invention, a technical solution for achieving the above objective is as followings:

Use of the compound mentioned above or the pharmaceutically acceptable salts, stereoisomers or pro-drugs thereof in the preparation of drugs for treating cancer.

Preferably, the mentioned cancer is any one of non-small cell lung cancer, small cell lung cancer, lung adenocarcinoma, squamous cell lung carcinoma, pancreatic cancer, breast cancer, prostate cancer, liver cancer, skin cancer, squamous cell carcinoma, nasopharyngeal carcinoma, leukemia, histiocytic lymphoma and nasopharyngeal carcinoma.

The 2-oxo-3,4-dihydropyrimido[4,5-d]pyrimidinyl compounds which have the general formula (I) or (II) could inhibit mutiplie cancer cells proliferation. They specially inhibit the proliferation of non-small-cell lung cancer (NSCLC) H1975 which bears $EGFR^{L858R/T790M}$ or $EGFR^{E745_A750/T790M}$. Compered to the cancer cell bearing the wild type EGFR, these compounds can work 10, 100 or 1000 times more effective on mutated cancer cells. This kind of compound is a new series inhibitors that can overcome the gefitinib-resistant nonsmall cell lung cancer.

The above mentioned compounds 7-(substituted amino)-3,4-dihydropyrimido[4,5-d]pyrimidin-(1H)-ones and their pharmaceutically acceptable salts or prodrugs can effectively inhibit mutiplie cancer cells proliferation. They can still inhibit the phosphorylation of EGFR and other members of Her family. These compounds can be used as anticancer drugs, and they can overcome the gefitinib-resistant. As can be understood by the technical person in the filed, the compounds and their pharmaceutically acceptable salts of the present invention can be used for the therapeutic application of over proliferative diseases including human cancers or mammalian cancers.

## BRIEF DESCRIPTION OF THE DRAWINGS

Figs.1-11 illustrate the efficacies using vitro kinase assays with WT EGFR.
Figs. 12-22 illustrate the efficacies using vitro kinase assays with EGFR T790.
Figs. 23-26 illustrate the efficacies agaist lung cancer cells using MTT.
Figs. 27 and 28 illustrate the effect of compound C-EGF06 on the lung cancer cell cycle.
Fig. 29 illustrates the effect of compound C-EGF06 on the lung cancer cell apoptosis.
Fig 30. illustrates the effect of compound C-EGF06 on the lung cancer cell signling pathwey.

## DETAILED DESCERIPTION OF THE INVENTION

In the compounds according to the present invention, when any variables (such as R1, R, etc.) appear more than once in any component, the definitions every time they occur are independent from the definitions they appear other times. Also, allow substituent and variable combination, as long as the combination makes stable compounds. The line crossing from the substituent to sing system means the bond indicated can link to any atom of the ring which can be substituted. If the ring system is multiple ring system, it means the bond only connects to any appropriate carbon atom of the adjacent ring. To understand the person with common techniques in the art can choose the compounds substituent and replacement type in order to provide the synthetic compounds that are chemically stable and can be

synthesized from easily available materials by the techniques in the field and the methods mentioned bellow. If the substituent itself is replaced by more than one group, these groups can be in the same carbon atom or different carbon atoms, as long as the structure is stable. The phrase "optionally substituted by one or more substituents" is equivalent to the phrase "optionally substituted by at least one substituent", and in a preferable embodiment, there will be 0-3 substituents.

In this invention, the term "alkyl" and "sub-alkyl" means a branched-chain or straight chain alkyl group with the certain number of carbon atoms. For example, the definition of "$C_1$-$C_5$" in "$C_1$-$C_5$ alkyl" means straight-chain or branched-chain alkyl group with 1, 2, 3, 4 or 5 carbon atoms. For example, "$C_1$-$C_5$ alkyl" includes methyl, ethyl, n-propyl, isopropyl, n-butyl, tert-butyl, isobutyl, pentyl, etc. The term "cycloalkyl" refers to a specific single saturated ring alkyl with the certain number of carbon atoms. For examples, "cycloalkyl" includes cyclopropyl-, methyl-cyclopropyl-, 2, 2-dimethyl-cyclobutyl, 2-ethyl-cyclopentyl-, cyclohexyl etc.

In this invention, the term of "hetero aryl" is a stable monocyclic ring with up to six atoms or a stable bicyclic ring in which each ring contains up to six atoms. At least one of the rings is an aromatic ring containing 1-4 atoms selected from O, N or S. Hetero aryl groups include but not limit to: imidazolyl, triazolyl, pyrazolyl, furanyl, thienyl, oxazolyl , isoxazolyl, pyrazinyl, pyridazinyl, pyridyl, pyrimidinyl, pyrrolyl. About the definition of hetero aryl, any hetero aryl N-oxidation derivatives containing N atom should also be added. When hetero aryl substitutent group is a bicyclic ring and one of the two rings is non-aromatic or non- heteroatom-containing ring, the bicyclic ring is connected via the aromatic ring or the heteroatom-containing ring.

The term of "heterocycle" or "heterocyclic" refers to an aromatic or nonaromatic ring containing 5 - 6 atoms, in which contains 1-4 hetero atoms such as O, N, S. "Heterocycle" includes hetero aromatic ring as mentioned above; it also includes dihydro and tetrahydro analogs. "Heterocycles" further include but not limit to: imidazolyl, indolyl, isothiazolyl, isoxazolyl, oxadiazolyl, oxazolyl, oxetanyl, pyranyl, pyrazinyl, pyrazolyl, pyridazinyl, pyridyl, pyrimidinyl, pyrrolyl, quinazolinyl, tetrazolyl, thiadiazolyl, thiazolyl, thienyl, triazolyl, 1, 4-alkyl-dioxinyl, alkyl pyrrolidinyl, dihydro-imidazolyl, dihydro-isoxazolyl, dihydro-iso thiazolyl, dihydro-oxadiazolyl, dihydro-oxazolyl, dihydro-pyrazinyl, dihydro-pyrazolyl, dihydro-pyridyl, dihydro-pyrimidinyl, dihydro-pyrrolyl, dihydro-tetrazolyl, dihydro-thiadiazolyl, dihydro-thiazolyl, dihydro-thienyl, dihydro-triazolyl, methylene dioxy-benzophenone acyl , tetrahydrofuranyl, tetrahydrothiopheneyl, and their N-oxides etc. The linkage of heterocycle substituent can be achieved through C atom or heteroatom.In one embodiment, heterocycle is selected from imidazolyl" pyridyl, 1-pyrrolidone, 2-piperidone, 2- pyrimidone, 2-pyrrolidone, thienyl, oxazolyl, triazolyl, isoxazolyl, etc.

As understood by the person skilled in the prior art, "halo" or "halogen" in the present specification means chlorine, fluorine, bromine and iodine.

Unless specially mentioned, alkyl, cycloalkyl, aryl, hetero aryl, heterocyclic groups can be substituted or not be substituted. For example, $C_1$ - $C_6$ alkyl group can be substituted 5 by one, two, or three substitutents selected from OH, halogens, alkoxyl, dialkylamino, or heterocyclic ring such as morpholinyl, piperidinyl groups.

In an embodiment, "Het" is defined as being able to cooperate with N atom which connects the Het to form a 4-7 membered mono-ring or a bicyclic heterocylic ring in which each ring is a 4-7 membered ring, wherein the mono-ring or bicyclic heterocylic ring may further comprises 1-2 hetero atoms selected from N, O, S, and said heterocyclecan also be optionally substituted by one or more substituents selected from $R_2$. The hetero cyclic rings formed include but not limit to the following heterocycles, and it shall be remembered said heterocycle selectively substituted by one or more(preferably one, two or three) substituents selected from $R_2$:

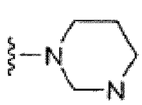 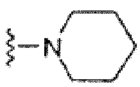 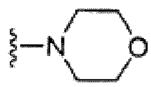 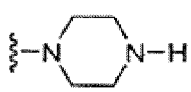

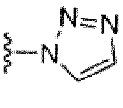 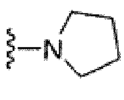 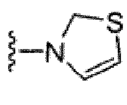 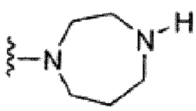

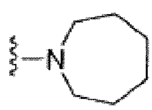 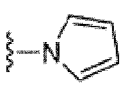 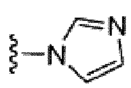 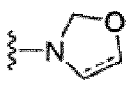 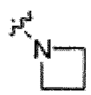

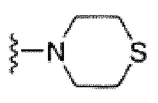 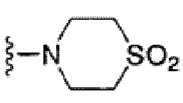 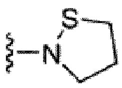 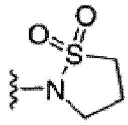

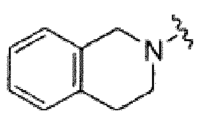 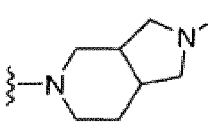 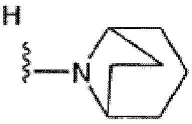 .

The present invention relates to the free form of compounds with formula (I)-(II), and it also relates to their pharmaceutically acceptable salts or steroisomers. The specifc examples in the invention are the protonated salts of amines. The term "free form" means that the amines are not in the form of salts. The included pharmaceutically acceptable salts include not only the exemplary salts of the specific compounds of the present disclosure, but also the typical pharmaceutically acceptable salts of all the compounds of formulas I-IV in free form. The specific compounds in free form can be separated by means of known technology in the art. For example, appropriate dilute aqueous solutions of alkali, such as dilute aqueous solution of NaOH, dilute aqueous solution of $K_2CO_3$, dilute ammonia, dilute aqueous solution of $NaHCO_3$, etc., can be used for treating the salts to make the free form regenerate. A compound in free form has some different properties with such compound in its salt form, for example, their respective solubilities in a polar solvent are different; however, the acid salts and basic salts according to the present invention are equivalent to their respective free form in other pharmaceutical aspects.

The pharmaceutically acceptable salts according to the present invention can be synthesized from the compound of the present invention containing a basic portion or an acidic portion by conventional chemical methods. Usually, a salt of a basic compound is prepared by ion exchange chromatography or by reacting a free-form base with stoichiometric amount or excessive amount of an inorganic or organic acid according to the required salt form in an appropriate solvent or combination of a variety of solvents. Similarly, a salt of an acidic compound is prepared by reacting the compound with an appropriate inorganic or organic base.

Therefore, "pharmaceutically acceptable salts" in the invention mean the normal nontoxic salts formed by the basic compounds in the invention with organic acids and inorganic acids. For example, the normal nontoxic salts are prepared from inorganic acids that include hydrochloric acid, hydrobromic acid, sulfuric acid, sulfamic acid, phosphoric acid, nitric acid, and from organic acids that include acetic acid, propionic acid, succinic acid, glycolic acid, stearic acid, lactic acid, malic acid, tartaric acid, lemon acid, ascorbic acid, bashing acid, maleic acid, hydroxy-maleic acid, phenylacetic acid, glutamic acid, benzoic acid, salicylic acid, sulfanilic acid, 2-acetoxy-benzoic acid , p-toluenesulfonic acid, methanesulfonic acid, ethane disulfonic, oxalic acid, hydroxyethyl sulfonic acid, trifluoroacetic acid etc.

When the compound of the present invention is acidic, then the appropriate "pharmaceutically acceptable salts" refer to salts prepared from pharmaceutically acceptable nontoxic bases including inorganic and organic bases. The salts prepared from inorganic bases includes aluminum salts, ammonium salts, calcium salts, copper salts, ferric salts, ferrous salts, lithium salts, magnesium salts, manganic salts, manganous salts, potassium salts, sodium salts, zinc salts, etc. Particulary, ammonium salts, calcium salts, magnesium salts, potassium salts and sodium salts are preferable. The salts prepared from organic nontoxic bases includes salts of primary amines, secondary amines and tertiary amines, wherein the amines can be substituted amines including naturally occurring substituted amines, cyclic amines and basic ion exchange resins such as arginine, betaine, caffeine, choline, N, N'- dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucamine, glucosamine, histidine, hydroxocobalamin, isopropylamine, lysine, methylglucamine, morpholine, piperazine, piperidine, polyamine resins, procaine, purines, theobromine, triethylamine, trimethylamine, tripropylamine, tromethamine, etc.

Berg et al described the preparation of pharmaceutically acceptable salts as above mentioned or other typical pharmaceutically acceptable salts in Pharmaceutical Salts, J. Pharm. Sci. 1977, 66: 1-19 in more details.

Since under physiological conditions, a deprotonated acidic moiety of a compound, e.g. a carboxyl, may be of anion with charge which can be balanced/offset by a protonated or alkylated basic moiety with cation contained therein, for example, a tetravalent nitrogen atom, therefore, it should be noted that compounds of the present invention are potentially internal salts or zwitter.

In addition to the known in the literature or exemplified in the experimental procedures in the standard methods, the compounds metioned in this invention can be prepared in the same way with the following program. Therefore, the following program is just used for illustrative. It is not limited to the listed compounds or any particular substituent. The number of substituents showed in this program is not required to match the number that used in the claim, and for the

purpose of clarity, mono substituent is shown to be connected to the compound of formula (I) or (II) which is capable of having multi substituents as defined above.

Program

The compounds I-IV mentioned in the invention could be synthesized in nine steps by using ethyl 4-chloro-2-(methylthio)pyrimidine-5-carboxylate as the starting material.

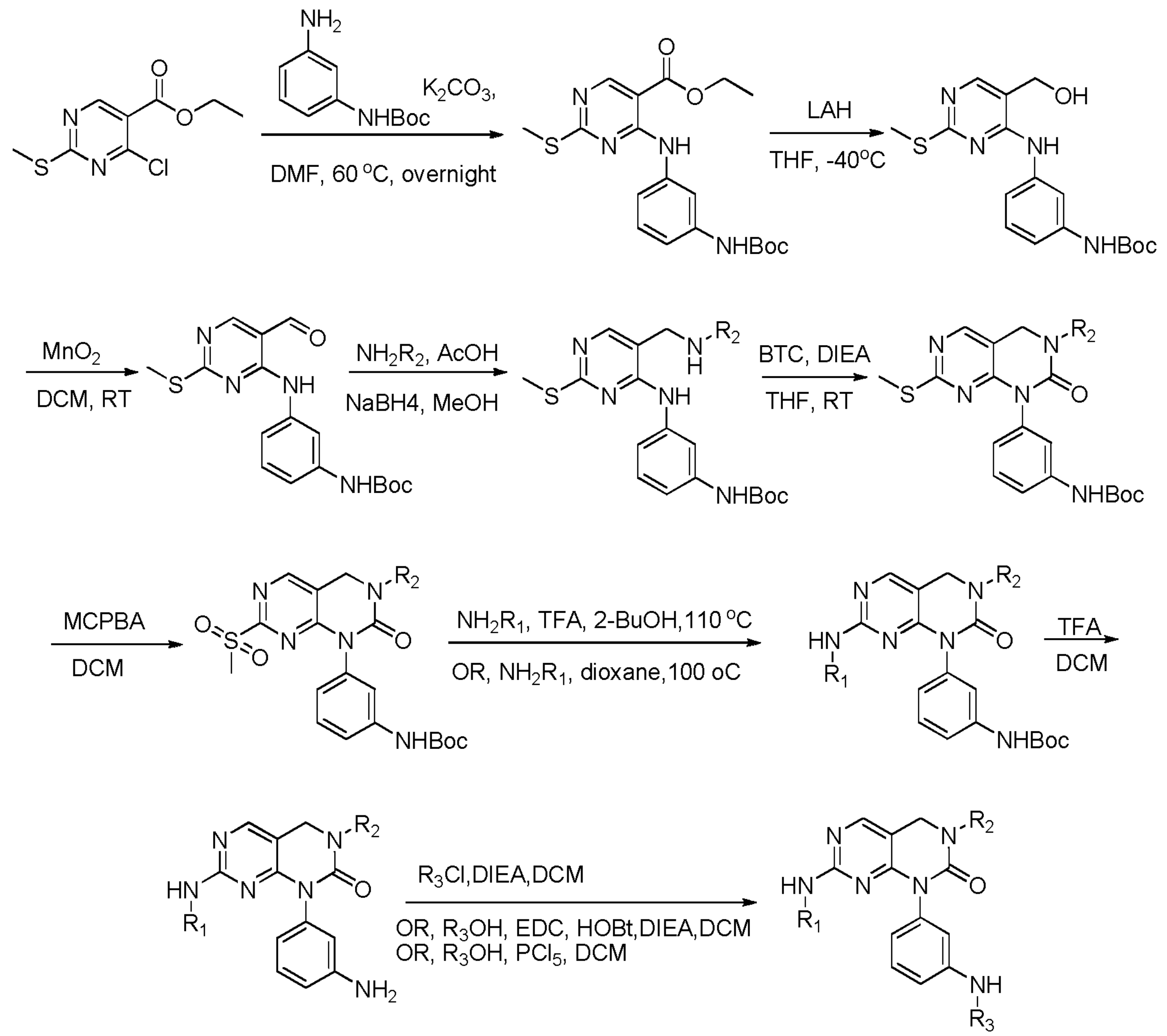


In one embodiment, this present invention provides a method of using compounds in formula (I) and their pharmaceutically acceptable salts for treatment of over proliferative diseases including human cancers or mammalian cancers.

In one embodiment, the invention also relates to the compounds designed in the present invention and their pharmaceutically acceptable salts which are used for the treatment or the prevention of over proliferative diseases, such as gastrointestinal stromal tumors (GIST), histiocytic lymphoma, non-small cell lung cancer, small cell lung cancer, lung adenocarcinoma, squamous cell lung carcinoma, pancreatic cancer, breast cancer, prostate cancer, liver cancer, skin cancer, squamous cell carcinoma, nasopharyngeal carcinoma, leukemia, histiocytic lymphoma and nasopharyngeal carcinoma, and so on.

**Metabolites-Prodrugs**

The metabolites of the compounds and their pharmaceutical salts in the present invention, and prodrugs that are converted to the compounds and their pharmaceutical salts in the present invention are comprised in the claims of the present application.

**Combination Therapy**

Compounds of Formula I -IV may be used in combination with other drugs that are known to be useful in the treatment or amelioration of the diseases or similar diseases.. In the combination administration, such other drugs may be administered, by a route administration and in an amount commonly used, and contemporaneously or sequentially with a compound of Formula I-IV. When a compound of Formula I-IV is used contemporaneously with one or more other drugs, a pharmaceutical composition containing one or more other known drugs and the compound of Formula I-IV is preferred. The combination therapy also comprises therapies in which the compound of Formula I-IV and one or more other known drugs are administered on overlapping schedules. When used in combination with one or more otherdrugs, the compound of Formua I -IV and the other known drugs may be used in lower dosage than when they are used alone.

Drugs or active ingredients used in combination with compounds of Formula I-IV comprises but are not limited to:

estrogen receptor modulator, androgen receptor modulator, retinoid receptor modulator, cell toxin/cell inhibitor, antiproliferative agents, protein transferase inhibitors, HMG-CoA reductase inhibitors, HIV protein kinase inhibitors, reverse transcriptase inhibitors, angiogenesis inhibitors, cell proliferation and survival signaling inhibitors, interference with the cell cycle checkpoint drugs and apoptosis inducing agent, cytotoxic drugs, protein tyrosine inhibitor, EGFR, VEGFR inhibitors, inhibitors of serine / threonine protein inhibitors, inhibitors of Bcr-Abl, c-Kit inhibitor, Met inhibitors, inhibitors of Raf, MEK inhibitor, MMP inhibitors, inhibitors of topoisomerase, histidine deacetylase inhibitors, proteasome inhibitors, inhibitors of CDK, Bcl-2 family protein inhibitor, MDM2 family protein inhibitors, inhibitors of IAP family proteins, inhibitor of STAT family proteins, PI3K, AKT inhibitors, inhibitors of integrin blockade, IFN-$\alpha$, interleukin-12, COX-2 inhibitor, p53, p53 activator inhibitor, VEGF antibody, EGF antibody, etc.

In one embodiment, drugs or active ingredients used in combination with compounds of Formula I-IV comprises but are not limited to: Aldesleukin, Alendronate, interferon, Alitretinoin, allopurinol, allopurinol sodium, palonosetron hydrochloride, Hemel, amino glutethimide, amifostine, amrubicin, Ann acridine, anastrozole, dolasetron, Aranesp, arglabin, arsenic trioxide, Aromasin, 5 - N cytidine, azathioprine, BCG or BCG, Bestatin hydrochloride, betamethasone acetate, betamethasone sodium phosphate, Bexarotene, bleomycin sulfate, broxuridine, bortezomib, busulfan, calcitonin, Alemtuzumab Campath, capecitabine, carboplatin, Casodex, cefesone, Seamus IL, DNR, chlorambucil, cisplatin, cladribine, cladribine, chloride phosphoric acid, Cytarabine, cyclophosphamide, Dacarbazine, Actinomycin D, DNX, dexamethasone, dexamethasone phosphate, estradiol valerate, cefdinir interleukin 2, Methylprednisolone acetate, deslorelin, dexrazoxane, diethylstilbestrol, Diflucan, docetaxel, doxorubicin, doxifluridine, dronabinol, chin -166- chitosan complexes, eligard, rasburicase, epirubicin hydrochloride, aprepitant, epirubicin, alfa-epoetin, erythropoietin, Eptaplatin, levamisole, estradiol formulation, 17- $\beta$ - estradiol, estramustine phosphate sodium, ethinylestradiol, Amifostine, hydroxyl phosphate, Etopophos, etoposide, Fadrozole, tamoxifen, filgrastim, finasteride, floxuridine, fluconazole, fludarabine, 5-fluorine BrdU a phosphate, 5-fluorouracil, fluoxymesterone, flutamide, formestane, Cytarabine hydrochloride, Fotemustine, fulvestrant, immunoglobulin, gemcitabine, gemtuzumab ozogamicin, imatinib mesylate, carmustine capsules, goserelin, hydrocortisone, erythro-hydroxy nonyl adenine, hydroxyurea, Ibritumomab Tiuxetan. Idarubicin, ifosfamide, interferon $\alpha$, IFN-$\alpha$2, interferon $\alpha$-2A,interferon $\alpha$-2B, interferon $\alpha$-n1, IFN $\alpha$-n3, interferon $\beta$, interferon $\gamma$-la, IL-2, intron A, Iressa, Irinotecan, Kytril, mushroom polysaccharide sulfate, letrozole, leucovorin, leuprolide, leuprorelin acetate, Levamisole, levorotation folinic acid calcium salt, levothyroxine sodium, levothyroxine sodium, lomustine, lonidamine, dronabinol, nitrogen mustard, Mecobalamin, medroxyprogesterone acetate, megestrol acetate, melphalan, esterified estrogens, 6-Mercaptopurine, mesna, methotrexate, aminolevulinic acid methyl ester, miltefosine, minocycline, mitomycin C, mitotane, mitoxantrone anthraquinone, Trilostane, citric acid adriamycin liposome, Nedaplatin, Pegfilgrastim, oprelvekin, neupogen, nilutamide, tamoxifen, NSC-631570, recombinant human interleukin 1- $\beta$, octreotide, Ondansetron hydrochloride, hydroprednisone oral solution, oxaliplatin, paclitaxel, prednisone, L-asparaginase enzyme sodium phosphate preparation, Pegasys, pentostatin, Picibanil, pilocarpine hydrochloride, adjoin THP, mithramycin, porfimer sodium, prednimustine, Prednisolone Steaglate, prednisolone, Premarin, C kappa umbilical, recombinant human erythropoietin, raltitrexed, Libby, etidronate rhenium-186, rituximab, Redoxon-A, Romo peptide, pilocarpine hydrochloride tablets, octreotide, Sargramostim, semustine, Schizophyllan, sobuzoxane, Methylprednisolone, Paphos acid, stem cell therapy, streptozocin, strontium chloride -89, levothyroxine sodium, tamoxifen, tamsulosin, TNF-alfa, tastolactone, docetaxel, teceleukin, temozolomide, teniposide, propionic acid testosterone, testosterone propionate, thioguanine, thiotepa, thyroid stimulating hormone, Tiludronic acid, topotecan, toremifene, tositumomab, trastuzumab, Treosulfan, Victoria A acid, methotrexate tablets, three methyl melamine, trimetrexate, triptorelin, double hydroxy acetic acidNaphthalene of triptorelin, UFT, uridine, valrubicin, vesnarinone, alkali, vincristine, Vindesine Vinorelbine, virulizin, dextral razoxane, Zinostatin ester, ondansetron, paclitaxel, acolbifene, Interferon r-lb, affinitak, aminopterin, Arzoxifene, Asoprisnil, atamestane, atrasentan, BAY 43-9006, Avastin, CCI-779, CDC-501, Celebrex, cetuximab, crisnatol, cyproterone acetate, decitabine, DN-101, Doxorubicin -MTC, dSLIM, dutasteride, edotecarin, eflornithine, Exatecan, Fenretinide, histamine hydrochloride, holmium -166 DOTMP, ibandronate, IFN -$\gamma$, intron -PEG, ixabepilone, intron keyhole shaped hemocyanin, L-651582,

Lanreotide, lasofoxifene, Libra, lonafamib, Miproxifene, MS-209, liposome MTP-PE, MX-6, Nafarelin, Nemorubicin, Neovastat, Nolatrexed, Aolimosen, onco-TCS, osidem, paclitaxel poly glutamic acid ester, pamidronate disodium injection, PN-401, QS-21, R -1549, raloxifene, ranpirnase, 13-cis-Victoria A acid, satraplatin, seocalcitol, T-138067, Tarceva, DHA-PTX, thymosin $\alpha 1$, Pirazofurin, tipifarnib, tirapazamine, TLK-286, toremifene, trans MID-lo7R, valspodar, vapreotide, vatalanib, verteporfin, Vinflunine, Z-100 and Zoledronic acid or their combination.

Further explanations are made as following, but those embodiments can not be used to limit the protection scope of the invention.

Example 1

N-(3-(3-methyl-7-(methylamino)-2-oxo-3,4-dihydropyrimido[4,5-d]pyrimidin-1(2H)-yl) phenyl)acrylamide(C-EGF21)

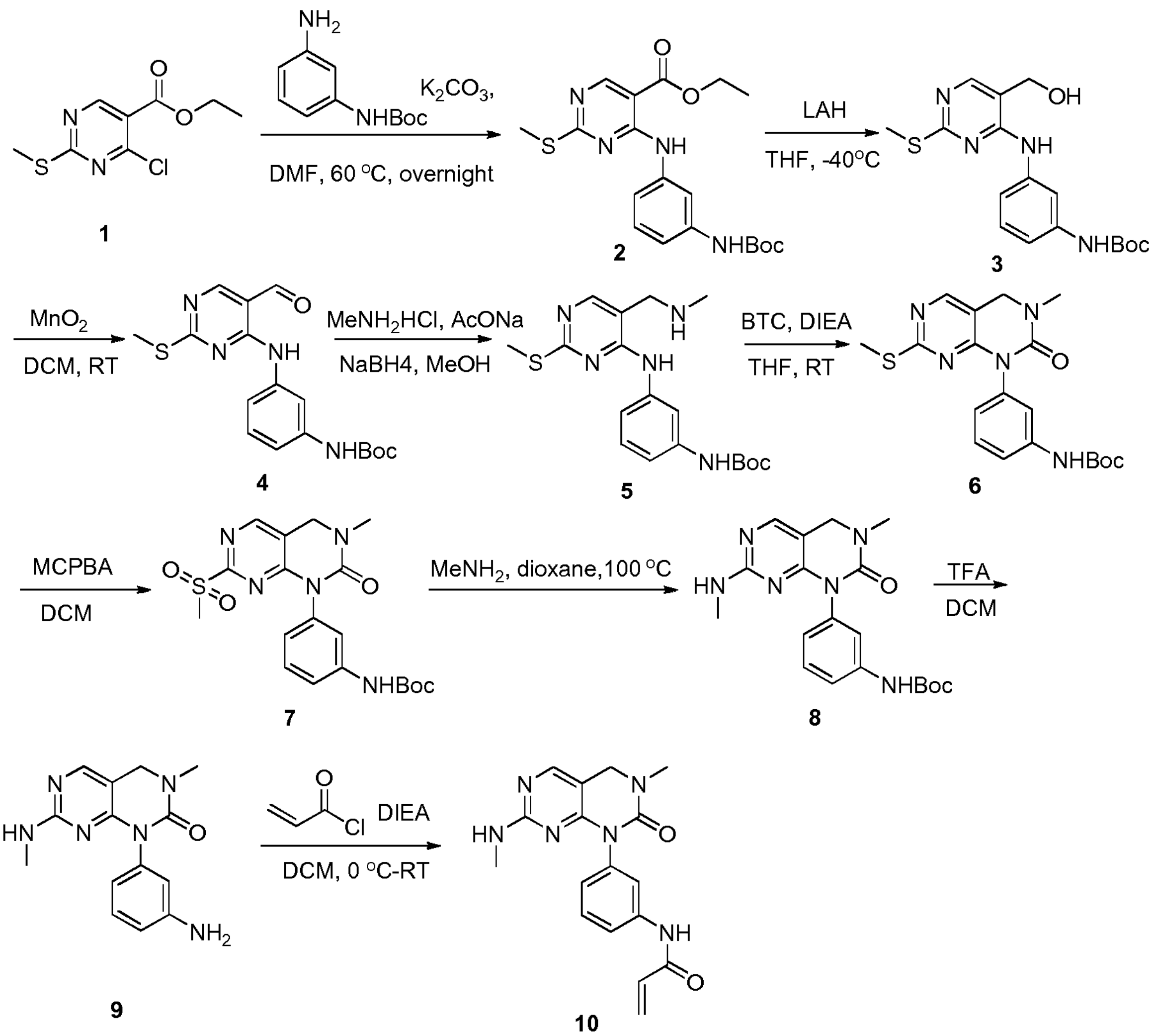


Step 1. ethyl 4-(3-(tert-butoxycarbonylamino)phenylamino)-2-(methylthio)pyrimidine-5-carboxylate(2)

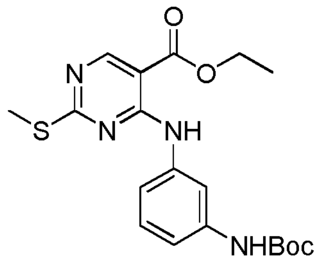

Compound 1 (23.3 g, 100 mmol), N-Boc m-Phenylenediamine (20.8 g, 100 mmol), Potassium carbonate (27.6 g, 200 mmol) were solved in DMF (300 mL). The reaction was heated to 80°C under nitrogen and stirred overnight. After being cooled to room temperature, the reaction mixture was added to ice water (1000 mL). Large amount of solid precipitated. The solid precipitate was filtered under reduced pressure, and vacuum dried to give the white solid (38.8 g, 96% yield) as a white solid.
$^1$H NMR (400Hz, $CDCl_3$) δ 10.37 (s, 1H), 8.76 (s, 1H), 7.90 (s, 1H), 7.34 (d, *J*= 8.0 Hz, 1H), 7.24 (t, *J* = 8.0 Hz, 1H), 7.02 (d, *J* = 8.0 Hz, 1H), 6.53 (s, 1H), 4.38 (q, *J* = 7.2, 14.4 Hz, 2H), 2.55 (s, 3H), 1.52 (s, 9H), 1.40 (t, *J*= 7.2 Hz, 3H).

Step 2. tert-butyl 3-(5-(hydroxymethyl)-2-(methylthio)pyrimidin-4-ylamino)phenylcarbamate (3)

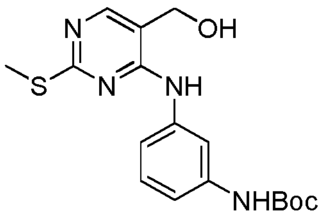

Compound 2 (20.2g, 50 mmol) was solved in anhydrous THF (500 mL) and cooled to -40°C. 2.0 M tetrahydro lithium aluminum (50 mL, 100 mmol) was added to the above reaction solution, warmed to the room temperature, and stirred for 2 hours. Under the ice bath, 20 mL methanol was added to quench the reaction. The reaction mixture was then treated with saturated $NaHCO_3$ solution (75 mL) to separate out aluminum hydroxide. The resulted mixture was filtered by diatomite under reduced pressure, then the solvent was concentrated and separated by column chromatography to yield a yellow solid (10.33 g, 57%).
$^1$H NMR (400Hz, $CDCl_3$) δ 8.02 (s, 1H), 7.86 (s, 1H), 7.80 (s, 1H), 7.36 (dd, *J*= 1.2, 8.0 Hz, 1H), 7.22 (t, *J* = 8.0 Hz, 1H), 6.95 (dd, *J* = 1.2, 8.0 Hz, 1H), 6.52 (s, 1H), 4.61 (s, 2H), 2.52 (s, 3H), 1.52 (s, 9H).

Step 3. tert-butyl 3-(5-formyl-2-(methylthio)pyrimidin-4-ylamino)phenylcarbamate (4)

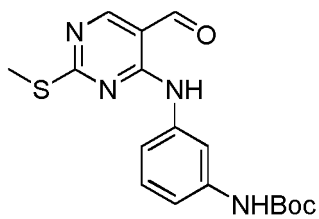

To a solution of compound 3(10.0 g, 27.6mmol) in dichloromethane (300 mL) was added activated manganese-(IV) oxide (24.0 g, 276 mmol) at room temperature, and the mixture was stirred overnight, and then filtered by diatomite. The solvent was evaporated under increased pressure to yield a yellow solid (8.36 g, 84%).
$^1$H NMR (400Hz, $CDCl_3$) δ 10.61 (s, 1H), 9.77 (s, 1H), 8.43 (s, 1H), 7.98 (s, 1H), 7.36 (dd, *J* =0.8, 8.0 Hz, 1H), 7.25-7.29 (m, 1H), 7.03 (dd, *J* = 1.2, 8.0 Hz, 1H), 6.51 (s, 1H), 2.59 (s, 3H), 1.53 (s, 9H).

Step 4. tert-butyl 3-(5-((methylamino)methyl)-2-(methylthio)pyrimidin-4-ylamino)Phenyl Carbamate (5)

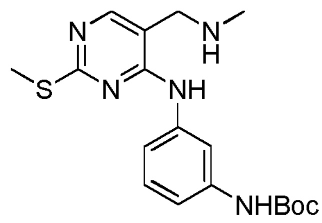

To a solution of compound 4 (7.21 g, 20.0 mmol) in methanol (200 mL) which was cooled to 0 °C were added sodium acetate (8.2 g, 100 mmol) and methanaminium chloride (6.75 g, 100 mmol), the mixture was move to room temperature and stirred for 1 h. The mixture was cooled to 0 °C again and NaBH4 (1.51g, 40.0 mmol) was added. The reaction mixture was move to room temperature and stirred overnight. Then, the solution was concentrated, extracted by DCM, washed by saturated $NaHCO_3$ solution, washed by saturated brine, dried by anhydrous $Na_2SO_4$, and purified by group chromatography to yield a white solid (5.25 g,71%).
$^1H$ NMR (400Hz, $CDCl_3$) $\delta$ 10.12 (s, 1H), 7.89 (s, 1H), 7.78 (s, 1H), 7.35 (d, *J*= 8.8 Hz, 1H), 7.21 (t, *J* = 8.0 Hz, 1H), 6.97 (dd, *J* = 1.2, 8.0 Hz, 1H), 6.50 (s, 1H), 3.74 (s, 2H), 3.55 (s, 3H), 2.44 (d, *J*= 0.4 Hz, 3H), 1.52 (s, 9H).

Step 5. tert-butyl 3-(3-methyl-7-(methylthio)-2-oxo-3,4-dihydropyrimido[4,5-d] pyrimidin-1(2H)-yl) phenylcarbamate (6)

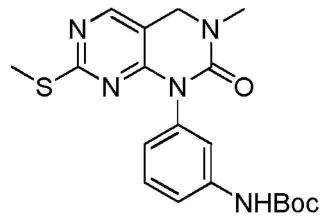

To a solution of compound 5 (5.20 g, 13.8 mmol) in THF (140 mL) were added DIEA (8 mL, 55.2 mmol) and 0.2 M triphosgene (25mL, 5.05 mmol) at 0 °C, and the mixture was stirred at room temperature for 1 h. The solvent was concentrated, extracted by DCM, washed by water for three times, washed by saturated brine for one time, dried by anhydrous $Na_2SO_4$, evaporated under reduced pressure, and then recrystallized by ethyl acetate to yield a white solid (4.71 g, 85%).
$^1H$ NMR (400Hz, $CDCl_3$) $\delta$ 8.10 (s, 1H), 7.44 (s, 1H), 7.34 (t, *J*= 8.0 Hz, 1H), 7.23 (d, *J*= 7.2 Hz, 1H), 6.90 (d, *J*= 8.0 Hz, 1H), 6.55 (s, 1H), 4.46 (s, 2H), 3.08 (s, 3H), 2.14 (s, 3H), 1.50 (s, 9H).

Step 6. tert-butyl 3-(3-methyl-7-(methylsulfonyl)-2-oxo-3,4-dihydropyrimido[4,5-d]Pyrimidin-1

(2H)-yl)phenylcarbamate(7)

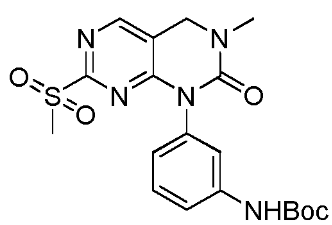

To a solution of compound 6(4.0 g, 10.0 mmol) in dichloromethane(100 mL) was added 85%m-chloroperbenzoic acid (6.1 g, 30.0 mmol) in batches at 0 °C. The reaction mixture was stirred for 3 h at room temperature, diluted by DCM, washed by saturated $NaHCO_3$ solution for three times, washed by saturated brine for one time, dried by anhydrous

$Na_2SO_4$, evaporated the solvent under reduced pressure, and then recrystallized by ethyl acetate to yield a white solid (3.90 g, 90% yield).
$^1H$ NMR (400Hz, $CDCl_3$) δ 8.45 (s, 1H), 7.58 (s, 1H), 7.35 (t, *J* = 8.0 Hz, 1H), 7.15 (dd, *J* = 1.2, 8.0 Hz, 1H), 6.89 (dd, *J* = 1.2, 8.0 Hz, 1H), 6.67 (s, 1H), 4.62 (s, 2H), 3.11 (s, 3H), 2.98 (s, 3H), 1.48 (s, 9H).

Step 7. tert-butyl 3-(3-methyl-7-(methylamino)-2-oxo-3,4-dihydropyrimido[4,5-d] pyrimidin -1- (2H)-yl)phenylcarbamate(8)

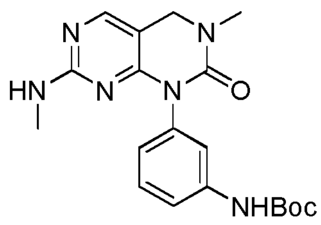

To a solution of compound 7 (260mg, 0.6mmol) in 1,4-Dioxane (1ml), were added Methylamine methylamine hydrochloride (405mg, 10eq), $CH_3COONa$ (492mg, 10eq). The reaction mixture was heated to 100 °C for reacting in a sealed tube for 24 h. The reaction mixture was diluted by DCM, washed by saturated $NaHCO_3$, washed by saturated brine, dried by anhydrous $Na_2SO_4$, evaporated the solvent under reduced pressure, and then purified by column chromatography to yield a whilt solid (205 mg, 89%).
$^1H$ NMR (400Hz, $CDCl_3$) δ 7.93 (s, 1H), 7.36 (s, 1H), 7.33 (t, *J*= 8.0 Hz, 1H), 7.28 (s, 1H), 6.90 (d, *J* = 8.0 Hz, 1H), 6.55 (s, 1H), 4.84 (d, *J* = 4.4 Hz, 1H), 4.37 (s, 2H), 3.06 (s, 3H), 2.76 (s, 3H), 1.49 (s, 9H)

Step 8. 1-(3-aminophenyl)-3-methyl-7-(methylamino)-3,4-dihydropyrimido[4,5-d]pyrimidin-2(1H)-one (9)

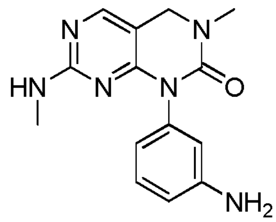

To a solution of compound **8** (205mg, 0.53mmol) in DCM (1ml) was added TFA (0.4ml, 10eq). The reaction mixture was stirred for 4 h at room temperature, diluted by DCM, washed by saturated $NaHCO_3$ solution, washed by saturated brine, dried by anhydrous $Na_2SO_4$, and then evaporated the solvent under reduced pressure to yield a yellow solid (140mg, 93%).

Step 9.N-(3-(3-methyl-7-(methylamino)-2-oxo-3,4-dihydropyrimido[4,5-d]pyrimidin-1(2H)-yl) phenyl)acrylamide(C-EGF21) (10)

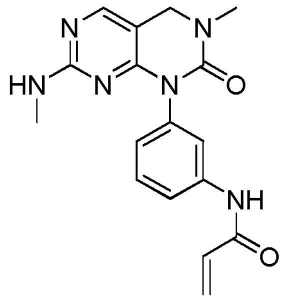

To a solution of compound **9** (128mg, 0.45mmol) in dichloromethane (2ml), were added diisopropylethylamine (65μl,1.0eq). The reaction mixture was cooled to 0 °C, and then added acryloyl chloride (37μl,1.0eq) slowly, stirred for 1 h at room temperature, evaporated the solvent under reduced pressure, and then purified by column chromatography to yield a white solid (114mg , 75%).
$^1$H NMR (400Hz, DMSO- $d_6$) δ 10.23 (s, 1H), 8.01 (s, 1H), 7.61 (d, *J*= 8.0 Hz, 1H), 7.55 (s, 1H), 7.36 (t, *J* = 8.0 Hz, 1H), 6.91 (d, *J* = 7.6 Hz, 1H), 6.71 (s, 1H), 6.43 (dd, *J* = 10.0, 16.8 Hz, 1H), 6.25 (d, *J* = 16.8 Hz, 1H), 5.76 (d, *J* = 10.4 Hz, 1H), 4.37 (s, 2H), 2.93 (s, 3H), 2.59 (s, 3H).
LCMS (ESI): m/z 339.1 $[M + H]^+$.

Example 2

N-(3-(3-methyl-2-oxo-7-(phenylamino)-3,4-dihydropyrimido[4,5-d]pyrimidin-1(2H)-yl)phenyl)acrylamide(C-EGF 10)

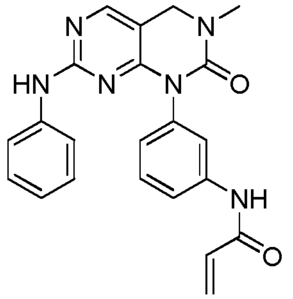

This compound was synthesized with similar procedures to that of example 1.
$^1$H NMR (400Hz, DMSO- $d_6$) δ 10.30 (s, 1H), 9.41 (s, 1H), 8.18 (s, 1H), 7.43 (d, *J* = 8.0 Hz, 1H), 7.66 (s, 1H), 7.45 (t, *J* = 8.0 Hz, 1H), 7.27 (d, *J* = 8.0 Hz, 2H), 6.99 (d, *J* = 8.0 Hz, 1H), 6.93 (t, *J* = 7.6 Hz, 2H), 6.75 (t, *J* = 7.2 Hz, 1H), 6.42 (dd, *J* = 10.4, 16.8 Hz, 1H), 6.24(d, *J*= 16.8 Hz, 1H), 5.75 (d, *J* = 10.4 Hz, 1H), 4.48 (s, 2H), 2.97 (s, 3H).
LCMS (ESI): m/z 401.1 $[M + H]^+$.

Example 3

N-(3-(3-methyl-7-(4-methylpiperazin-1-yl)-2-oxo-3,4-dihydropyrimido[4,5-d]pyrimidin-1 (2H)-yl)phenyl)acrylamide(C-EGF 19)

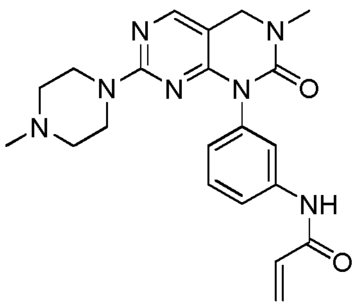

This compound was synthesized with similar procedures to that of example 1.
$^1$H NMR (400Hz, DMSO- $d_6$) δ 8.25 (s, 1H), 7.95 (s, 1H), 7.53 (s, 1H), 7.38 (d, *J* = 7.6 Hz, 1H), 7.23-7.27 (m, 1H), 6.88 (d, *J* = 7.6 Hz, 1H), 6.32 (d, *J* = 16.4 Hz, 1H), 6.12 (dd, *J* = 10.0, 16.4 Hz, 1H), 5.64 (d, *J* = 10.0 Hz, 1H), 4.41 (s, 2H), 3.52 (m, 4H), 3.10 (s, 3H), 2.30 (m, 4H), 2.25 (s, 3H).
LCMS (ESI): m/z 408.2 $[M + H]^+$.

Example 4

N-(3-(7-(2-methoxy-4-(4-methylpiperazin-1-yl)phenylamino)-3-methyl-2-oxo-3,4-dihydropyrimido[4,5-d]pyrimidin-1(2H)-yl)phenyl)acrylamide(C-EGF06)

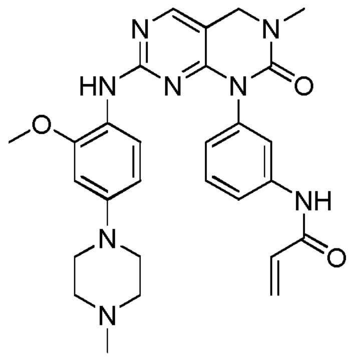

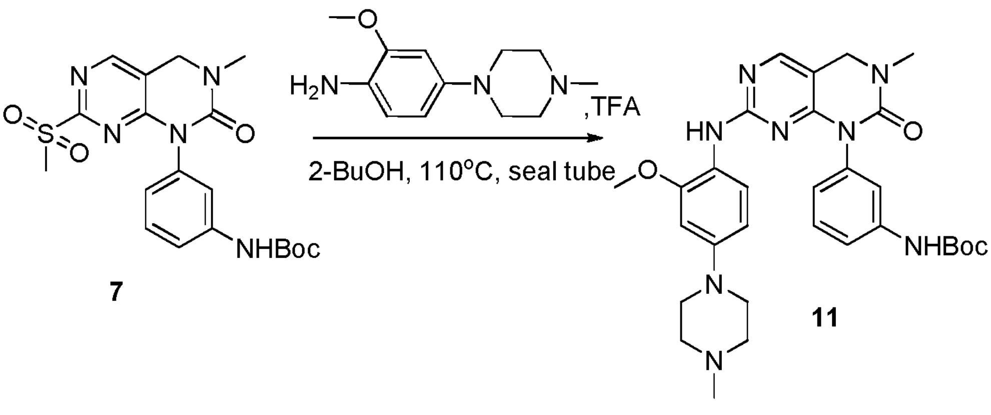


7

11

tert-butyl 3-(7-(2-methoxy-4-(4-methylpiperazin-1-yl)phenylamino)-3-methyl-2-oxo-3,4-dihydropyrimido[4,5-d]pyrimidin-1(2H)-yl)phenylcarbamate (11)

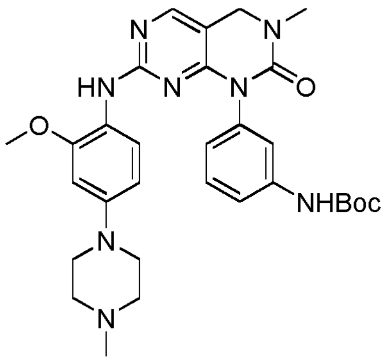

To a solution of compound 7 (260 mg, 0.6 mmol) in butan-2-ol (1 mL) were added 2-methoxy-4-(4-methylpiperazin-1-yl)aniline (133 mg, 0.6mmol) and trifluoroacetic acid (48μL, 0.6 mmol). The reaction mixture was heated to 110 °C and reacted in a sealed tube for 24 h, diluted by DCM, washed by saturated $NaHCO_3$ solution, washed by saturated brine, dried by anhydrous $Na_2SO_4$, evaporated the solvent under reduced pressure, and then purified by column chromatography to yield a yellow solid (151 mg, 44%).
$^1$H NMR (400Hz, $CDCl_3$) δ 7.99 (s, 1H), 7.48 (d, *J*= 7.5 Hz, 1H), 7.44 (s, 1H), 7.41 (t, *J*= 8.0 Hz, 1H), 7.35 (s, 1H), 6.97 (d, *J* = 7.5 Hz, 1H), 6.63 (s,1H), 6.42 (d, *J* = 2.0 Hz, 1H), 6.16 (d, *J* = 6.4 Hz, 1H), 4.42 (s, 2H), 3.80 (s, 3H), 3.22 (m, 4H), 3.08 (s, 3H), 2.79 (m, 4H), 1.47 (s, 9H).

The other steps are similar to that of example 1.

N-(3-(7-(2-methoxy-4-(4-methylpiperazin-1-yl)phenylamino)-3-methyl-2-oxo-3,4-dihydropyrimido[4,5-d]pyrimidin-1(2H)-yl)phenyl)acrylamide(C-EGF06)

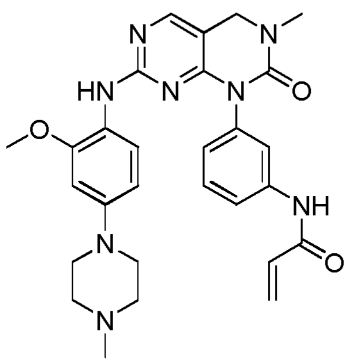

$^1$H NMR (400Hz, DMSO- $d_6$) δ 10.31 (s, 1H), 8.11 (s, 1H), 7.81 (d, *J* = 7.6 Hz, 1H), 7.56 (s, 1H), 7.48 (s, 1H), 7.43 (t, *J* = 8.0 Hz, 1H), 7.27 (d, *J* = 8.4 Hz, 1H), 6.95 (d, *J* = 8.0 Hz, 1H), 6.51 (s, 1H), 6.44 (dd, *J* = 10.0, 16.8 Hz, 1H), 6.25(d, *J* = 16.8 Hz, 1H), 6.02 (d, *J* = 8.0 Hz, 1H), 5.76 (d, *J* = 10.0 Hz, 1H), 4.46 (s, 2H), 3.76 (s, 3H), 2.99 (m, 4H), 2.96 (s, 3H), 2.50 (m, 4H), 2.22 (s, 3H).
LCMS (ESI): m/z 529.2 $[M + H]^+$.

Example 5

N-(3-(7-(2-methoxy-4-(piperidin-1-yl)phenylamino)-3-methyl-2-oxo-3,4-dihydropyrimido [4,5-d]pyrimidin-1(2H)-yl)phenyl)acrylamide(C-EGF11)

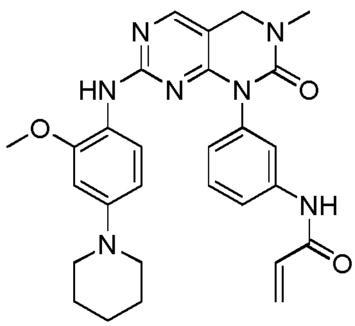

This compound was synthesized with similar procedures to that of example 4.
$^1$H NMR (400Hz, DMSO- $d_6$) δ 10.29 (s, 1H), 8.11 (s, 1H), 7.82 (d, *J* = 8.0 Hz, 1H), 7.56 (s, 1H), 7.46 (s, 1H), 7.43 (t, *J* = 8.0 Hz, 1H), 7.25 (d, *J* = 8.0 Hz, 1H), 6.95 (d, *J* = 8.0 Hz, 1H), 6.49 (d, *J* = 1.6 Hz, 1H), 6.43 (dd, *J* = 8.0, 16.8 Hz, 1H), 6.25 (dd, *J* = 1.6, 16.8 Hz, 1H), 6.02 (d, *J*= 7.2 Hz, 1H), 5.76 (d, *J* = 11.2 Hz, 1H), 4.46 (s, 2H), 3.75 (s, 3H), 2.96 (m, 7H), 1.59 (m, 4H), 1.49-1.50 (m, 2H).
LCMS (ESI): m/z 514.2 $[M + H]^+$.

Example 6

N-(3-(7-(2-methoxy-4-(pyrrolidin-1-yl)phenylamino)-3-methyl-2-oxo-3,4-dihydropyrimido [4,5-d]pyrimidin-1 (2H)-yl)phenyl)acrylamide(C-EGF 12)

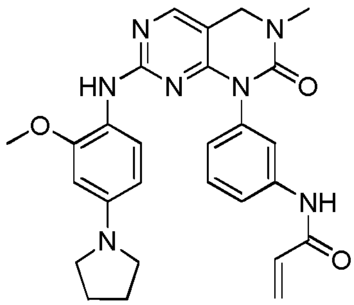

This compound was synthesized with similar procedures to that of example 4.
$^1$H NMR (400Hz, DMSO- $d_6$) δ 10.31 (s, 1H), 8.07 (s, 1H), 7.77 (d, *J*= 8.0 Hz, 1H), 7.59 (s, 1H), 7.41 (t, *J* = 8.0 Hz, 1H), 7.15 (d, *J* = 8.0 Hz, 1H), 6.95 (d, *J* = 8.0 Hz, 1H), 6.44 (dd, *J* =10.0, 16.8 Hz, 1H), 6.25 (d, *J* = 16.4 Hz, 1H), 6.10 (s, 1H), 5.76 (d, *J* = 10.0 Hz, 2H), 4.44 (s, 2H), 3.74 (s, 3H), 3.14 (m, 4H), 2.96 (s, 3H), 1.92 (m, 4H).
LCMS (ESI): m/z 500.2 $[M + H]^+$.

Example 7

N-(3-(7-(2-methoxy-4-morpholinophenylamino)-3-methyl-2-oxo-3,4-dihydropyrimido[4,5-d]pyrimidin-1(2H)-yl)phenyl)acrylamide(C-EGF08)

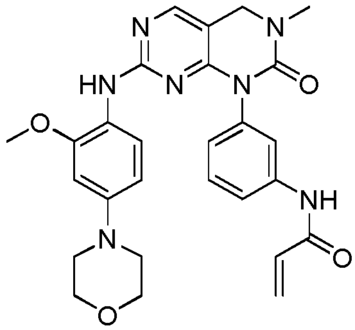

This compound was synthesized with similar procedures to that of example 4.
$^1$H NMR (400Hz, DMSO- $d_6$) $\delta$ 10.30 (s, 1H), 8.12 (s, 1H), 7.81 (d, *J*= 6.8 Hz, 1H), 7.56 (s, 1H), 7.50 (s, 1H), 7.43 *(t, J* = 8.0 Hz, 1H), 7.28 (d, *J* = 10.0 Hz, 1H), 6.96 (d, *J* = 10.0 Hz, 1H), 6.52 (d, *J* = 2.0 Hz, 1H), 6.43 (dd, *J*=10.0, 16.8 Hz, 1H), 6.22-6.27 (m, 1H), 6.02 (d, *J* = 8.0 Hz, 1H), 5.74-5.77 (m, 1H), 4.46 (s, 2H), 3.76 (s, 3H), 3.71 (t, *J* = 4.4 Hz, 4H), 2.96 (m, 7H).
LCMS (ESI): m/z 516.2 $[M + H]^+$.

Example 8

N-(3-(7-(2-methoxy-4-thiomorpholinophenylamino)-3-methyl-2-oxo-3,4-dihydropyrimido[4,5-d]pyrimidin-1(2H)-yl)phenyl)acrylamide(C-EGF09)

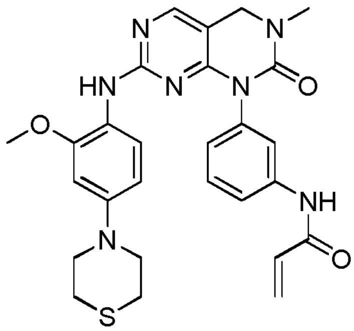

This compound was synthesized with similar procedures to that of example 4.
$^1$H NMR (400Hz, DMSO- $d_6$) $\delta$ 10.31 (s, 1H), 8.11 (s, 1H), 7.82 (d, *J* = 7.6 Hz, 1H), 7.56 (s, 1H), 7.51 (s, 1H), 7.43 (t, *J* = 8.0 Hz, 1H), 7.27 (d, *J* = 8.8 Hz, 1H), 6.97 (d, *J* = 7.6 Hz, 1H), 6.49 (d, *J* = 2.0 Hz, 1H), 6.43 (dd, *J* =10.0, 16.8 Hz, 1H), 6.26(dd, *J* =2.0, 16.8 Hz, 1H), 6.02 (d, *J* = 7.6 Hz, 1H), 5.75-5.77 (m, 1H), 4.45 (s, 2H), 3.76 (s, 3H), 3.33 (t, *J* = 4.8 Hz, 4H), 2.96 (s, 3H), 2.65 (t, *J*= 4.8 Hz, 4H).
LCMS (ESI): m/z 532.2 $[M + H]^+$.

Example 9

N-(3-(7-(2-methoxy-4-(4-(4-methylpiperazin-1-yl)piperidin-1-yl)phenylamino)-3-methyl-2-oxo-3,4-dihydropyrimido [4,5-d]pyrimidin-1 (2H)-yl)phenyl)acrylamide(C-EGF 14)

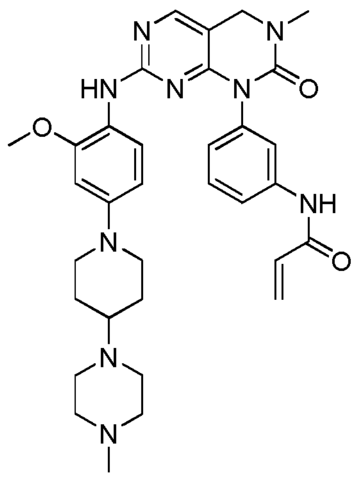

This compound was synthesized with similar procedures to that of example 4.
$^1$H NMR (400Hz, DMSO- $d_6$) δ 10.28 (s, 1H), 8.11 (s, 1H), 7.82 (d, *J* = 8.0 Hz, 1H), 7.55 (s, 1H), 7.47 (s, 1H), 7.42 *(t, J* = 8.0 Hz, 1H), 7.24 (d, *J* = 8.8 Hz, 1H), 6.95 (d, *J* = 8.0 Hz, 1H), 6.49 (d, *J* = 2.0 Hz, 1H), 6.43 (dd, *J* = 10.0, 16.8 Hz, 1H), 6.25 (dd, *J* = 1.6, 16.8 Hz, 1H), 6.02 (d, *J* = 7.2 Hz, 1H), 5.76 (dd, *J* = 1.6, 10.0 Hz, 1H), 4.46 (s, 2H), 3.75 (s, 3H), 3.53 (d, *J* = 12.0 Hz, 2H), 2.96 (s, 3H), 2.50-2.55 (m, 6H), 2.21-2.30 (m, 5H), 2.14 (s, 3H), 1.80 (d, *J* = 11.2 Hz, 2H), 1.42-1.50 (m, 2H).
LCMS (ESI): m/z 612.3 $[M + H]^+$.

Example 10

N-(3-(7-(4-(4-(dimethylamino)piperidin-1-yl)-2-methoxyphenylamino)-3-methyl-2-oxo-3,4-dihydropyrimido[4,5-d]pyrimidin-1 (2H)-yl)phenyl)acrylamide(C-EGF 13)

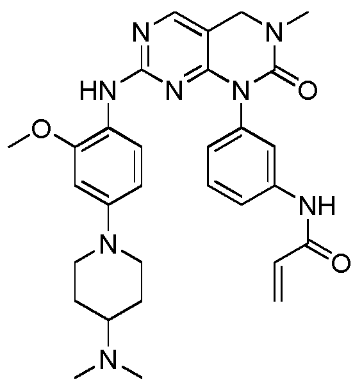

This compound was synthesized with similar procedures to that of example 4.
$^1$H NMR (400Hz, DMSO- $d_6$) δ 10.34 (s, 1H), 8.11 (s, 1H), 7.84 (d, *J* = 7.6 Hz, 1H), 7.55 (s, 1H), 7.48 (s, 1H), 7.42 (t, *J* = 8.0 Hz, 1H), 7.25 (d, *J* = 8.4 Hz, 1H), 6.95 (d, *J* = 7.2 Hz, 1H), 6.51 (s, 1H), 6.45 (dd, *J* = 10.0, 17.2 Hz, 1H), 6.25 (d, *J* = 16.8 Hz, 1H), 6.04 (s, 1H), 5.76 (d, *J*= 9.6 Hz, 1H), 4.46 (s, 2H), 3.75 (s, 3H), 3.56 (d, *J*= 12.4 Hz, 2H), 3.35 (s, 3H), 2.87 (m, 1H), 2.49-2.56 (m, 2H), 2.34 (s, 6H), 1.86 (d, *J* = 10.4 Hz, 2H), 1.46-1.55 (m, 2H).
LCMS (ESI): m/z 557.2 $[M + H]^+$.

Example 11

N-(3-(7-(2-methoxy-4-(4-methyl-1,4-diazepan-1-yl)phenylamino)-3-methyl-2-oxo-3,4-dihydropyrimido [4,5-d]pyrimidin-1(2H)-yl)phenyl)acrylamide(C-EGF 17)

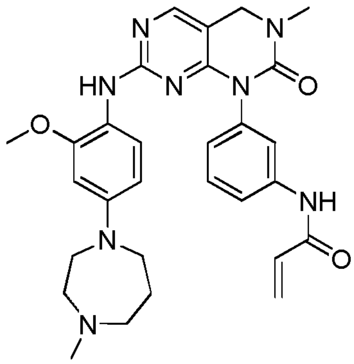

This compound was synthesized with similar procedures to that of example 4.
$^1$H NMR (400Hz, DMSO- $d_6$) δ 10.30 (s, 1H), 8.19 (s, 1H), 7.85 (s, 1H), 7.69-7.70 (m, 2H ), 7.51 *(t, J* = 8.0 Hz, 1H), 7.45 (t, *J* = 8.0 Hz, 1H), 6.96-6.99 (m, 2H), 6.40-6.46 (m, 2H), 6.24 (dd, *J* = 1.6, 16.8 Hz, 1H), 5.75 (dd, *J* = 1.6, 10.0 Hz, 1H), 4.50 (s, 2H), 3.33 (m, 7H), 2.98 (s, 3H), 2.74 (t, *J*=4.4 Hz, 4H), 2.49 (m, 2H), 2.24 (s, 3H).
LCMS (ESI): m/z 543.2 $[M + H]^+$.

Example 12

(R)-N-(3-(7-(4-(3-(dimethylamino)pyrrolidin-1-yl)-2-methoxyphenylamino)-3-methyl-2-oxo-3,4-dihydropyrimido[4,5-d] pyrimidin-1(2H)-yl)phenyl)acrylamide(C-EGF15)

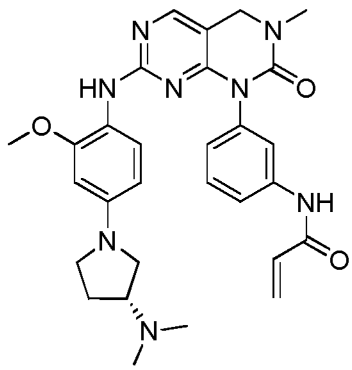

This compound was synthesized with similar procedures to that of example 4.
$^1$H NMR (400Hz, $CDCl_3$) δ 10.29 (s, 1H), 8.07 (s, 1H), 7.82 (d, *J* = 8.0 Hz, 1H), 7.54 (s, 1H), 7.41 *(t, J* = 8.0 Hz, 2H), 7.20 (d, *J* = 8.4 Hz, 1H), 6.94 *(d, J* = 7.6 Hz, 1H), 6.43 (dd, *J* = 10.0, 16.8 Hz, 1H), 6.25 (dd, *J* = 1.6, 16.8 Hz, 1H), 6.09 *(d, J* = 2.0 Hz, 1H), 5.75 (dd, *J* = 2.0, 10.0 Hz, 1H), 5.68 (d, *J* = 6.4 Hz, 1H), 4.44 (s, 2H), 3.75 (s, 3H), 3.23-3.33 (m, 2H), 3.11-3.17 (m, 1H), 2.91-2.96 (m, 4H), 2.69-2.77 (m, 1H), 2.14 (s, 6H), 2.08-2.12 (m, 1H), 1.70-1.80 (m, 1H).
LCMS (ESI): m/z 543.2 $[M + H]^+$.

Example 13

(S)-N-(3-(7-(4-(3-(dimethylamino)pyrrolidin-1-yl)-2-methoxyphenylamino)-3-methyl-2-oxo-3,4-dihydropyrimido[4,5-d] pyrimidin-1(2H)-yl)phenyl)acrylamide(C-EGF16)

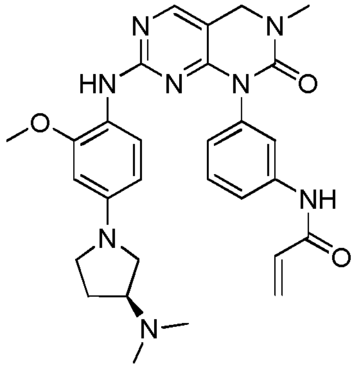

This compound was synthesized with similar procedures to that of example 4.
$^1$H NMR (400Hz, $CDCl_3$) δ 10.27 (s, 1H), 8.07 (s, 1H), 7.82 (d, *J* = 7.2 Hz, 1H), 7.54 (s, 1H), 7.41 (t, *J* = 8.0 Hz, 2H), 7.20 (d, *J* = 8.4 Hz, 1H), 6.94 (d, *J* = 7.6 Hz, 1H), 6.43 (dd, *J* = 10.0, 17.2 Hz, 1H), 6.25 (dd, *J* = 2.0, 16.8 Hz, 1H), 6.09 (d, *J* = 2.4 Hz, 1H), 5.75 (dd, *J* = 2.0, 10.0 Hz, 1H), 5.69 (s, 1H), 4.44 (s, 2H), 3.75 (s, 3H), 3.23-3.33 (m, 2H), 3.11-3.17 (m, 1H), 2.92-2.96 (m, 4H), 2.73-2.78 (m, 1H), 2.21 (s, 6H), 2.08-2.15 (m, 1H), 1.71-1.81 (m, 1H).
LCMS (ESI): m/z 543.2 $[M + H]^+$.

Example 14

N-(3-(7-(4-(4-acetylpiperazin-1-yl)-2-methoxyphenylamino)-3-methyl-2-oxo-3,4-dihydropyrimido[4,5-d]pyrimidin-1(2H)-yl)phenyl)acrylamide(C-EGF20)

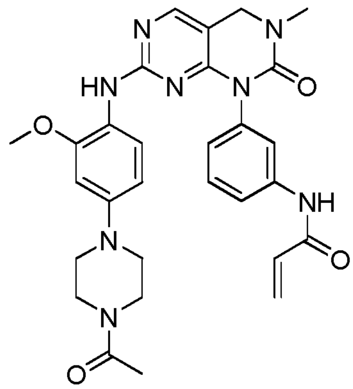

This compound was synthesized with similar procedures to that of example 4.
$^1$H NMR (400Hz, DMSO- $d_6$) δ 10.28 (s, 1H), 8.12 (s, 1H), 7.82 (d, *J*= 7.6 Hz, 1H), 7.55 (s, 1H), 7.50 (s, 1H), 7.43 (t, *J* = 8.0 Hz, 1H), 7.28 (d, d, *J* = 8.8 Hz, 1H), 6.96 (d, *J* = 7.6 Hz, 1H), 6.55 (d, d, *J* = 1.6 Hz, 1H), 6.43 (dd, *J* = 10.0, 16.8 Hz, 1H), 6.25 (dd, *J* = 2.0, 17.2 Hz, 1H), 6.04 (d, *J* = 8.8 Hz, 1H), 5.75 (dd, *J* = 2.0, 10.0 Hz, 1H), 4.46 (s, 2H), 3.77 (s, 3H), 3.52-3.56 (m, 4H), 3.00 (t, *J*= 4.8 Hz, 2H), 2.96 (s, 3H), 2.93 (t, *J*= 4.8 Hz, 2H), 2.04 (s, 3H).
LCMS (ESI): m/z 557.2 $[M + H]^+$.

Example 15

N-(3-(7-(4-(dimethylamino)-2-methoxyphenylamino)-3-methyl-2-oxo-3,4-dihydropyrimido[4,5-d]pyrimidin-1(2H)-yl)phenyl)acrylamide(C-EGF23)

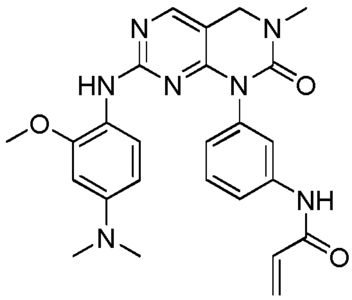

This compound was synthesized with similar procedures to that of example 4.
$^1$H NMR (400Hz, DMSO- $d_6$) δ 10.28 (s, 1H), 8.09 (s, 1H), 7.79 (d, *J*= 8.8 Hz, 1H), 7.57 (s, 1H), 7.42 *(t, J* = 8.0 Hz, 2H), 7.22 (d, *J* = 8.8 Hz, 1H), 6.95 (d, *J* = 8.0 Hz, 1H), 6.43 (dd, *J* = 10.0, 16.8 Hz, 1H), 6.30 (d, *J* = 2.4 Hz, 1H), 6.25 (dd, *J* = 2.0, 16.8 Hz, 1H), 5.87 (d, *J* = 7.6 Hz, 1H), 5.75 (dd, *J* = 2.0, 10.0 Hz, 1H), 4.45 (s, 2H), 3.76 (s, 3H), 2.96 (s, 3H), 2.78 (s, 6H).
LCMS (ESI): m/z 474.2 $[M + H]^+$.

Example 16

N-(3-(7-(2-fluoro-4-(4-methylpiperazin-1-yl)phenylamino)-3-methyl-2-oxo-3,4-dihydropyrimido[4,5-d]pyrimidin-1(2H)-yl)phenyl)acrylamide(C-EGF18)

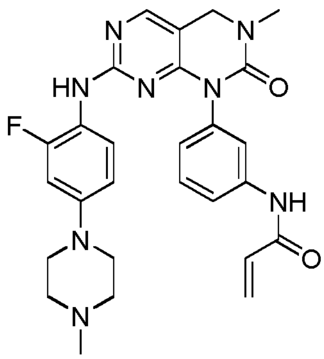

This compound was synthesized with similar procedures to that of example 4.
$^1$H NMR (400Hz, $CDCl_3$) δ 10.30 (s, 1H), 8.19 (s, 1H), 7.86 (s, 1H), 7.69-7.70 (m, 2H), 7.51 (t, *J* = 8.0 Hz, 1H), 7.45 (t, *J* = 8.0 Hz, 1H), 6.96-6.99 (m, 2H), 6.40-6.47 (m, 2H), 6.24 (dd, *J* = 2.0, 17.2 Hz, 1H), 5.75 (dd, *J* = 2.0, 10.0 Hz, 1H), 4.50 (s, 2H), 2.98 (s, 3H), 2.74 (t, *J* = 4.4 Hz, 4H), 2.50 (m, 4H), 2.24 (s, 3H).
LCMS (ESI): m/z 517.2 $[M + H]^+$.

Example 17

N-(3-(7-(2-methoxy-4-(2-methoxyethoxy)phenylamino)-3-methyl-2-oxo-3,4-dihydropyrimido[4,5-d]pyrimidin-1(2H)-yl)phenyl)acrylamide(C-EGF22)

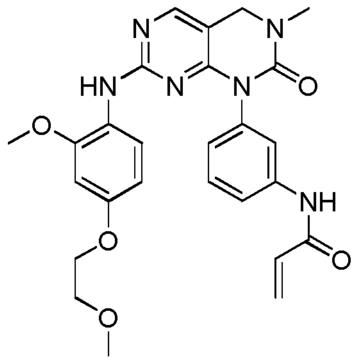

This compound was synthesized with similar procedures to that of example 4.
$^1$H NMR (400Hz, $CDCl_3$) δ 10.30 (s, 1H), 8.12 (s, 1H), 7.75 (d, *J*= 7.6 Hz, 1H), 7.61 (s, 1H), 7.54 (s, 1H), 7.43 *(t, J* = 8.0 Hz, 1H), 7.30 (d, *J* = 8.4 Hz, 1H), 6.96 (d, *J* = 7.6 Hz, 1H), 6.52 (d, *J* = 2.4 Hz, 1H), 6.43 (dd, *J* = 10.0, 16.8 Hz, 1H), 6.25 (dd, *J* = 1.2, 16.8 Hz, 1H), 6.04 (d, *J* = 8.4 Hz, 1H), 5.75 (d, *J* = 11.6 Hz, 1H), 4.46 (s, 2H), 3.97 (t, *J* = 4.4 Hz, 2H), 3.76 (s, 3H), 3.61 (t, *J* = 4.4 Hz, 2H), 3.30 (s, 3H), 2.96 (s, 3H).
LCMS (ESI): m/z 505.0 $[M + H]^+$.

Example 18

N-(3-(3-isopropyl-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF24)

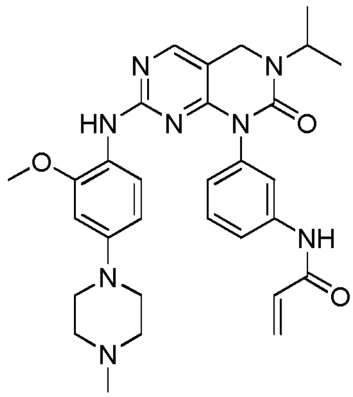

This compound was synthesized with similar procedures to that of example 4.
$^1$H NMR (400Hz, DMSO- $d_6$) δ 10.28 (s, 1H), 8.14 (s, 1H), 7.79 (d, *J*= 7.2 Hz, 1H), 7.57 (s, 1H), 7.46 (s, 1H), 7.42 (t, *J* = 8.0 Hz, 1H), 7.30 (d, *J* = 8.0 Hz, 1H), 6.97 (d, *J* = 8.0 Hz, 1H), 6.51 (s, 1H), 6.43 (dd, *J* = 10.0, 16.8 Hz, 1H), 6.25 (d, *J* = 16.8 Hz, 1H), 6.04 (d, *J* = 6.0 Hz, 1H), 5.76 (d, *J* = 10.0 Hz, 1H), 4.54-4.57 (m, 1H), 4.37 (s, 2H), 3.76 (s, 3H), 2.99 (m, 4H), 2.42 (m, 4H), 2.21 (s, 3H), 1.19 (d, *J* = 6.4 Hz, 6H).
LCMS (ESI): m/z 557.2 $[M + H]^+$.

Example 19

N-(3-(7-(2-methoxy-4-(4-methylpiperazin-1-yl)phenylamino)-3-(4-methoxyphenyl)-2-oxo-3,4-dihydropyrimido[4,5-d]pyrimidin-1(2H)-yl)phenyl)acrylamide(C-EGF25)

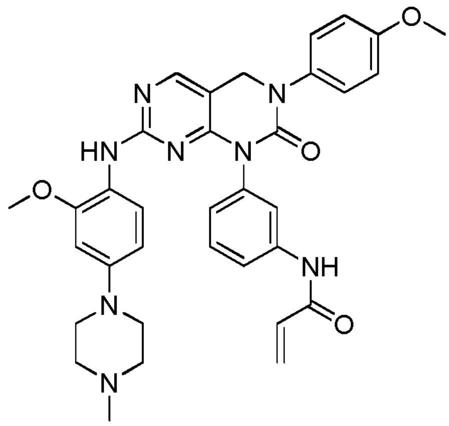

This compound was synthesized with similar procedures to that of example 4.
$^1$H NMR (400Hz, DMSO- $d_6$) δ 10.33 (s, 1H), 8.15 (s, 1H), 7.81 (d, *J*= 7.2 Hz, 1H), 7.66 (s, 1H), 7.56 (s, 1H), 7.45 *(t, J* = 8.0 Hz, 1H), 7.36 (d, *J* = 8.4 Hz, 2H), 7.30 (d, *J* = 8.8 Hz, 1H), 7.05 (d, *J* = 7.6 Hz, 1H), 6.97 (d, *J* = 8.4 Hz, 2H), 6.52 (s, 1H), 6.44 (dd, *J* = 10.0, 17.2 Hz, 1H), 6.26 (d, *J* = 16.8 Hz, 1H), 6.05 (s, 1H), 5.76 (d, *J* = 9.6 Hz, 1H), 4.81 (s, 2H), 3.77 (s, 6H), 3.01 (m, 4H), 2.44 (m, 4H), 2.23 (s, 3H).
LCMS (ESI): m/z 621.2 $[M + H]^+$.

Example 20

N-(3-(3-cyclopropyl-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF26)

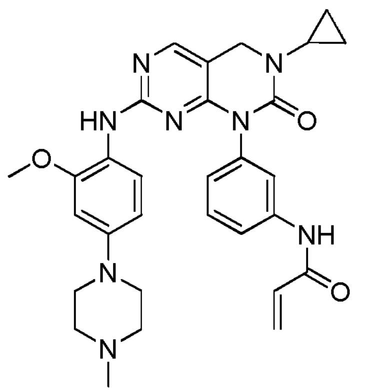

This compound was synthesized with similar procedures to that of example 4.
$^1$H NMR (400Hz, DMSO- $d_6$) δ 10.29 (s, 1H), 8.12 (s, 1H), 7.81 (d, *J*= 8.4 Hz, 1H), 7.56 (s, 1H), 7.48 (s, 1H), 7.43 (t, *J* = 8.0 Hz, 1H), 7.27 (d, *J* = 8.8 Hz, 1H), 6.96 (d, *J* = 8.0 Hz, 1H), 6.50 (d, *J* = 2.4 Hz, 1H), 6.43 (dd, *J* = 10.0, 16.8 Hz, 1H), 6.25 (dd, *J* = 2.0, 16.8 Hz, 1H), 6.03 (d, *J* = 7.6 Hz, 1H), 5.76 (dd, *J* = 2.0, 10.0 Hz, 1H), 4.43 (s, 2H), 3.76 (s, 3H), 2.99 (t, *J* = 4.8 Hz, 4H), 2.65-2.70 (m, 1H), 2.42 (t, *J* = 4.8 Hz, 4H), 2.21 (s, 3H), 0.73-0.79 (m, 2H), 0.66-0.70 (m, 2H).
LCMS (ESI): m/z 555.2 $[M + H]^+$.

Example 21

N-(3-(3-methyl-7-(4-(4-methylpiperazin-1-yl)phenylamino)-2-oxo-3,4-dihydropyrimido [4,5-d]pyrimidin-1(2H)-yl)phenyl)acrylamide(x001)

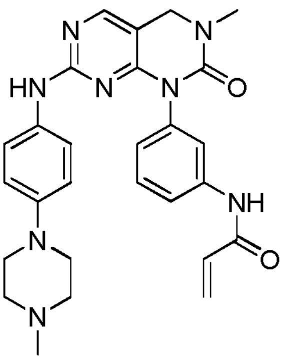

This compound was synthesized with similar procedures to that of example 4. $^{1}H$ NMR (400 MHz, DMSO) δ 10.31 (s, 1H), 9.17 (s, 1H), 8.13 (s, 1H), 7.84 (d, *J* = 8.0 Hz, 1H), 7.56 (s, 1H), 7.44 *(t, J* = 8.0 Hz, 1H), 7.12 *(d, J* = 7.2 Hz, 2H), 6.96 *(d, J* = 7.6 Hz, 1H), 6.53 (d, *J* = 8.0 Hz, 2H), 6.43 (dd, *J* = 16.4, 9.6 Hz, 1H), 6.25 (d, *J* = 16.4 Hz, 1H), 5.76 (d, *J* = 10.0 Hz, 1H), 4.46 (s, 2H), 2.97 (s, 3H), 2.92 (s, 4H), 2.41 (s, 4H), 2.20 (s, 3H).
LCMS (ESI): m/z 499.2 $[M + H]^+$.

Example 22

N-(3-(3-methyl-2-oxo-7-(4-(piperidin-1-yl)phenylamino)-3,4-dihydropyrimido[4,5-d]pyrimidin-1(2H)-yl)phenyl)acrylamide(x002)

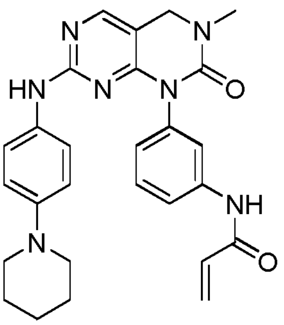

This compound was synthesized with similar procedures to that of example 4.
$^{1}H$ NMR (400 MHz, DMSO) δ 10.31 (s, 1H), 9.16 (s, 1H), 8.12 (s, 1H), 7.85 (d, *J* = 7.2 Hz, 1H), 7.56 (s, 1H), 7.44 *(t, J* = 8.0 Hz, 1H), 7.10 *(d, J* = 7.6 Hz, 2H), 6.96 *(d, J* = 7.2 Hz, 1H), 6.52 (d, *J*= 8.0 Hz, 2H), 6.43 (dd, *J*= 16.8, 9.6 Hz, 1H), 6.25 (d, *J*= 17.6 Hz, 1H), 5.75 (d, *J*= 10.0 Hz, 1H), 4.45 (s, 2H), 2.96 (s, 3H), 2.90 (s, 4H), 1.57 (s, 4H), 1.47 (d, *J*= 5.2 Hz, 2H).
LCMS (ESI): m/z 484.1 $[M + H]^+$.

Example 23

N-(3-(3-methyl-7-(4-(4-(4-methylpiperazin-1-yl)piperidin-1-yl)phenylamino)-2-oxo-3,4-dihydropyrimido[4,5-d]pyrimidin-1(2H)-yl)phenyl)acrylamide(x003)

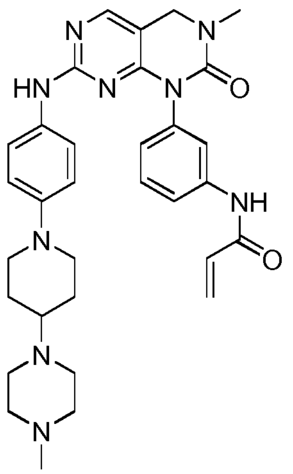

This compound was synthesized with similar procedures to that of example 4.
$^1$H NMR (400 MHz, DMSO) δ 10.31 (s, 1H), 9.15 (s, 1H), 8.12 (s, 1H), 7.85 (d, *J* = 8.8 Hz, 1H), 7.55 (s, 1H), 7.43 *(t, J* = 8.0 Hz, 1H), 7.10 *(d, J* = 8.4 Hz, 2H), 6.96 *(d, J* = 8.4 Hz, 1H), 6.53 (d, *J*= 8.0 Hz, 2H), 6.43 (dd, *J*= 17.0, 9.0 Hz, 1H), 6.23-6.27 (m, 1H), 5.76 (d, *J* = 11.6 Hz, 1H), 4.45 (s, 2H), 3.47 (d, *J* = 11.6 Hz, 2H), 3.33 (s, 3H), 2.97 (s, 3H), 2.33-2.50 (m, 7H), 2.21 (s, 4H), 1.79 (d, *J*= 11.6 Hz, 2H), 1.41-1.49 (m, 2H).
LCMS (ESI): m/z 582.2 $[M + H]^+$.

Example 24

N-(3-(7-(4-(4-(dimethylamino)piperidin-1-yl)phenylamino)-3-methyl-2-oxo-3,4-dihydropyrimido[4,5-d]pyrimidin-1(2H)-yl)phenyl)acrylamide(x004)

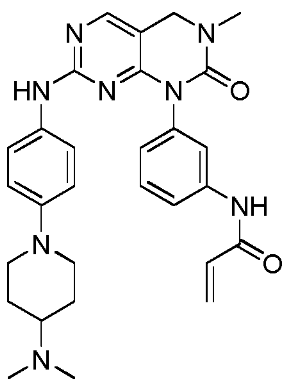

This compound was synthesized with similar procedures to that of example 4.
$^1$H NMR (400 MHz, DMSO) δ 10.31 (s, 1H), 9.17 (s, 1H), 8.12 (s, 1H), 7.85 (d, *J* = 7.6 Hz, 1H), 7.55 (s, 1H), 7.44 *(t, J* = 8.0 Hz, 1H), 7.10 *(d, J* = 6.0 Hz, 2H), 6.96 *(d, J* = 7.6 Hz, 1H), 6.53 (d, *J*= 7.2 Hz, 2H), 6.39 (dd, *J*= 16.8, 10.4 Hz, 1H), 6.25 (d, *J*= 16.4 Hz, 1H), 5.76 (d, *J*= 9.6 Hz, 1H), 4.45 (s, 2H), 3.45 (d, *J*=11.6 Hz, 2H), 2.96 (s, 3H), 2.45 (d, J=11.2 Hz, 2H), 2.18 (s, 7H), 1.78 (d, J=11.2 Hz, 2H), 1.41-1.46 (m, 2H).
LCMS (ESI): m/z 527.2 $[M + H]^+$

Example 25

N-(3-(3-methyl-2-oxo-7-(4-thiomorpholinophenylamino)-3,4-dihydropyrimido[4,5-d]pyrimidin-1(2H)-yl)phenyl)acrylamide(x005)

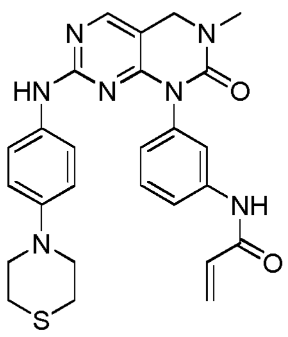

This compound was synthesized with similar procedures to that of example 4.
$^1$H NMR (400 MHz, DMSO) δ 10.31 (s, 1H), 9.20 (s, 1H), 8.13 (s, 1H), 7.84 (d, *J*= 7.6 Hz, 1H), 7.56 (s, 1H), 7.44 (t, *J* = 7.8 Hz, 1H), 7.13 (d, *J* =7.2 Hz, 2H), 6.96 (d, *J* =7.6 Hz, 1H), 6.55 (d, *J* =8.4 Hz, 2H), 6,43 (dd, *J* = 17.0 Hz, 9.8 Hz, 1H), 6.25 (d, *J* = 16.8 Hz, 1H), 5.76 (d, *J* = 10.0 Hz, 1H), 4.46 (s, 2H), 3.25-3.26 (m, 4H), 2.97 (s, 3H), 2.62-2.64 (m, 4H).
LCMS (ESI): m/z 502.1 $[M + H]^+$.

Example 26

N-(3-(7-(2-isopropoxy-4-(4-methylpiperazin-1-yl)phenylamino)-3-methyl-2-oxo-3,4-dihydropyrimido[4,5-d]pyrimidin-1(2H)-yl)phenyl)acrylamide(x006)

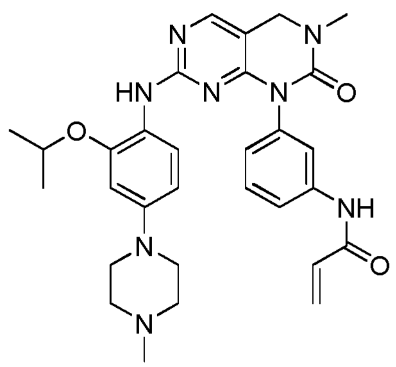

This compound was synthesized with similar procedures to that of example 4.
$^1$H NMR (400 MHz, DMSO) δ 10.30 (s, 1H), 8.13 (s, 1H), 7.84 (d, *J* =8.0 Hz, 1H), 7.57 (s, 1H), 7.44 (t, *J* = 8.0 Hz, 1H), 7.40 (s, 1H), 7.27 (d, *J* =9.2 Hz, 1H), 6.97 (d, *J* =7.6 Hz, 1H), 6.52 (s, 1H), 6.43 (dd, *J* = 10.0, 16.8 Hz, 1H), 6.24 (d, *J* = 16.8 Hz, 1H), 5.98 (d, *J* = 8.0 Hz, 1H), 5.76 (d, *J* = 10.4 Hz, 1H), 4.57-4.63 (m, 1H), 4.47 (s, 2H), 2.97 (m, 7H), 2.41 (m, 4H), 2.21 (s, 3H), 1.23 (d, *J* =6.0 Hz, 6H).
LCMS (ESI): m/z 557.3 $[M + H]^+$.

Example 27

N-(3-(3-methyl-7-(4-morpholinophenylamino)-2-oxo-3,4-dihydropyrimido[4,5-d]pyrimidin-1(2H)-yl)phenyl)acrylamide(x007)

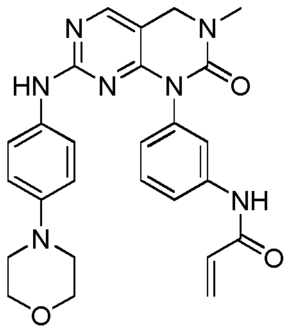

This compound was synthesized with similar procedures to that of example 4.
$^1$H NMR (400 MHz, DMSO) δ 10.31 (s, 1H), 9.19 (s, 1H), 8.13 (s,1H), 7.83 (d, *J*= 8.8 Hz, 1H), 7.56 (s, 1H), 7.44 (t, *J* = 8.0 Hz, 1H), 7.14 (d, *J* = 7.6 Hz, 2H), 6.96 (d, *J* = 7.2 Hz, 1H), 6.54 (d, *J* = 7.6 Hz, 2H), 6.43 (dd, *J* = 17.2, 10.0 Hz, 1H), 6.24 (d, *J* = 17.2 Hz, 1H), 5.76 (d, *J* = 12.0 Hz, 1H), 4.46 (s, 2H), 3.70 (t, *J* = 4.4 Hz, 4H), 2.97 (s, 3H), 2.90 (t, *J* = 4.4 Hz, 4H).
LCMS (ESI): m/z 486.1 $[M + H]^+$.

Example 28

N-(3-(7-(2-methoxy-4-(2-morpholinoethoxy)phenylamino)-3-methyl-2-oxo-3,4-dihydropyrimido[4,5-d]pyrimidin-1(2H)-yl)phenyl)acrylamide(x008)

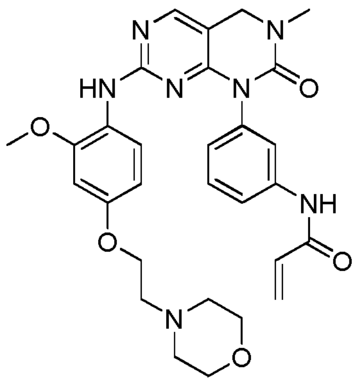

This compound was synthesized with similar procedures to that of example 4.
$^1$H NMR (400 MHz, $CDCl_3$) δ 8.18 (s, 1H), 8.00 (s, 1H), 7.85 (d, *J*= 8.4 Hz, 1H), 7.39-7.43 (m, 4H), 6.99 (d, *J* = 7.6 Hz, 1H), 6.37-6.39 (m, 2H), 6.33 (s, 1H), 6.18-6.24 (m, 1H), 6.07-6.10 (m, 1H), 5.67 (d, J = 10.4 Hz, 1H), 4.45 (s, 2H), 4.10 (s, 2H), 3.80 (s, 4H), 3.77 (s, 3H), 3.09 (s, 3H), 2.85 (s, 2H), 2.68 (s, 4H).
LCMS (ESI): m/z 560.1 $[M + H]^+$.

Example 29

N-(3-(7-(2-ethoxy-4-(4-methylpiperazin-1-yl)phenylamino)-3-methyl-2-oxo-3,4-dihydropyrimido[4,5-d]pyrimidin-1(2H)-yl)phenyl)acrylamide(x009)

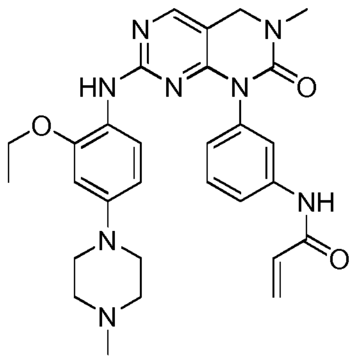

This compound was synthesized with similar procedures to that of example 4.
$^1$H NMR (400 MHz, $CDCl_3$) δ 8.28 (s, 1H), 7.99 (s, 1H), 7.93 (d, *J*= 7.6 Hz, 1H), 7.48 (s, 1H), 7.40 (t, *J* = 8.0 Hz, 2H), 7.28 (s, 1H), 6.96 (d, *J* = 8.0 Hz, 1H), 6.38 (s, 1H), 6.34 (d, *J* = 16.4 Hz, 1H), 6.18 (dd, *J* = 16.8, 10.0 Hz, 1H), 6.10 (d, *J* = 8.4 Hz, 1H), 5.65 (d, *J* = 10.4 Hz, 1H), 4.42 (s, 2H), 4.00 (q, *J* = 13.6, 6.8 Hz, 2H), 3.13 (s, 4H), 3. 06 (s, 3H), 2.70 (s, 4H), 2.44 (s, 3H), 1.39 (t, J = 6.8 Hz, 3H).
LCMS (ESI): m/z 543.2 $[M + H]^+$.

Example 30

N-(3-(3-methyl-7-(2-methyl-4-(4-methylpiperazin-1-yl)phenylamino)-2-oxo-3,4-dihydropyrimido [4,5-d]pyrimidin-1(2H)-yl)phenyl)acrylamide(x010)

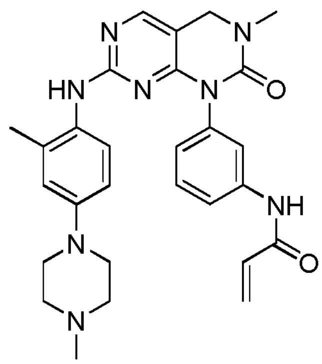

This compound was synthesized with similar procedures to that of example 4.
$^1$H NMR (400 MHz, $CDCl_3$) δ 8.35 (s, 1H), 7.97 (s, 1H), 7.62 (d, *J*= 7.6 Hz, 1H), 7.45 (s, 1H), 7.27-7.33 (m, 2H), 6.91 (d, *J* = 8.0 Hz, 1H), 6.64 (s, 1H), 6.56 (s, 1H), 6.49 (d, *J* = 8.0 Hz, 1H), 6.33 (d, *J* = 16.8 Hz, 1H), 6.17 (dd, *J* = 16.8, 10.0 Hz, 1H), 5.64 (d, *J* = 10.4 Hz, 1H), 4.44 (s, 2H), 3.14 (s, 4H), 3.10 (s, 3H), 2.65 (s, 4H), 2.41 (s, 3H), 2.14 (s, 3H).
LCMS (ESI): m/z 513.2 $[M + H]^+$.

Example 31

N-(3-(7-(3-methoxy-4-(4-methylpiperazin-1-yl)phenylamino)-3-methyl-2-oxo-3,4-dihydropyrimido[4,5-d]pyrimidin-1(2H)-yl)phenyl)acrylamide(x011)

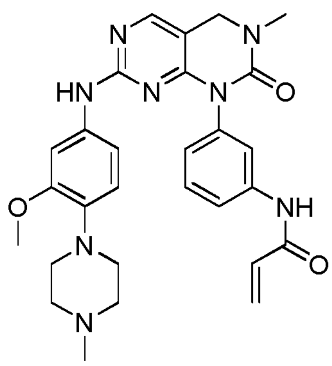

This compound was synthesized with similar procedures to that of example 4.
$^1$H NMR (400 MHz, DMSO) δ 10.30 (s, 1H), 9.13 (s, 1H), 8.15 (s, 1H), 7.79 (d, *J*= 8.4 Hz, 1H), 7.58 (s, 1H), 7.42 (d, *J* = 8.0 Hz, 1H), 6.91-6.97 (m, 2H), 6.86 (s, 1H), 6.39-6.46 (m, 2H), 6.24 (d, *J* = 16.8 Hz, 1H), 5.75 (d, *J* = 10.0 Hz, 1H), 4.46 (s, 2H), 3.58 (s, 3H), 2.96 (s, 3H), 2.79 (s, 4H), 2.39 (s, 4H), 2.19 (s, 3H).
LCMS (ESI): m/z 529.2 $[M + H]^+$.

Example 32

N-(3-(7-(2-methoxy-4-(4-methylpiperazin-1-yl)phenylamino)-2-oxo-3-phenyl-3,4-dihydropyrimido[4,5-d]pyrimidin-1(2H)-yl)phenyl)acrylamide(C-EGF27)

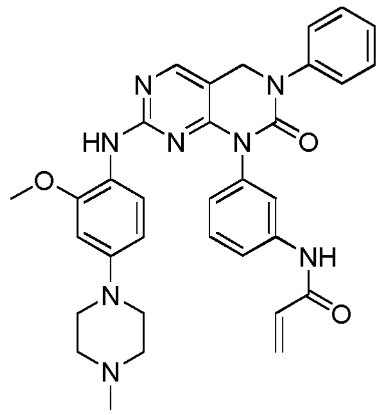

This compound was synthesized with similar procedures to that of example 4.
$^1$H NMR (400 MHz, DMSO) δ 10.32 (s, 1H), 8.17 (s, 1H), 7.82 (d, *J*= 8.0 Hz, 1H), 7.68 (s, 1H), 7.56 (s, 1H), 7.41-7.47 (m, 5H), 7.26-7.32 (m, 2H), 7.07 (d, *J*= 7.6 Hz, 1H), 6.52 (d, *J* = 2.0 Hz, 1H), 6.44 (dd, *J* = 10.0, 16.8 Hz, 1H), 6.26 (dd, *J* = 1.6, 16.8 Hz, 1H), 6.05 (d, *J*= 7.2 Hz, 1H), 5.76 (d, *J*= 11.6 Hz, 1H), 4.87 (s, 2H), 3.77 (s, 3H), 3.01 (m, 4H), 2.43 (m, 4H), 2.22 (s, 3H).
LCMS (ESI): m/z 591.2 $[M + H]^+$.

Example 33

N-(3-(3-benzyl-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF29)

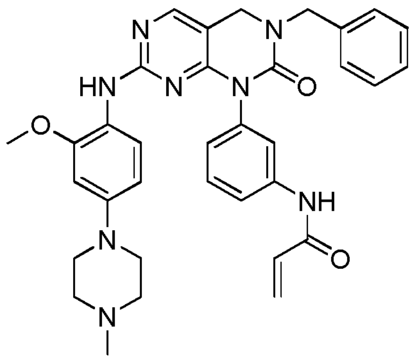

This compound was synthesized with similar procedures to that of example 4.

$^1$H NMR (400 MHz, DMSO) δ 10.30 (s, 1H), 8.08 (s, 1H), 7.84 (d, *J*= 8.0 Hz, 1H), 7.60 (s, 1H), 7.49 (s, 1H), 7.45 (t, *J* = 8.0 Hz, 1H), 7.33-7.41 (m, 4H), 7.26-7.32 (m, 1H), 7.02 (d, *J* = 7.6 Hz, 1H), 6.50 (d, *J* = 2.4 Hz, 1H), 6.43 (dd, *J* = 10.0, 16.4 Hz, 1H), 6.25 (dd, *J* = 2.0, 16.8 Hz, 1H), 6.03 (d, *J* = 6.8 Hz, 1H), 5.76 (dd, *J* = 2.0, 10.0 Hz, 1H), 4.64 (s, 2H), 4.40 (s, 2H), 3.75 (s, 3H), 2.99 (t, *J*= 4.8 Hz, 4H), 2.42 (t, *J*= 4.8 Hz, 4H), 2.21 (s, 3H).

LCMS (ESI): m/z 605.3 $[M + H]^+$.

Example 34

N-(3-(7-(2-methoxy-4-(4-methylpiperazin-1-yl)phenylamino)-2-oxo-3-(4-phenoxyphenyl)-3,4-dihydropyrimido[4,5-d]pyrimidin-1 (2H)-yl)phenyl)acrylamide(C-EGF31)

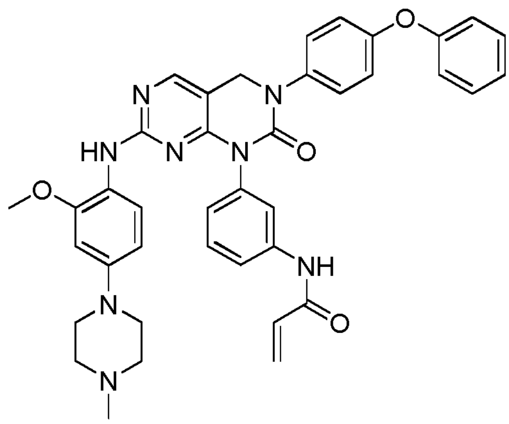

This compound was synthesized with similar procedures to that of example 4.

$^1$H NMR (400 MHz, DMSO) δ 10.32 (s, 1H), 8.17 (s, 1H), 7.81 (d, J= 8.4 Hz, 1H), 7.67 (s, 1H), 7.56 (s, 1H), 7.40-7.47 (m, 5H), 7.30 *(d, J* = 8.8 Hz, 1H), 7.16 *(t, J* = 7.6 Hz, 1H), 7.04-7.07 (m, 5H), 6.52 (d, *J* = 2.4 Hz, 1H), 6.44 (dd, *J* = 10.0, 16.8 Hz, 1H), 6.25 (dd, *J* = 2.0, 16.8 Hz, 1H), 6.04 (s, 1H), 5.76 (dd, *J* = 2.0, 10.0 Hz, 1H), 4.86 (s, 2H), 3.77 (s, 3H), 3.00 (t, J= 4.4 Hz, 4H), 2.43 (t, *J*= 4.4 Hz, 4H), 2.22 (s, 3H).

LCMS (ESI): m/z 683.6 $[M + H]^+$.

Example 35

N-(3-(7-(2-methoxy-4-(4-methylpiperazin-1-yl)phenylamino)-3-(naphthalen-1-yl)-2-oxo-3,4-dihydropyrimido[4,5-d]pyrimidin-1(2H)-yl)phenyl)acrylamide(C-EGF28)

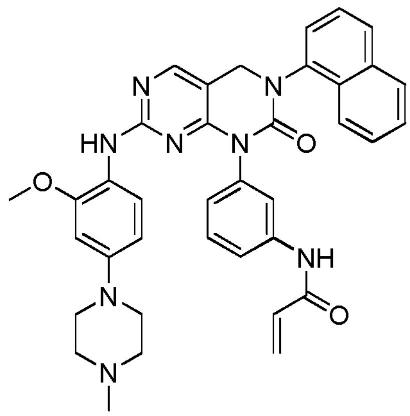

This compound was synthesized with similar procedures to that of example 4.
$^1$H NMR (400 MHz, DMSO) δ 10.34 (s, 1H), 8.21 (s, 1H), 7.93-7.97 (m, 4H), 7.83 (d, *J*= 8.4 Hz, 1H), 7.72 (s, 1H), 7.63 (dd, *J* = 2.0, 8.4 Hz, 1H), 7.59 (s, 1H), 7.50-7.57 (m, 2H), 7.47 (t, *J* = 8.0 Hz, 1H), 7.33 (d, *J* = 8.8 Hz, 1H), 7.11 (d, *J* = 7.6 Hz, 1H), 6.53 (d, *J* = 2.0 Hz, 1H), 6.45 (dd, *J* = 10.0, 16.8 Hz, 1H), 6.26 (dd, *J* = 1.6, 16.8 Hz, 1H), 6.06 (d, *J* = 8.0 Hz, 1H), 5.77 (dd, *J* = 1.6, 10.0 Hz, 1H), 5.01 (s, 2H), 3.78 (s, 3H), 3.01 (m, 4H), 2.44 (m, 4H), 2.22 (s, 3H).
LCMS (ESI): m/z 641.2 $[M + H]^+$.

Example 36

N-(3-(3-(biphenyl-4-yl)-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF30)

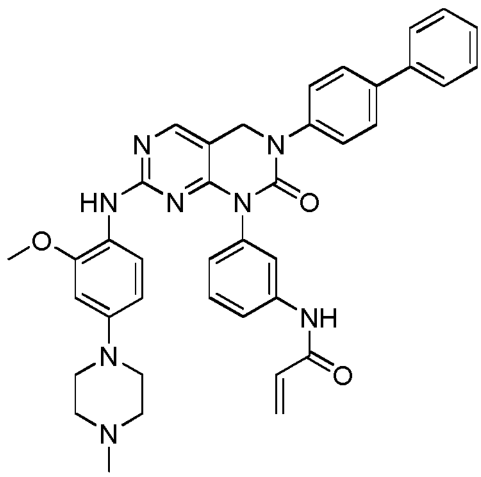

This compound was synthesized with similar procedures to that of example 4.
$^1$H NMR (400 MHz, DMSO) δ 10.33 (s, 1H), 8.19 (s, 1H), 7.83 (d, *J*= 7.6 Hz, 1H), 7.69-7.74 (m, 5H), 7.46-7.58 (m, 6H), 7.37 (t, *J*= 7.2 Hz, 1H), 7.32 (d, *J*= 8.8 Hz, 1H), 7.09 (d, *J* = 7.6 Hz, 1H), 6.52 (s, 1H), 6.45 (dd, *J* = 10.0, 16.4 Hz, 1H), 6.26 (d, *J* = 16.8 Hz, 1H), 6.06 (d, *J*= 6.8 Hz, 1H), 5.77 (d, *J*= 10.0 Hz, 1H), 4.92 (s, 2H), 3.77 (s, 3H), 3.01 (m, 4H), 2.43 (m, 4H), 2.22 (s, 3H).
LCMS (ESI): m/z 667.2 $[M + H]^+$.

Example 37

N-(3-(3-(4-(benzyloxy)phenyl)-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF32)

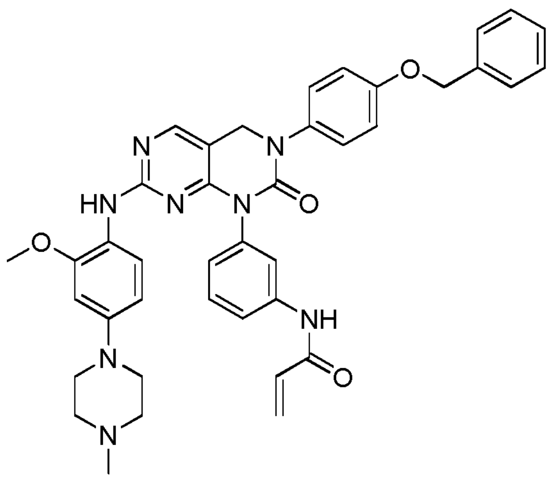

This compound was synthesized with similar procedures to that of example 4.
$^1$H NMR (400 MHz, DMSO) δ 10.31 (s, 1H), 8.15 (s, 1H), 7.81 (d, *J* = 8.4 Hz, 1H), 7.66 (s, 1H), 7.54 (s, 1H), 7.29-7.47 (m, 8H), 7.04-7.07 (m, 3H), 6.52 (d, J= 2.0 Hz, 1H), 6.44 (dd, *J* = 10.0, 16.8 Hz, 1H), 6.25 (dd, *J* = 2.0, 16.8 Hz, 1H), 6.05 (d, *J* = 8.0 Hz, 1H), 5.76 (dd, *J* = 2.0, 10.0 Hz, 1H), 5.13 (s, 2H), 4.81 (s, 2H), 3.77 (s, 3H), 3.00 (t, *J* = 4.4 Hz, 4H), 2.42 (t, *J* = 4.4 Hz, 4H), 2.22(s, 3H).
LCMS (ESI): m/z 698.2 $[M + H]^+$.

Example 38

N-(3-(7-(2-methoxy-4-(4-methylpiperazin-1-yl)phenylamino)-3-methyl-2-oxo-3,4-dihydropyrimido[4,5-d]pyrimidin-1(2H)-yl)phenyl)propionamide(C-EGF34)

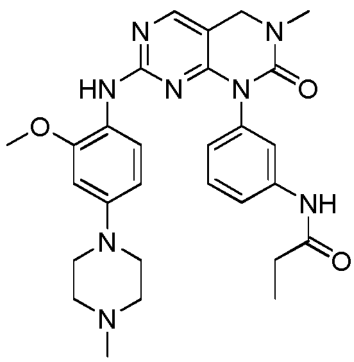

This compound was synthesized with similar procedures to that of example 4.
$^1$H NMR (400 MHz, DMSO) δ 9.99 (s, 1H), 8.10 (s, 1H), 7.69 (d, J= 8.4 Hz, 1H), 7.50 (s, 1H), 7.46 (s, 1H), 7.39 (t, *J* = 8.0 Hz, 1H), 7.26 (d, *J* = 8.4 Hz, 1H), 6.90 (d, *J* = 8.0 Hz, 1H), 6.51 (d, *J* = 2.4 Hz, 1H), 6.03 (d, *J* = 7.6 Hz, 1H), 4.45 (s, 2H), 3.76 (s, 3H), 3.01 (t, *J* = 4.4 Hz, 4H), 2.96 (s, 3H), 2.43 (t, *J* = 4.4 Hz, 4H), 2.30 (q, *J*= 7.2, 14.8 Hz, 2H), 2.22 (s, 3H), 1.06 (t, *J* = 7.2 Hz, 3H).
LCMS (ESI): m/z 531.2 $[M + H]^+$.

Example 39

1-(3-aminophenyl)-7-(2-methoxy-4-(4-methylpiperazin-1-yl)phenylamino)-3-methyl-3,4-dihydropyrimido[4,5-d]pyrimidin-2(1H)-one(C-EGF33)

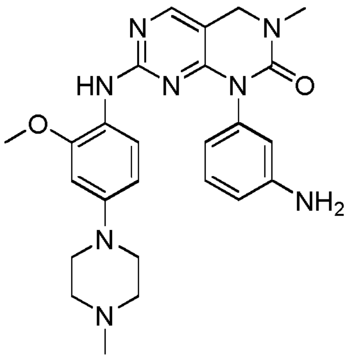

This compound was synthesized with similar procedures to that of example 4.
$^1H$ NMR (400Hz, $CDCl_3$) $\delta$ 7.99 (s, 1H), 7.61 (s, 1H ), 7.46 (s, 1H), 7.28 (t, J = 8.0 Hz, 2H), 6.78 (dd, J = 2.0, 8.0 Hz, 1H), 6.67 (d, J = 7.6 Hz, 1H), 6.61 (t, J = 2.0 Hz, 1H), 6.45 (d, J = 2.4 Hz, 1H), 6.21 (d, J = 7.6 Hz, 1H), 4.42 (s, 2H), 3.81 (s, 3H), 3.13 (t, J = 4.8 Hz, 4H), 3.09 (s, 3H), 2.64 (t, J = 4.8 Hz, 4H), 2.29 (s, 3H).
LCMS (ESI): m/z 475.2 $[M + H]^+$.

Example 40

N-(3-(7-(cyclopropylamino)-3-methyl-2-oxo-3,4-dihydropyrimido[4,5-d]pyrimidin-1(2H)-yl)phenyl)acrylamide(FLB-5-0226)

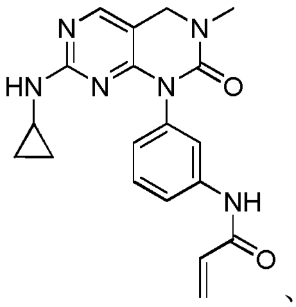

This compound was synthesized with similar procedures to that of example 1.
$^1H$ NMR (400 MHz, $CDCl_3$) $\delta$ 8.34 (s, 1H), 8.00 (s, 1H), 7.50 (s, 1H), 7.34 (d, *J*= 8.4 Hz, 1H), 7.22-7.26 (m, 1H), 6.86 (d, *J* = 6.4 Hz, 1H), 6.32 (d, *J* = 16.8 Hz, 1H), 6.09 (dd, *J* = 10.0, 16.8 Hz, 1H), 5.63 (d, *J* = 10.4 Hz, 1H), 5.14 (s, 1H), 4.43 (s, 2H), 3.10 (s, 3H), 2.51 (m, 1H), 0.61 (m, 2H), 0.37 (m, 2H).
LCMS (ESI): m/z 365.2 $[M + H]^+$

Example 41

N-(3-(7-(isopropylamino)-3-methyl-2-oxo-3,4-dihydropyrimido[4,5-d]pyrimidin-1(2H)-yl)phenyl)acrylamide(FLB-5-0227)

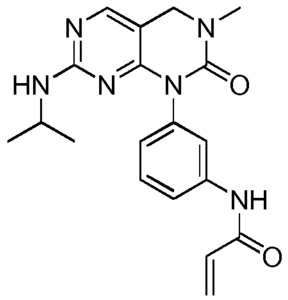

This compound was synthesized with similar procedures to that of example 1.
$^1H$ NMR (400 MHz, $CDCl_3$) δ 8.21 (s, 1H), 7.91 (s, 1H), 7.43-7.46 (m, 2H), 7.25-7.29 (m, 1H), 6.88 (d, *J* = 7.6 Hz, 1H), 6.33 (d, *J* = 16.8 Hz, 1H), 6.10 (dd, *J* = 10.4, 17.2 Hz, 1H), 5.64 (d, *J* = 11.2 Hz, 1H), 4.78 (d, *J* = 6.4 Hz, 1H), 4.41 (s, 2H), 3.48 (m, 1H), 3.10 (s, 3H), 1.02 (d, 6H).
LCMS (ESI): m/z 367.2 $[M + H]^+$

Example 42

N-(3-(7-(cyclohexylamino)-3-methyl-2-oxo-3,4-dihydropyrimido[4,5-d]pyrimidin-1(2H)-yl)phenyl)acrylamide(FLB-5-0230)

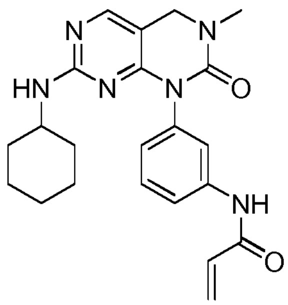

This compound was synthesized with similar procedures to that of example 1.
$^1H$ NMR (400 MHz, $CDCl_3$) δ 8.05 (s, 1H), 7.91 (s, 1H), 7.50 (s, 1H), 7.44 (d, *J*= 8.0 Hz, 1H), 7.28 (t, *J* = 8.0 Hz, 1H), 6.90 (d, *J* = 7.2 Hz, 1H), 6.34 (d, *J* = 17.6 Hz, 1H), 6.12 (dd, *J* = 10.0, 16.8 Hz, 1H), 5.66 (dd, *J* = 1.6, 10.0 Hz, 1H), 4.78 (d, *J* = 7.6 Hz, 1H), 4.40 (s, 2H), 3.10 (s, 3H), 1.75-1.88 (m, 2H), 1.50-1.66 (m, 4H), 1.03-1.23 (m, 4H).

Example 43

N-(3-(7-(ethylamino)-3-methyl-2-oxo-3,4-dihydropyrimido[4,5-d]pyrimidin-1(2H)-yl)phenyl)acrylamide(FLB-5-0231)

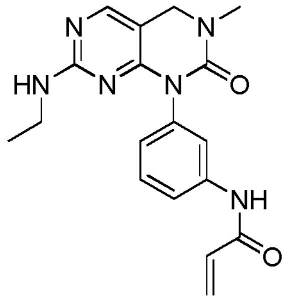

This compound was synthesized with similar procedures to that of example 1.

$^1$H NMR (400Hz, $CDCl_3$) δ 8.47 (s, 1H), 7.92 (s, 1H), 7.49 (s, 1H), 7.35 (d, *J* = 8.8 Hz, 1H), 7.23 *(t, J* = 8.0 Hz, 1H), 6.85 (d, *J* = 7.6 Hz, 1H), 6.32 (dd, *J* = 6.8, 16.8 Hz, 1H), 6.08-6.14 (m, 1H), 5.61 (d, *J* = 10.4 Hz, 1H), 4.91 (m, 1H), 4.41(s, 2H), 3.07-3.18 (m, 5H), 0.85 (t, *J= 7.2* Hz, 3H).
LCMS (ESI): m/z 353.1 $[M + H]^+$

Example 44

N-(3-(3-methyl-7-(2-morpholinoethylamino)-2-oxo-3,4-dihydropyrimido[4,5-d]pyrimidin-1(2H)-yl)phenyl)acrylamide(FLB-5-0235)

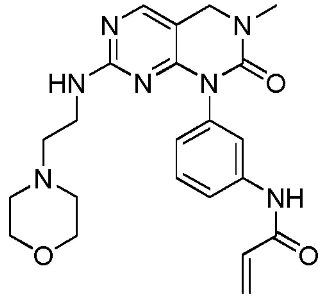

This compound was synthesized with similar procedures to that of example 1. LCMS (ESI): m/z 438.1 $[M + H]^+$

Example 45

N-(3-(7-(benzylamino)-3-methyl-2-oxo-3,4-dihydropyrimido[4,5-d]pyrimidin-1(2H)-yl)phenyl)acrylamide(FLB-5-0238)

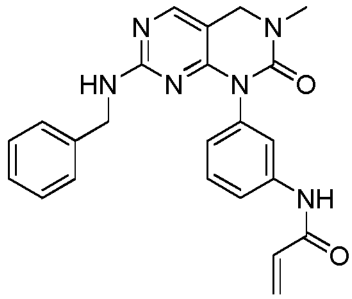

This compound was synthesized with similar procedures to that of example 1.
LCMS (ESI): m/z 415.1 $[M + H]^+$

Example 46

N-(3-(3-(3-chlorophenyl)-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF35)

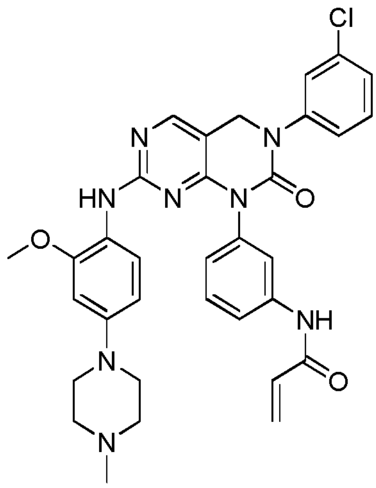

This compound was synthesized with similar procedures to that of example 4.
$^1$H NMR (400 MHz, DMSO) δ 10.32 (s, 1H), 8.17 (s, 1H), 7.82 (d, J = 6.8 Hz, 1H), 7.68 (s, 1H), 7.58 (s, 2H), 7.45 (m, 3H), 7.34 (d, J = 7.2 Hz, 1H), 7.30 (d, J = 8.8 Hz, 1H), 7.08 (d, J = 7.6 Hz, 1H), 6.52 (s, 1H), 6.47 (dd, J =10.0, 16.8 Hz, 1H), 6.26 (d, J = 16.8 Hz, 1H), 6.06 (s, 1H), 5.76 (d, J = 10.0 Hz, 1H), 4.89 (s, 2H), 3.77 (s, 3H), 3.01 (m, 4H), 2.45 (m, 4H), 2.23 (s, 3H).
LCMS (ESI): m/z 625.2 $[M + H]^+$

Example 47

N-(3-(3-(3-cyanophenyl)-7-(2-methoxy-4-(4-methylpiperazin-1-yl)phenylamino)-2-oxo-3,4-dihydropyrimido[4,5-d]pyrimidin-1(2H)-yl)phenyl)acrylamide(C-EGF36)

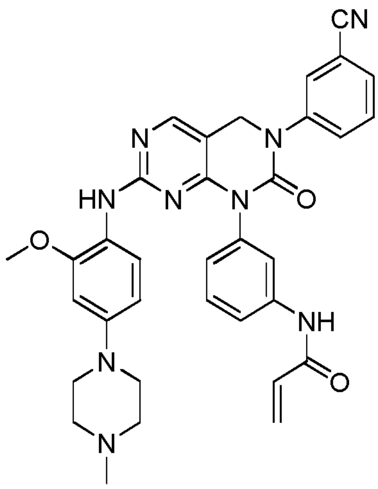

This compound was synthesized with similar procedures to that of example 4.
$^1$H NMR (400Hz, DMSO- d6) δ 10.34 (s, 1H), 8.18 (s, 1H), 7.97 (s, 1H), 7.82 (d, J = 6.4 Hz, 2H), 7.71-7.74 (m, 2H), 7.64 (t, J = 8.0 Hz, 1H), 7.60 (s, 1H), 7.46 (t, J = 8.0 Hz, 1H), 7.30 (d, J = 8.8 Hz, 1H), 7.09 (d, J = 7.6 Hz, 1H), 6.52 (d, J = 2.0 Hz, 1H), 6.47 (dd, J =10.0, 16.8 Hz, 1H), 6.26(d, J = 16.8 Hz, 1H), 6.06 (d, J = 6.4 Hz, 1H), 5.76 (d, J = 11.2 Hz, 1H), 4.92 (s, 2H), 3.77 (s, 3H), 3.01 (m, 4H), 2.43 (m, 4H), 2.22 (s, 3H).
LCMS (ESI): m/z 616.3 $[M + H]^+$

Example 48

N-(3-(7-(2-methoxy-4-(4-methylpiperazin-1-yl)phenylamino)-3-(3-nitrophenyl)-2-oxo-3,4-dihydropyrimido[4,5-d]pyrimidin-1(2H)-yl)phenyl)acrylamide(C-EGF37)

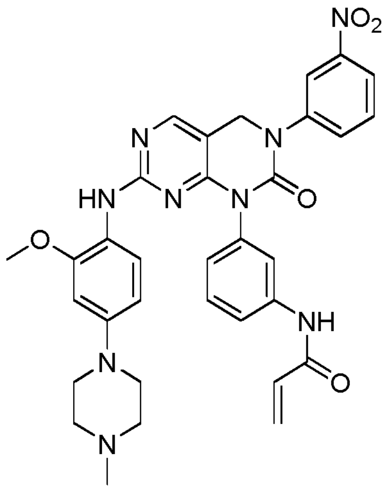

This compound was synthesized with similar procedures to that of example 4.
LCMS (ESI): m/z 636.3 $[M + H]^+$

Example 49

N-(3-(3-(3-chloro-4-fluorophenyl)-7-(2-methoxy-4-(4-methylpiperazin-1-yl)phenylamino)-2-oxo-3,4-dihydropyrimido[4,5-d]pyrimidin-1 (2H)-yl)phenyl)acrylamide(C-EGF3 8)

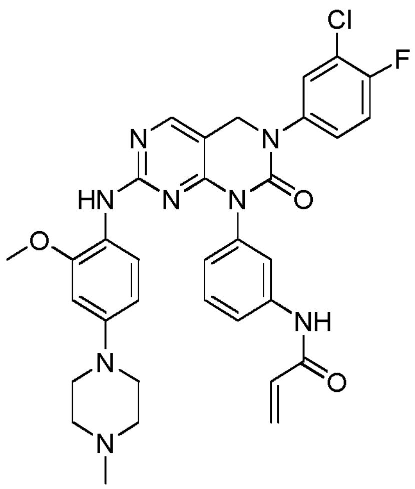

This compound was synthesized with similar procedures to that of example 4.
LCMS (ESI): m/z 643.2 $[M + H]^+$
Example 50

N-(3-(7-(2-methoxy-4-(4-methylpiperazin-1-yl)phenylamino)-2-oxo-3-o-tolyl-3,4-dihydropyrimido[4,5-d]pyrimidin-1(2H)-yl)phenyl)acrylamide(C-EGF39)

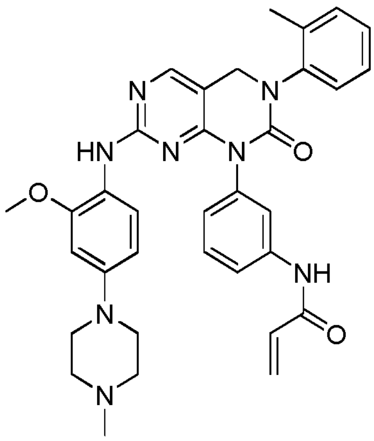

This compound was synthesized with similar procedures to that of example 4.
LCMS (ESI): m/z 605.3 $[M + H]^+$

Example 51

N-(3-(7-(2-methoxy-4-(4-methylpiperazin-1-yl)phenylamino)-3-methyl-2-oxo-3,4-dihydropyrimido[4,5-d]pyrimidin-1(2H)-yl)phenyl)ethenesulfonamide(C-EGF40)

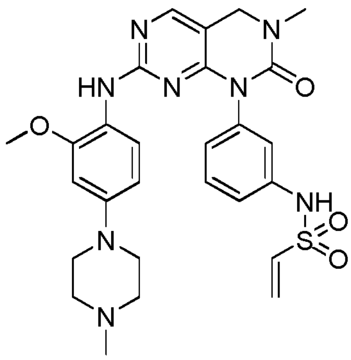

This compound was synthesized with similar procedures to that of example 4.
LCMS (ESI): m/z 565.2 $[M + H]^+$

Example 52

N-(3-(3-benzyl-7-(2-methoxy-4-(4-methylpiperazin-1-yl)phenylamino)-2-oxo-3,4-dihydropyrimido [4,5-d]pyrimidin-1(2H)-yl)-4-fluorophenyl)acrylamide(C-EGF41)

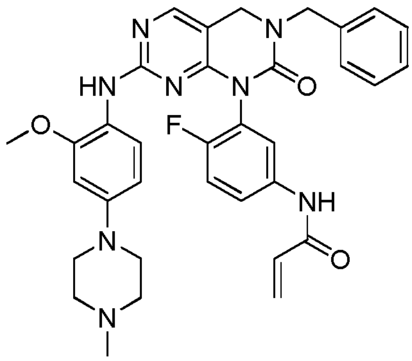

This compound was synthesized with similar procedures to that of example 4.
LCMS (ESI): m/z 623.3 [M + H]$^+$

Example 53

In vitro enzymatic activity assay of 7-(substituted amino)-3,4-dihydropyrimido[4,5-d]pyrimidin-(1H)-ones against the wild type EGFR and T790M mutant EGFR.

In vitro enzymatic activity assay: using Z'-LYTE™ technology(detect fluorescence, coupled-enzyme format, based on the differential sensitivity of phosphorylated and non-phosphorylated peptides to proteolytic cleavage), based on FRET, using Z' LYTE™ FRET peptide substrate, detect the activity of compounds against enzymatic.(invitrogen, Z'-LYTE™ KINASE ASSAY KIT - TYR 2 PEPTIDE, PV3191)EGFR T790M kinase(invitrogen, PV4803) was diluted progressively, then FRET-peptide, ATP and different concentrations of compound are added to the solution. After one hour, site specific protease which can recognize and cleaves non-phosphorylated FRET-peptide. After another hour, using an excitatation wavelength of 400nM excites, then detect the absorption at 445nm and 520nm.

$$\text{Emission Ratio} = \frac{\text{Coumarin Emission (445 nm)}}{\text{Fluorescein Emission (520 nm)}} \qquad \text{\% Phosphorylation} = 1 - \frac{(\text{Emission Ratio} \times \text{F100\%}) - \text{C100\%}}{(\text{C0\%} - \text{C100\%}) + [\text{Emission Ratio} \times (\text{F100\%} - \text{F0\%})]}$$

The inhibition rate was positively correlated with the drug concentration, then make the kinase activity and concentration curves, the $IC_{50}$ will be calucated.

Table 1. kinase activity results of the compounds.

Table 1

| Compd | EGFRWT | EGFRT790M | EGFRL858R | EGFRL861Q | EGFRL858R+T790M |
|---|---|---|---|---|---|
| C-EGF06 | ≤10 nM | ≤10 nM | ≤10 nM | ≤10 nM | ≤10 nM |
| C-EGF08 | 10 nM-100 nM | 10 nM-100 nM | 10 nM-100 nM | 10 nM-100 nM | 10 nM-100 nM |
| C-EGF09 | 10 nM-100 nM | 10 nM-100 nM | 10 nM-100 nM | ≤10 nM | 10 nM-100 nM |
| C-EGF10 | <10 nM | <10 nM | 10 nM-100 nM 1 | 10 nM-100 nM | 10 nM-100 nM |
| C-EGF11 | 10 nM-100 nM | 100 nM-1000 nM | 10 nM-100 nM | <10 nM | 10 nM-100 nM |
| C-EGF12 | 10 nM-100 nM | 100 nM-1000 nM | 10 nM-100 nM | <10 nM | 10 nM-100 nM |

(continued)

| Compd | EGFRWT | EGFRT790M | EGFRL858R | EGFRL861Q | EGFRL858R+T790M |
|---|---|---|---|---|---|
| C-EGF13 | ≤10 nM | ≤10 nM | <10 nM | <10 nM | ≤10 nM |
| C-EGF14 | ≤10 nM | 10 nM-100 nM | ≤10 nM | ≤10 nM | 10 nM-100 nM |
| C-EGF15 | 10 nM-100 nM | 10 nM-100 nM | <10 nM | <10 nM | ≤10 nM |
| C-EGF16 | ≤10 nM | 10 nM-100 nM | ≤10 nM | ≤10 nM | ≤10 nM |
| C-EGF17 | ≤10 nM | ≤10 nM | <10 nM | <10 nM | ≤10 nM |
| C-EGF18 | 1000 nM-10000 nM | ≥10000 nM | ≥10000 nM | >1000 | 1000 nM-10000 nM |
| C-EGF19 | ≥10000 nM | ≥10000 nM | ≥10000 nM | 100 nM-1000 nM | 1000 nM-10000 nM |
| C-EGF20 | ≤10 nM | ≤10 nM | <10 nM | <10 nM | ≤10 nM |
| C-EGF21 | 100 nM-1000 nM | >10000 nM | 100 nM-1000 nM | 1000 nM-10000 nM | 100 nM-1000 nM |
| C-EGF22 | ≤10 nM | 10 nM-100 nM | ≤10 nM | ≤10 nM | 10 nM-100 nM |
| C-EGF23 | ≤10 nM | 10 nM-100 nM | ≤10 nM | ≤10 nM | 10 nM-100 nM |
| C-EGF24 | ≤10 nM | ≤10 nM | <10 nM | <10 nM | ≤10 nM |
| C-EGF25 | ≤10 nM | ≤10 nM | <10 nM | <10 nM | ≤10 nM |
| C-EGF26 | ≤10 nM | ≤10 nM | <10 nM | <10 nM | ≤10 nM |
| x001 | ≤10 nM | ≤10 nM | <10 nM | <10 nM | ≤10 nM |
| X002 | ≤10 nM | ≤10 nM | <10 nM | <10 nM | ≤10 nM |
| X003 | ≤10 nM | ≤10 nM | <10 nM | <10 nM | ≤10 nM |
| X004 | ≤10 nM | ≤10 nM | ≤10 nM | ≤10 nM | ≤10 nM |
| X005 | ≤10 nM | ≤10 nM | ≤10 nM | ≤10 nM | ≤10 nM |
| X006 | ≤10 nM | 10 nM-100 nM | 10 nM-100 nM | 10 nM-100 nM | 10 nM-100 nM |
| X007 | ≤10 nM | ≤10 nM | ≤10 nM | ≤10 nM | ≤10 nM |
| X008 | ≤10 nM | 10 nM-100 nM | ≤10 nM | ≤10 nM | ≤10 nM |
| X009 | ≤10 nM | 10 nM-100 nM | ≤10 nM | ≤10 nM | ≤10 nM |
| X010 | ≤10 nM | ≤10 nM | <10 nM | <10 nM | ≤10 nM |
| X011 | ≤10 nM | ≤10 nM | <10 nM | <10 nM | ≤10 nM |
| C-EGF27 | ≤10 nM | ≤10 nM | <10 nM | <10 nM | ≤10 nM |
| C-EGF28 | ≤10 nM | ≤10 nM | <10 nM | <10 nM | ≤10 nM |
| C-EGF29 | ≤10 nM | ≤10 nM | <10 nM | <10 nM | ≤10 nM |
| C-EGF30 | ≤10 nM | ≤10 nM | <10 nM | <10 nM | ≤10 nM |
| C-EGF31 | ≤10 nM | ≤10 nM | <10 nM | <10 nM | ≤10 nM |
| C-EGF32 | ≤10 nM | ≤10 nM | <10 nM | <10 nM | ≤10 nM |
| C-EGF33 | 100 nM-1000 nM | 1000 nM-10000 nM | 100 nM-1000 nM | 1000 nM-10000 nM | 1000 nM-10000 nM |
| C-EGF34 | 1000 nM-10000 nM | 1000 nM-10000 nM | 1000 nM-10000 nM | 1000 nM-10000 nM | 1000 nM-10000 nM |

(continued)

| Compd | EGFRWT | EGFRT790M | EGFRL858R | EGFRL861Q | EGFRL858R+T790M |
|---|---|---|---|---|---|
| C-EGF35 | ≤10 nM | ≤10 nM | ≤10 nM | ≤10 nM | ≤10 nM |
| C-EGF36 | ≤10 nM | ≤10 nM | ≤10 nM | ≤10 nM | ≤10 nM |
| C-EGF37 | ≤10 nM | 10 nM-100 nM | ≤10 nM | ≤10 nM | 10 nM-100 nM |
| C-EGF38 | ≤10 nM | ≤10 nM | ≤10 nM | ≤10 nM | ≤10 nM |
| C-EGF39 | ≤10 nM | ≤10 nM | ≤10 nM | ≤10 nM | ≤10 nM |
| C-EGF40 | ≤10 nM | 10 nM-100 nM | ≤10 nM | ≤10 nM | 10 nM-100 nM |
| C-EGF41 | ≤10 nM | ≤10 nM | ≤10 nM | ≤10 nM | ≤10 nM |

In the enzymatic activity assay of 7-(substituted amino)-3,4-dihydropyrimido[4,5-d]pyrimidin-(1H)-ones against the EGFR kinase, due to the presence of the irreversible Michael addition reaction with the protein cysteine sites, some compounds show a relatively good inhibitive activity to both the two kinases. Referring to Figs. 1-22, the compounds C-EGF33, C-EGF34 not containing Michael addition reaction sites show a property of an reversible inhibitor that the inhibitive activity at a relatively high test concentration (1μM) for inhibiting a kinase is poor, such compounds need a higher concentration to achieve an inhibition effect; this directly reflects that it is very important to introduce a functional group having unsaturated binding site like acryloyl into such compounds, such that the compounds can display an activity for inhibiting kinase at a relatively low concentration and get a relatively small IC50 value. The $R_1$ of 7-(substituted amino)-3,4-dihydropyrimido[4,5-d]pyrimidin-(1H)-ones plays a key role in the interaction between compounds and protein, especially when $R_1$ is aryl, it can get the best activity. When $R_1$ is aryl, no matter $R_2$ is a relatively small group of alkyl branched chain or a bigger rigid aryl, the activity can be maintained, that is, various types of group can tolerated in the $R_2$ position.

Example 54

Cell Test of EGFR Activity

Detecting the effect of the compounds on cell proliferation by MTT: 1500 cells /well, NCI-H820 (lung cancer cells, EGFR wild type), A431 (Human epidermal carcinoma, EGFR high expression), HCC827 (lung cancer cells, EGFR E746 - A750 deletion), H1975 ((lung cancer cells, EGFR L858R&T790M) were cultured in 96-well plates for 24 h, treated with the compounds in DMSO with various concentrations (DMSO final concentration 1‰, 3-5 parallel controls), and 72 h later, added MTT (5 mg/ml,10 ul/well), and then incubated at 37 °C for 4 h. Supernatant was removed, and 150 uL DMSO was added. OD570 was detected and the data were treated by the software GraphPad Prism 4 Demo. The obtained results show that, the treatment with the 7-(substituted amino)-3,4-dihydropyrimido[4,5-d]pyrimidin-(1H)-one compounds can significantly reduce the MTT absorption of the above cells, which means that the 7-(substituted amino)-3,4-dihydropyrimido[4,5-d]pyrimidin-(1H)-one compounds can significantly inhibit the proliferation of the above cells, particularly inhibit the proliferation of two types of cells namely H1975(L858R/T790) and HCC827(DEL746-750); the inhibition rate was positively correlated with the drug concentration. 50% inhibitory concentration (IC50) values thereof were calculated according to the inhibition effect of the 7-(substituted amino)-3,4-dihydropyrimido[4,5-d]pyrimidin-(1H)-one compounds on the growth of the cells and listed in Table 2. (The compounds used were compounds prepared in Examples 1-31 respectively, and shown as the sequence numbers of the examples in the Table 2).

Table 2

| Compd No | H1975 (μM) | HCC827 (μM) | A431 (μM) | NCI-H820 (μM) |
|---|---|---|---|---|
| C-EGF06 | 0.085 | 0.052 | 2.93 | >100 |
| C-EGF08 | 0.159 | 0.159 | 21.2 | >100 |
| C-EGF09 | 0.379 | 0.212 | 10.7 | >100 |
| C-EGF10 | 0.213 | 0.028 | 1.58 | >100 |
| C-EGF11 | 2.56 | 0.310 | 17.7 | >100 |
| C-EGF12 | 1.53 | 0.409 | 8.16 | 35.6 |

(continued)

| Compd No | H1975 (μM) | HCC827 (μM) | A431 (μM) | NCI-H820 (μM) |
|---|---|---|---|---|
| C-EGF13 | 0.568 | 0.198 | >100 | >100 |
| C-EGF14 | 1.22 | 0.188 | >100 | >100 |
| C-EGF15 | 0.449 | - | - | - |
| C-EGF16 | 0.380 | - | - | - |
| C-EGF17 | 0.710 | 0.062 | 11.1 | >100 |
| C-EGF18 | >100 | >100 | >100 | 4.85 |
| C-EGF19 | 12.1 | >100 | >100 | 95.7 |
| C-EGF20 | 0.143 | - | - | - |
| C-EGF21 | 22.5 | 11.9 | >100 | >100 |
| C-EGF22 | 1.07 | 0.495 | 24.9 | >100 |
| C-EGF23 | 3.86 | 0.616 | 28.2 | >100 |
| C-EGF24 | 0.129 | 0.0002 | 24.7 | >100 |
| C-EGF25 | 0.501 | 0.198 | 1.23 | 54.6 |
| C-EGF26 | 0.146 | 0.007 | 52.6 | >100 |
| x001 | 0.115 | 0.029 | 3.97 | >100 |
| X002 | 0.420 | 0.154 | 8.03 | >100 |
| X003 | 0.179 | 0.125 | 3.44 | >100 |
| X004 | 0.261 | 0.181 | >100 | >100 |
| X005 | 0.333 | 0.023 | 16.2 | >100 |
| X006 | 0.543 | 0.108 | 62.5 | >100 |
| X007 | 0.696 | 0.256 | 3.48 | >100 |
| X008 | 1.10 | 0.179 | 29.9 | >100 |
| X009 | 0.710 | 0.003 | >5.0 | >100 |
| X010 | 0.173 | 0.053 | 1.45 | 41.5 |
| X011 | 0.024 | 0.041 | 0.360 | >100 |
| C-EGF27 | 0.016 | 0.003 | 0.11 | - |
| C-EGF28 | 0.030 | 0.006 | 0.57 | - |
| C-EGF29 | 0.014 | 0.003 | 0.17 | - |
| C-EGF30 | 0.095 | 0.018 | 1.21 | - |
| C-EGF31 | 0.048 | 0.014 | 1.20 | - |
| C-EGF32 | 0.033 | 0.023 | 1.15 | - |
| C-EGF33 | >20 | 4.89 | >30 | - |
| C-EGF34 | >20 | 6.79 | >30 | - |

As can be seen from figure 23, 24, 25 and 26, 3,4-dihydropyrimido[4,5-*d*]pyrimidin-2(1*H*)-one derivatives obviously inhibit the growth of H1975 (L858R/T790M) and HCC827 (DEL 746-750) cell lines, while do not significantly suppress the growth of A431 (High level) and NCI-H820 (WT) at low concentration. In the meantime, we also investigated the cytotoxicity of 3,4-dihydropyrimido[4,5-*d*]pyrimidin-2(1*H*)-one derivatives to the normal human cell, HL7702 (WT), where these compounds displayed low toxicity. These results demonstrated that these 3,4-dihydropyrimido[4,5-*d*]pyrimidin-2(1*H*)-one derivatives can be used to target the gefitinib-resistant lung cells with single or multi mutations, but

show low toxicity to wild-type lung cells or normal cells. Therefore, the selectivity has significant advantage in clinic.

Example 55

Effect of 7-(substituted amino)-3,4-dihydropyrimido[4,5-d]pyrimidin-(1H)-one compounds on H1975 cell apoptosis and cell cycle

Detecting the effect of the compounds on cell apoptosis by Annexin V: $8\times10^5$ cells /well, NCI-H820, A431, HCC827, EGFR E746 - A750 deletion, H1975were plated in 6-well plates, and 24 h later, treated with the compounds at various concentrations and a control (1‰ DMSO) for 24, 48h. Growth medium was collected and cells were trypsined and collected correspondingly to the medium. Suspension (500 g) was centrifuged for 10 minutes. The cells were re-suspended in PBS to get a suspension and then the suspension (500 g) centrifuged for 10 min; this step was repeated twice. The cells were re-suspended in binding buffer, $5\times10^5$-$1\times10^6$ cells/mL. PE-Annexin V (556421, BD Pharmingen) 5 mL respectively. The cells were incubated for 15 min at RT in the dark. The samples were detected with FACS Calibur flow cytometer (Becton Dickinson).

Detecting the effect of the compounds on cell cycle by PI: $8\times10^5$ cells /well, NCI-H820, A431, HCC827, EGFR E746 - A750 deletion, H1975 were plated in 6-well plates, and 24 h later, treated with the compounds at various concentrations and a control (1‰ DMSO) for 24, 48h.. Cells were trypsined, collected and then centrifuged for 10 minutes. The cells were re-suspended in PBS to get a suspension and then the suspension (500 g) centrifuged for 10 min; this step was repeated twice: firstly 300 uL pre-cooled PBS was added and mixed, then 700 uL pre-cooled (i.e. -20 °C) anhydrous ethanol was added and mixed, placed in -20 °C for 24 h. The mixture was 5000rpm centrifuged for 2 min, washed by PBS twice, resuspended with 200 uL PBS (it needs to ensure the cell concentration as about $10^{-6}$), added RNase to achieve the final concentration of 50ug/mL, and trypsined at 37 °C for 30 min. The mixture was added PI achieve the final concentration of 10ug/mL, dyed in dark for 30 min, and then detected within 1 h.

Compound C-EGFR06 can significantly induce cell cycle arrest and cell apoptosisi. The results can be seen in figure 27, 28 and 29.

Example 56

The Effects of 7-(substituted amino)-3,4-dihydropyrimido[4,5-d]pyrimidin-(1H)-one compounds on Cell Signaling Pathway in H1975 Cell

Conventional Western Blot (Immune blotting method) was used, wherein the method includes four steps: sample preparation, electrophoretic separation, film transfer of protein, and immuno hybridization and color display (protein detection).

Sample preparation

1. culturing cells or treating the cells with drugs;
2. discarding mediums, washing the cells with 1X PBS twice to completely remove the mediums;
3. adding 1X SDS sample buffer (6-well plate, 100 μl /w or 75 $cm^2$ plate, 500-1000 μl/bottle), scrapping the cells and transferring them to Ep tube;
4. ultrasonic shearing DNA for 10-15 seconds to reduce the viscosity of the sample;
5. boiling the sample for 5 minutes;
6. centrifuging 12000 g sample for 5 minutes and taking the supernatant.

Electrophoretic separation: Taking 15-20 μL supernatant to SDS-PAGE gel (10 cm x 10 cm) to electrophoretic separate.

Electrophoretic separation (pleaes refer to SDS-PAGE Electrophoretic separation method) Film transfer

1. immersing the gel in a transferring buffer to balance for 10 min;
2. clipping 6 sheets of film and filter paper according to the size of the gel, putting the sheets into the transferring buffer to balance for 10 min, wherein PVDF film should be immersed in pure methanol for 3-5 seconds;
3. installing a transfer sandwich: sponge→3 layers of filter paper→gel→film→3 layers of filter paper→sponge, driving away bubbles when each layer is placed;
4. putting a transfer tank in ice bath, placing the sandwich in (with black surface facing black surface), adding the transfer buffer, plugging electrodes with 100 V, 1 h (the current is 0.3 A); when the film transfer is completed, shutting

off the power supply and taking out the hybrid film.

Immuno hybridization and color display

1. washing the film with 25 mL TBS for 5 min at RT, shaking;
2. placing the film in 25 mL sealed buffer for 1 h at RT, shaking;
3. washing the film with 15 mL TBS/T for six times (5 min/T);
4. adding a primary antibody with appropriate dilution, incubating at RT for 1-2 h or overnight at 4 °C;
5. washing the film with 15 mL TBS/T for six times (5 min/T);
6. adding a secondary antibody with appropriate dilution, labeled by AP or HRP, incubating at RT for 1 h, shaking slowly;
7. washing the film with 15 mL TBS/T for three times (5 min/T);
8. washing the film with 15 mL TBS for one time;
9. preparing protein tablet by ECL;
10. developing.

Compound C-EGF06 can significantly induce EGFR phosphorylation arrest, and further block the downstream pathway phosphorylation, as illustrated in Fig. 30.

Those skilled in the art will recognize, or be able to ascertain using no more than routine experimentation, many equivalents of the specific embodiments of the invention described herein. Such equivalents are intended with be encompassed by the following claims.

## Claims

**1.** A compound of formula (I) or (II):

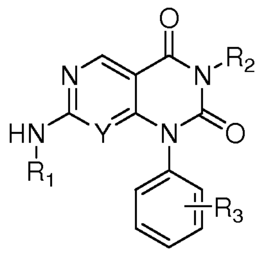
(I)

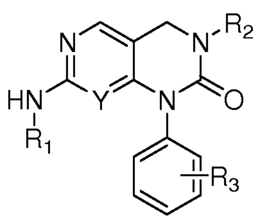
(II)

or pharmaceutically acceptable salts, isomers or prodrugs thereof,
wherein, Y is CH or N;
$R_1$ is selected from:

1) H;
2) $C_1$-$C_5$alkyl;
3) $C_3$-$C_6$ cycloalkyl;
4) $C_1$-$C_5$ fluoroalkyl;
5) $((CH_2)_nX$, n is an integer of 0-6, X is -OH, $-NH_2$, $C_1$-$C_6$ heteroalkyl, or $C_3$-$C_7$ heterocycloalkyl;
6)

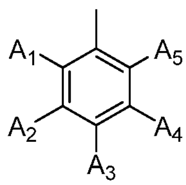
,

wherein each of $A_1$, $A_2$, $A_3$, $A_4$, $A_5$ is independently selected from:

a. H;
b. halo;
c. -CN;
d. $-NO_2$;
e. -OH;
f. $-NH_2$;
g. $C_1$-$C_6$alkyl;
h. $C_3$-$C_6$ cycloalkyl;
i. $C_1$-$C_6$ fluoroalkyl;
j. $C_1$-$C_6$heteroalkyl;
k. $C_1$-$C_7$heterocycloalkyl;
l. ester, amide, sulfone, sulfoxide, urea formed from the above alkyl; wherein, the heteroatom in the above $C_1$-$C_6$ heteroalkyl or $C_1$-$C_7$ heterocycloalkyl is O, N or S;

$R_2$ is selected from:

1) H;
2) $C_1$-$C_5$ alkyl;
3) $C_3$-$C_6$ cycloalkyl;
4) $C_1$-$C_5$ fluoroalkyl;
5) aryl;
6) heterocycloalkyl;
wherein, the heteroatom in the above heterocycloalkyl is O, N or S;

$R_3$ is selected from:

1) H;
2) halo;
3) $-NH_2$, -OH, -CN, $-NO_2$;
4) $C_1$-$C_5$ alkyl;
5) $C_3$-$C_6$ cycloalkyl;
6) aryl;

7) 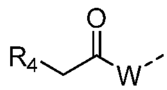 ; 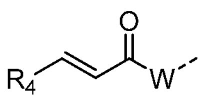 ; 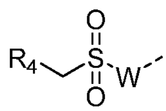 ; 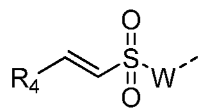 ; 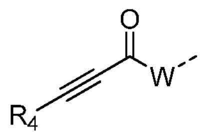 ;

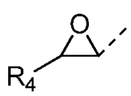 ; 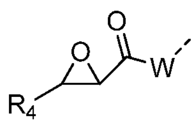 ; 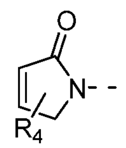 ; 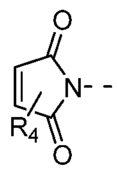 ; 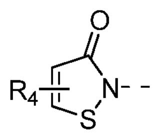 ; 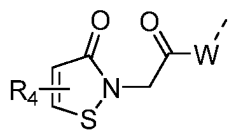 ;

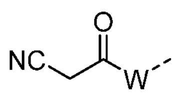 ; 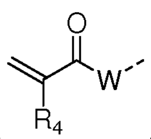 ; 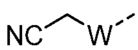 ; 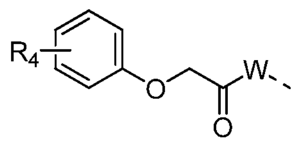 ; 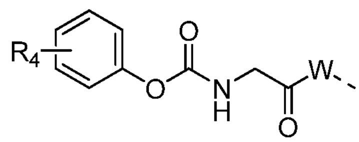 ,

wherein W is selected from $-CH_2$, $-CH_2CH_2$, O, S, -NH, -NR; R is $C_1$-$C_5$ alkyl or aryl;

$R_4$ is selected from:

1) H;
2) halo;
3) $C_1$-$C_5$ alkyl;
4) $C_3$-$C_6$ cycloalkyl;
5) aryl;
wherein, the above $C_3$-$C_6$ cycloalkyl and aryl each can be independently substituted by 0, 1, 2 or 3 substituents selected from $R_5$;

wherein $R_5$ is selected from:

1) H;
2) halo;
3) $C_1$-$C_3$ alkyl;
4) $C_3$-$C_6$ cycloalkyl;
5) $C_1$-$C_3$ alkoxy;
6) $C_1$-$C_3$ fluoroalkyl;
7) heterocycloalkyl;
8) $C_0$-$C_3$ alkylene heterocyclic;
9) phenyl;
wherein, the heteroatom in the above heterocycloalkyl or $C_0$-$C_3$ alkylene heterocyclic is O, N or S.

**2.** The compound of claim 1, wherein the compound has the structure of formula (III)

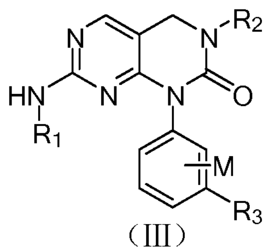

(III)

or the pharmaceutically acceptable salts, isomers or prodrugs thereof,
wherein $R_3$ is defined as in Claim 1,
$R_1$ is selected from:

1) H;
2) methyl, ethyl, n-propyl, isopropyl, n-butyl, isobutyl, t-butyl, n-pentyl, isopentyl, neopentyl;
3) cyclopropyl, cyclobutyl, cyclopentyl, cyclohexyl;
4) $(CH_2)_nX$, wherein, n is an integer of 0-6, X is -OH, $-NH_2$, $-OCH_3$, $-OCH_2CH_3$, $-OCH_2CH_3OCH_3$, $-OCH_2CH_3OCH_2CH_3$, $-SCH_3$, $-N(CH_3)_2$, N-methylpiperazinyl, morpholinyl, thiomorpholinyl, piperdinyl, pyrrolidinyl, 4-N,N-dimethylpiperidinyl, 1-methyl-4-(piperidin-4-yl)piperazinyl, imidazole, 6-(4-methylpiperazine-1-yl)-3-pyridinyl;
5)

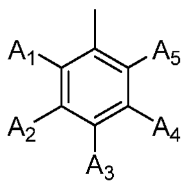
,

wherein each of $A_1$, $A_2$, $A_3$, $A_4$, $A_5$ is independently selected from:

a. H;
b. F, Cl, Br, I;

c. methyl, ethyl, n-propyl, isopropyl, n-butyl, isobutyl, t-butyl, n-pentyl, isopentyl, neopentyl;
d. $-OCH_3$, $-OCH_2CH_3$, $-OCH(CH_3)_2$, $-OC(CH_3)_3$, $-OCH_2CH_3OCH_3$, $-OCH_2CH_3OCH_2CH_3$;
e. $-CF_3$;
f. N,N-dimethylaminoethoxyl, N,N-dimethylaminopropoxyl, 2-(N-methylpiperazine)ethoxyl, 2-(N-acetylpiperazine)ethoxyl, 2-morpholinylethoxyl, 2-thiomorpholinylethoxyl, 2-pyridinylethoxyl, 2-pyrrolidinylethoxyl, N-methylpiperazinyl, morpholinyl, thiomorpholinyl, piperdinyl, pyrrolidinyl, imidazole, 3-N,N-dimethylpyrrolidinyl, 4-N,N-dimethylpyridinyl, 4-acetylpiperazinyl, 1-methyl-4-(piperazine-4-yl)pyridinyl, 4-(4-methylpiperazine-1-yl)methyl, (1-methylpiperidine-4-yl)amino, piperazine-2-one-4-yl, 1-methylpiperazine-2-one-4-yl;
g. the ester, amide, sulfone, sulfoxide, urea formed from the above groups;

$R_2$ is selected from:

1) H;
2) methyl, ethyl, n-propyl, isopropyl, n-butyl, isobutyl, t-butyl, n-pentyl, isopentyl, neopentyl;
3) cyclopropyl, cyclobutyl, cyclopentyl, cyclohexyl;
4) phenyl,4-methoxylphenyl, biphenyl, 4-phenoxylphenyl , 4-benzyloxylphenyl, 2,4-dichlorophenyl, 3-chloro-4-fluorophenyl, single or multiple substituted phenyl, benzyl, substituted benzyl, 1-naphthyl, 2-naphthy, pyridinyl;

the 2, 4, 5 or 6-position of the aromatic ring which contains M is mono or multi-substituted by M, wherein M is selected from:

1) H;
2) halo;
3) -CN;
4) $-NO_2$;
5) -OH;
6) $-NH_2$;
7) $C_1$-$C_6$ alkyl;
8) $C_3$-$C_6$ cycloalkyl;
9) $C_1$-$C_6$ fluoroalkyl;
10) $C_1$-$C_6$ heteroalkyl;
11) $C_1$-$C_7$ heterocycloalkyl;
12) the ester, amide, sulfone, sulfoxide, urea formed from the above groups;
wherein, the heteroatom contained in the above $C_1$-$C_6$ heteroalkyl or $C_1$-$C_7$ heterocycloalkyl is O, NorS.

**3.** The compound of claim 2, wherein the compound has the structure of formula (IV)

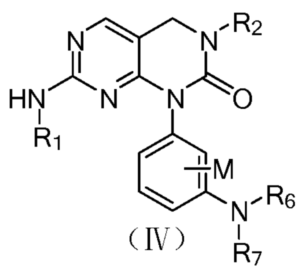

(IV)

or the pharmaceutically acceptable salts, isomers or prodrugs thereof,
wherein, $R_3$, $R_2$, M are defined as in claim 2;
$R_6$, $R_7$, each is independently selected from:

1) H;
2) methyl, ethyl, n-propyl, isopropyl, n-butyl, isobutyl, t-butyl, n-pentyl, isopentyl, neopentyl;
3) cyclopropyl, cyclobutyl, cyclopentyl, cyclohexyl;
4) phenyl;

5) 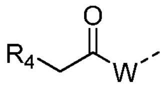 ; 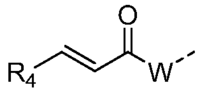 ; 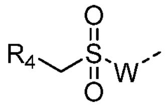 ; 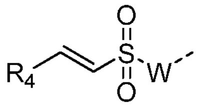 ; 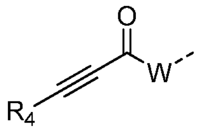 ; 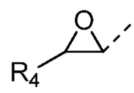 ;

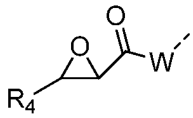 ; 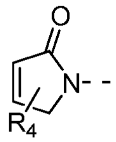 ; 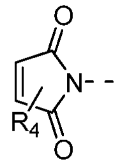 ; 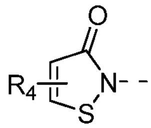 ; 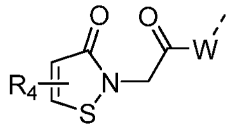 ; 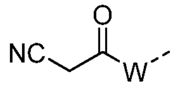 ;

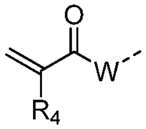 ; 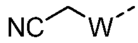 ; 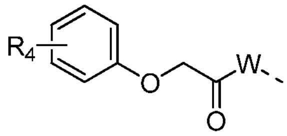 ; 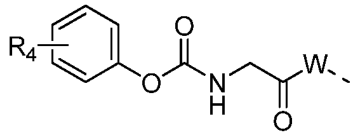 。

W is selected from $-CH_2$, $-CH_2CH_2$, O, S, -NH and -NR; R is methyl, ethyl or phenyl;

$R_4$ is selected from:

1) H;
2) F, Cl, Br, I;
3) methyl, ethyl, n-propyl, isopropyl, n-butyl, isobutyl, t-butyl;
4) cyclopropyl, cyclobutyl, cyclopentyl, cyclohexyl;
5) phenyl, mono- or multi- substituted phenyl.

**4.** The compound of claims 1-3, wherein the compound is selected from:

N-(3-(3-methyl-7-(methylamino)-2-oxo-3,4-dihydropyrimido[4,5-d]pyrimidin-1(2H)-yl) phenyl)acrylamide,
N-(3-(3-methyl-2-oxo-7-(phenylamino)-3,4-dihydropyrimido[4,5-d]pyrimidin-1(2H)-yl)phenyl)a crylamide,
N-(3-(3-methyl-7-(4-methylpiperazin-1-yl)-2-oxo-3,4-dihydropyrimido[4,5-d]pyrimidin-1(2H)-y l)phenyl)acrylamide,
N-(3-(7-(2-methoxy-4-(4-methylpiperazin-1-yl)phenylamino)-3-methyl-2-oxo-3,4-dihydropyrimi do[4,5-d]pyrimidin-1(2H)-yl)phenyl)acrylamide,
N-(3-(7-(2-methoxy-4-(piperidin-1-yl)phenylamino)-3-methyl-2-oxo-3,4-dihydropyrimido[4,5-d] pyrimidin-1(2H)-yl)phenyl)acrylamide,
N-(3-(7-(2-methoxy-4-(pyrrolidin-1-yl)phenylamino)-3-methyl-2-oxo-3,4-dihydropyrimido[4,5-d]pyrimidin-1(2H)-yl)phenyl)acrylamide,
N-(3-(7-(2-methoxy-4-morpholinophenylamino)-3-methyl-2-oxo-3,4-dihydropyrimido[4,5-d]pyr imidin-1 (2H)-yl)phenyl)acrylamide,
N-(3-(7-(2-methoxy-4-thiomorpholinophenylamino)-3-methyl-2-oxo-3,4-dihydropyrimido[4,5-d] pyrimidin-1(2H)-yl)phenyl)acrylamide,
N-(3-(7-(2-methoxy-4-(4-(4-methylpiperazin-1-yl)piperidin-1-yl)phenylamino)-3-methyl-2-oxo-3,4-dihydropyrimido[4,5-d]pyrimidin-1(2H)-yl)phenyl)acrylamide,
N-(3-(7-(4-(4-(dimethylamino)piperidin-1-yl)-2-methoxyphenylamino)-3-methyl-2-oxo-3,4-dihy dropyrimido[4,5-d]pyrimidin-1(2H)-yl)phenyl)acrylamide,
N-(3-(7-(2-methoxy-4-(4-methyl-1,4-diazepan-1-yl)phenylamino)-3-methyl-2-oxo-3,4-dihydrop yrimido[4,5-d] pyrimidin-1(2H)-yl)phenyl)acrylamide,
(R)-N-(3-(7-(4-(3-(dimethylamino)pyrrolidin-1-yl)-2-methoxyphenylamino)-3-methyl-2-oxo-3,4 -dihydropyrimido[4,5-d]pyrimidin-1(2H)-yl)phenyl)acrylamide,
(S)-N-(3-(7-(4-(3-(dimethylamino)pyrrolidin-1-yl)-2-methoxyphenylamino)-3-methyl-2-oxo-3,4-dihydropyrimido [4,5-d]pyrimidin-1(2H)-yl)phenyl)acrylamide,
N-(3-(7-(4-(4-acetylpiperazin-1-yl)-2-methoxyphenylamino)-3-methyl-2-oxo-3,4-dihydropyrimi do[4,5-d]pyrimidin-1(2H)-yl)phenyl)acrylamide,
N-(3-(7-(4-(dimethylamino)-2-methoxyphenylamino)-3-methyl-2-oxo-3,4-dihydropyrimido[4,5-d]pyrimidin-1(2H)-yl)phenyl)acrylamide,

N-(3-(7-(2-fluoro-4-(4-methylpiperazin-1-yl)phenylamino)-3-methyl-2-oxo-3,4-dihydropyrimido [4,5-d]pyrimidin-1(2H)-yl)phenyl)acrylamide,
N-(3-(7-(2-methoxy-4-(2-methoxyethoxy)phenylamino)-3-methyl-2-oxo-3,4-dihydropyrimido[4,5-d]pyrimidin-1(2H)-yl)phenyl)acrylamide,
N-(3-(3-isopropyl-7-(2-methoxy-4-(4-methylpiperazin-1-yl)phenylamino)-2-oxo-3,4-dihydropyri mido[4,5-d]pyrimidin-1(2H)-yl)phenyl)acrylamide,
N-(3-(7-(2-methoxy-4-(4-methylpiperazin-1-yl)phenylamino)-3-(4-methoxyphenyl)-2-oxo-3,4-di hydropyrimido[4,5-d]pyrimidin-1(2H)-yl)phenyl)acrylamide,
N-(3-(3-cyclopropyl-7-(2-methoxy-4-(4-methylpiperazin-1-yl)phenylamino)-2-oxo-3,4-dihydrop yrimido[4,5-d]pyrimidin-1(2H)-yl)phenyl)acrylamide,
N-(3-(3-methyl-7-(4-(4-methylpiperazin-1-yl)phenylamino)-2-oxo-3,4-dihydropyrimido[4,5-d]p yrimidin-1(2H)-yl)phenyl)acrylamide,
N-(3-(3-methyl-2-oxo-7-(4-(piperidin-1-yl)phenylamino)-3,4-dihydropyrimido[4,5-d]pyrimidin-1 (2H)-yl)phenyl)acrylamide,
N-(3-(3-methyl-7-(4-(4-(4-methylpiperazin-1-yl)piperidin-1-yl)phenylamino)-2-oxo-3,4-dihydro pyrimido[4,5-d]pyrimidin-1(2H)-yl)phenyl)acrylamide,
N-(3-(7-(4-(4-(dimethylamino)piperidin-1-yl)phenylamino)-3-methyl-2-oxo-3,4-dihydropyrimid o[4,5-d]pyrimidin-1(2H)-yl)phenyl)acrylamide,
N-(3-(3-methyl-2-oxo-7-(4-thiomorpholinophenylamino)-3,4-dihydropyrimido[4,5-d]pyrimidin-1 (2H)-yl)phenyl)acrylamide,
N-(3-(7-(2-isopropoxy-4-(4-methylpiperazin-1-yl)phenylamino)-3-methyl-2-oxo-3,4-dihydropyri mido[4,5-d]pyrimidin-1(2H)-yl)phenyl)acrylamide,
N-(3-(3-methyl-7-(4-morpholinophenylamino)-2-oxo-3,4-dihydropyrimido[4,5-d]pyrimidin-1(2 H)-yl)phenyl)acrylamide,
N-(3-(7-(2-methoxy-4-(2-morpholinoethoxy)phenylamino)-3-methyl-2-oxo-3,4-dihydropyrimido [4,5-d]pyrimidin-1(2H)-yl)phenyl)acrylamide,
N-(3-(7-(2-ethoxy-4-(4-methylpiperazin-1-yl)phenylamino)-3-methyl-2-oxo-3,4-dihydropyrimid o[4,5-d]pyrimidin-1(2H)-yl)phenyl)acrylamide,
N-(3-(3-methyl-7-(2-methyl-4-(4-methylpiperazin-1-yl)phenylamino)-2-oxo-3,4-dihydropyrimid o[4,5-d]pyrimidin-1(2H)-yl)phenyl)acrylamide,
N-(3-(7-(3-methoxy-4-(4-methylpiperazin-1-yl)phenylamino)-3-methyl-2-oxo-3,4-dihydropyrimi do[4,5-d]pyrimidin-1(2H)-yl)phenyl)acrylamide,
N-(3-(7-(2-methoxy-4-(4-methylpiperazin-1-yl)phenylamino)-2-oxo-3-phenyl-3,4-dihydropyrimi do[4,5-d]pyrimidin-1(2H)-yl)phenyl)acrylamide,
N-(3-(3-benzyl-7-(2-methoxy-4-(4-methylpiperazin-1-yl)phenylamino)-2-oxo-3,4-dihydropyrimi do[4,5-d]pyrimidin-1(2H)-yl)phenyl)acrylamide,
N-(3-(7-(2-methoxy-4-(4-methylpiperazin-1-yl)phenylamino)-2-oxo-3-(4-phenoxyphenyl)-3,4-di hydropyrimido[4,5-d]pyrimidin-1(2H)-yl)phenyl)acrylamide,
N-(3-(7-(2-methoxy-4-(4-methylpiperazin-1-yl)phenylamino)-3-(naphthalen-1-yl)-2-oxo-3,4-dih ydropyrimido[4,5-d]pyrimidin-1(2H)-yl)phenyl)acrylamide,
N-(3-(3-(biphenyl-4-yl)-7-(2-methoxy-4-(4-methylpiperazin-1-yl)phenylamino)-2-oxo-3,4-dihyd ropyrimido[4,5-d]pyrimidin-1(2H)-yl)phenyl)acrylamide,
N-(3-(3-(4-(benzyloxy)phenyl)-7-(2-methoxy-4-(4-methylpiperazin-1-yl)phenylamino)-2-oxo-3, 4-dihydropyrimido[4,5-d]pyrimidin-1(2H)-yl)phenyl)acrylamide,
N-(3-(7-(2-methoxy-4-(4-methylpiperazin-1-yl)phenylamino)-3-methyl-2-oxo-3,4-dihydropyrimi do[4,5-d]pyrimidin-1(2H)-yl)phenyl)propionamide,
1-((3-aminophenyl)-7-(2-methoxy-4-(4-methylpiperazin-1-yl)phenylamino)-3-methyl-3,4-dihydro pyrimido[4,5-d]pyrimidin-2(1H)-one,
N-(3-(7-(cyclopropylamino)-3-methyl-2-oxo-3,4-dihydropyrimido[4,5-d]pyrimidin-1(2H)-yl)phe nyl)acrylamide,
N-(3-(7-(isopropylamino)-3-methyl-2-oxo-3,4-dihydropyrimido[4,5-d]pyrimidin-1(2H)-yl)pheny l)acrylamide,
N-(3-(7-(cyclohexylamino)-3-methyl-2-oxo-3,4-dihydropyrimido[4,5-d]pyrimidin-1(2H)-yl)phen yl)acrylamide,
N-(3-(7-(ethylamino)-3-methyl-2-oxo-3,4-dihydropyrimido[4,5-d]pyrimidin-1(2H)-yl)phenyl)acr ylamide,
N-(3-(3-methyl-7-(2-morpholinoethylamino)-2-oxo-3,4-dihydropyrimido[4,5-d]pyrimidin-(2H) -yl)phenyl)acrylamide,
N-(3-(7-(benzylamino)-3-methyl-2-oxo-3,4-dihydropyrimido[4,5-d]pyrimidin-1(2H)-yl)phenyl)a crylamide,
N-(3-(3-(3-chlorophenyl)-7-(2-methoxy-4-(4-methylpiperazin-1-yl)phenylamino)-2-oxo-3,4-dihy dropyrimido [4,5-d]pyrimidin-1(2H)-yl)phenyl)acrylamide,
N-(3-(3-(3-cyanophenyl)-7-(2-methoxy-4-(4-methylpiperazin-1-yl)phenylamino)-2-oxo-3,4-dihy dropyrimido

[4,5-d]pyrimidin-1(2H)-yl)phenyl)acrylamide,
N-(3-(7-(2-methoxy-4-(4-methylpiperazin-1-yl)phenylamino)-3-(3-nitrophenyl)-2-oxo-3,4-dihyd ropyrimido[4,5-d]pyrimidin-1(2H)-yl)phenyl)acrylamide,
N-(3-(3-chloro-4-fluorophenyl)-7-(2-methoxy-4-(4-methylpiperazin-1-yl)phenylamino)-2-oxo-3, 4-dihydropyrimido[4,5-d]pyrimidin-1(2H)-yl)phenyl)acrylamide,
N-(3-(7-(2-methoxy-4-(4-methylpiperazin-1-yl)phenylamino)-2-oxo-3-o-tolyl-3,4-dihydropyrimi do [4,5-d]pyrimidin-1(2H)-yl)phenyl)acrylamide,
N-(3-(7-(2-methoxy-4-(4-methylpiperazin-1-yl)phenylamino)-3-methyl-2-oxo-3,4-dihydropyrimi do[4,5-d]pyrimidin-1(2H)-yl)phenyl)ethenesulfonamide,
N-(3-(3-benzyl-7-(2-methoxy-4-(4-methylpiperazin-1-yl)phenylamino)-2-oxo-3,4-dihydropyrimi do[4,5-d]pyrimidin-1(2H)-yl)-4-fluorophenyl)acrylamide,
or the pharmaceutically acceptable salts, isomers or prodrugs thereof.

**5.** A pharmaceutical composition comprising of the compound of any one of claims 1-4, or the pharmaceutically acceptable salts, prodrugs or stereoisomers thereof.

**6.** Use of the compound of any one of claims 1-4 or the pharmaceutically acceptable salts, stereoisomers or pro-drugs thereof in the preparation of drugs for treating cancer.

**7.** The use of claim 6, wherein the cancer is any one of non-small cell lung cancer, small cell lung cancer, lung adenocarcinoma, squamous cell lung carcinoma, pancreatic cancer, breast cancer, prostate cancer, liver cancer, skin cancer, squamous cell carcinoma, nasopharyngeal carcinoma, leukemia, histiocytic lymphoma and nasopharyngeal carcinoma.

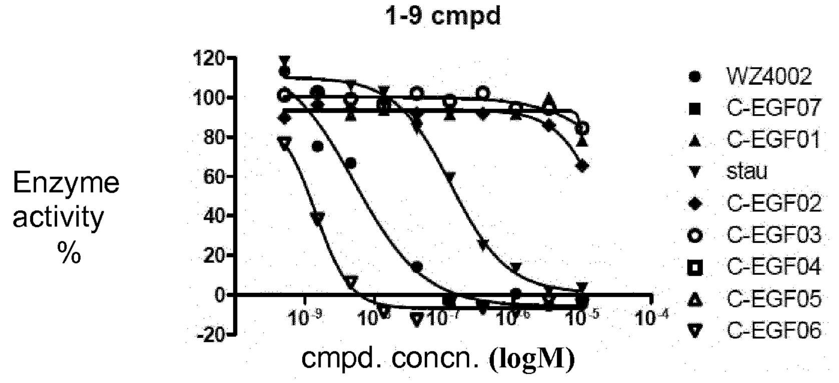


| | WZ4002 | C-EGF07 | C-EGF01 | stau | C-EGF02 | C-EGF03 | C-EGF04 | C-EGF05 | C-EGF06 |
|---|---|---|---|---|---|---|---|---|---|
| EC50 | 5.166e-009 | | ~ 1.154e-005 | 1.285e-007 | ~ 2.332e-005 | ~ 0.0001083 | | | 1.425e-009 |

Fig. 1

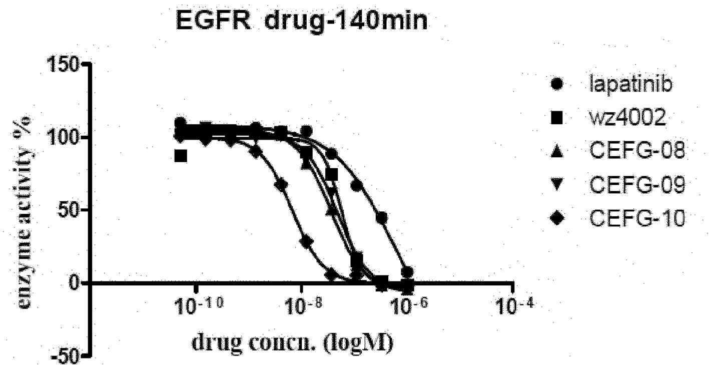


| | lapatanib | wz4002 | CEFG-08 | CEFG-09 | CEFG-10 |
|---|---|---|---|---|---|
| LOGEC50 | -6.250 | -7.240 | -7.439 | -7.325 | -8.175 |
| HILLSLOPE | -0.7557 | -2.363 | -1.367 | -1.436 | -1.468 |
| EC50 | 5.617e-007 | 5.757e-008 | 3.636e-008 | 4.728e-008 | 6.677e-009 |

Fig. 2

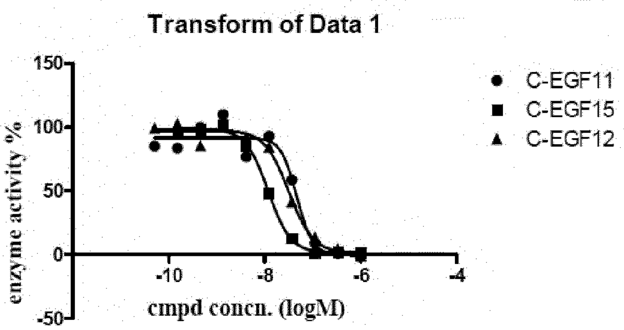


| | C-EGF11 | C-EGF15 | C-EGF12 |
|---|---|---|---|
| EC50 | 4.648e-008 | 1.183e-008 | 3.188e-008 |

Fig. 3

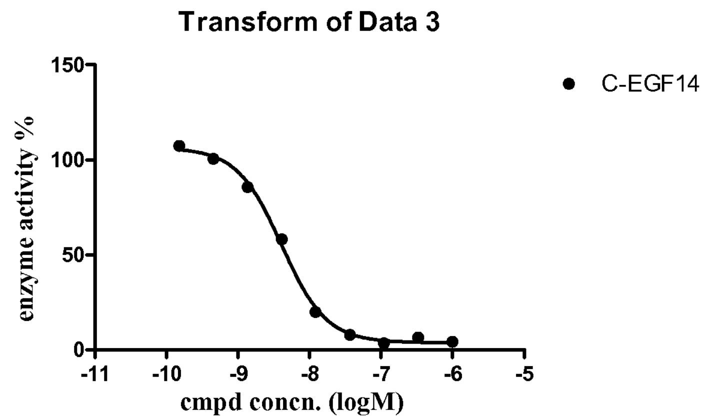


| | C-EGF14 |
|---|---|
| EC50 | 4.116e-009 |

Fig. 4

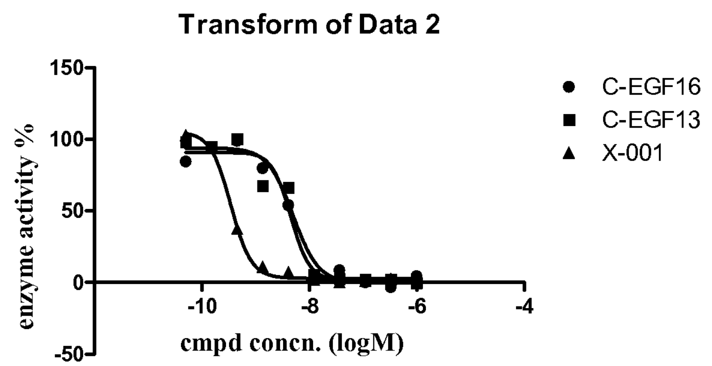


| | C-EGF16 | C-EGF13 | X-001 |
|---|---|---|---|
| EC50 | 4.547e-009 | 5.024e-009 | 3.504e-010 |

Fig. 5

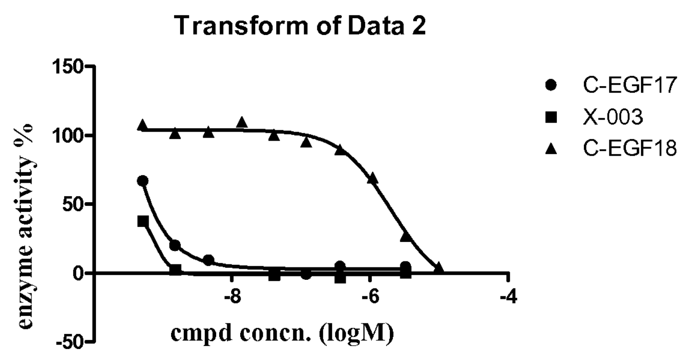


| | C-EGF17 | X-003 | C-EGF18 |
|---|---|---|---|
| EC50 | ~ 1.511e-012 | ~ 7.492e-010 | 2.033e-006 |

Fig. 6

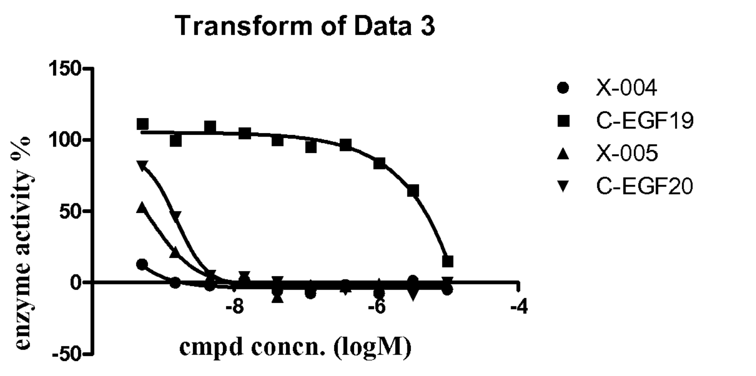


| | X-004 | C-EGF19 | X-005 | C-EGF20 |
|---|---|---|---|---|
| EC50 | ~ 3.892e-012 | ~ 1.392 | 6.568e-010 | 1.548e-009 |

Fig. 7

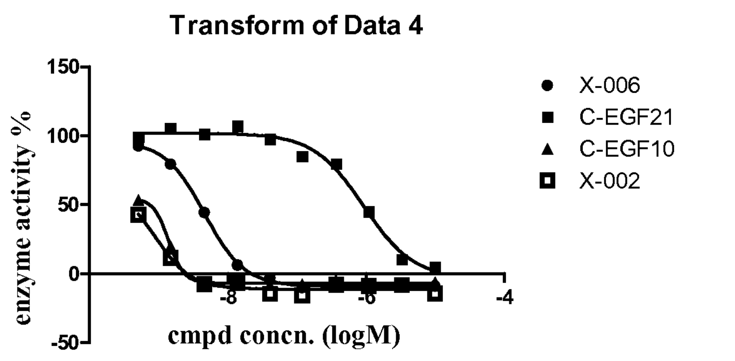


| | X-006 | C-EGF21 | C-EGF10 | X-002 |
|---|---|---|---|---|
| EC50 | 4.637e-009 | 9.293e-007 | 1.402e-009 | 9.146e-010 |

Fig. 8

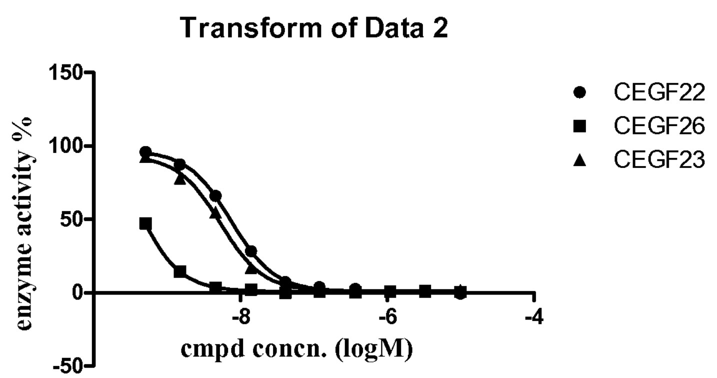


| | CEGF22 | CEGF26 | CEGF23 |
|---|---|---|---|
| EC50 | 7.503e-009 | 3.230e-010 | 5.353e-009 |

Fig. 9

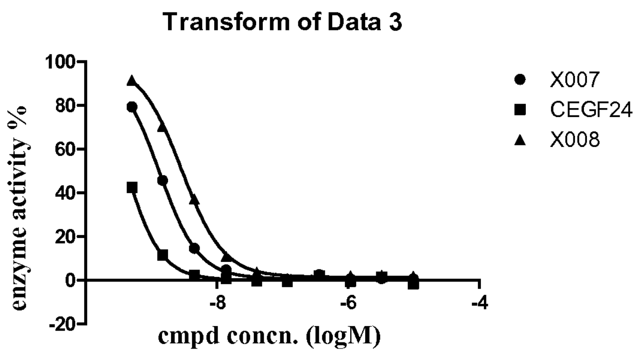


| | X007 | CEGF24 | X008 |
|---|---|---|---|
| EC50 | 1.346e-009 | 3.656e-010 | 2.945e-009 |

Fig. 10

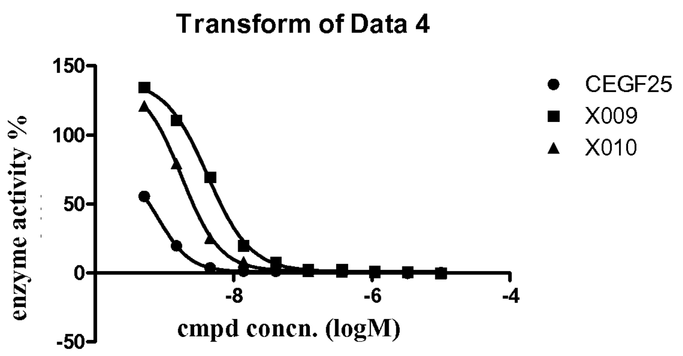


| | CEGF25 | X009 | X010 |
|---|---|---|---|
| EC50 | 8.230e-010 | 4.241e-009 | 1.784e-009 |

Fig. 11

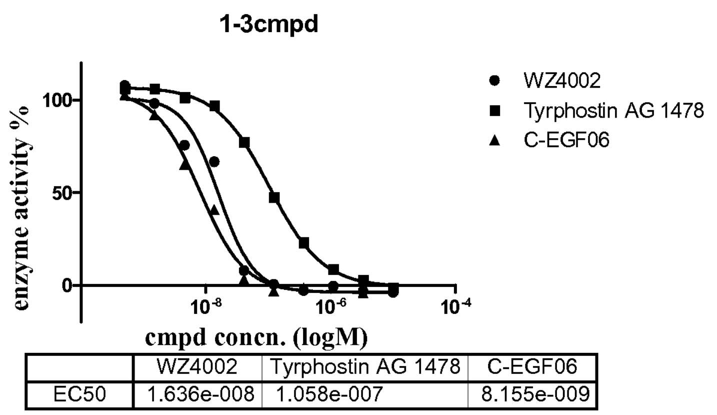


| | WZ4002 | Tyrphostin AG 1478 | C-EGF06 |
|---|---|---|---|
| EC50 | 1.636e-008 | 1.058e-007 | 8.155e-009 |

Fig. 12

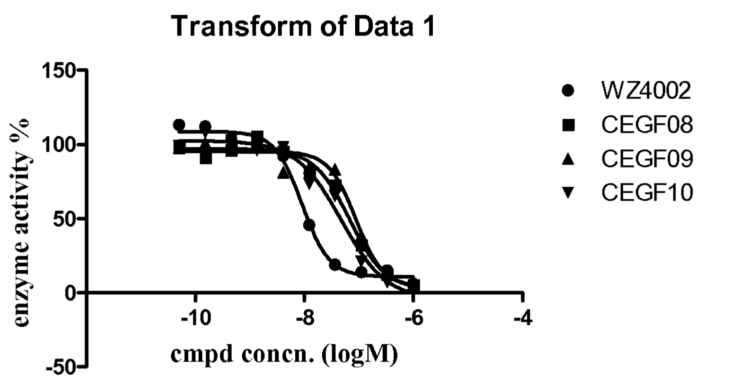


| | WZ4002 | CEGF08 | CEGF09 | CEGF10 |
|---|---|---|---|---|
| EC50 | 9.090e-009 | 6.792e-008 | 8.842e-008 | 4.802e-008 |

Fig. 13

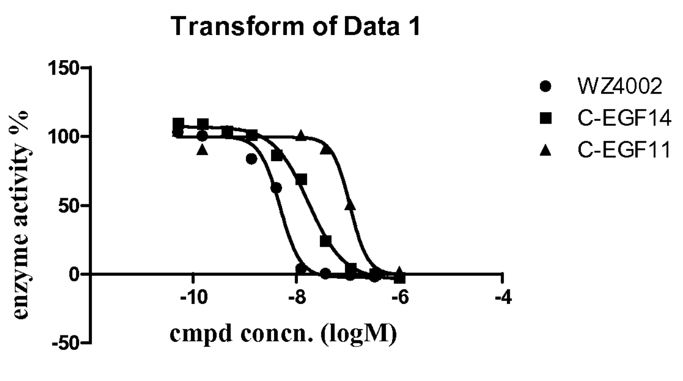


| | WZ4002 | C-EGF14 | C-EGF11 |
|---|---|---|---|
| EC50 | 4.880e-009 | 1.744e-008 | 1.110e-007 |

Fig. 14

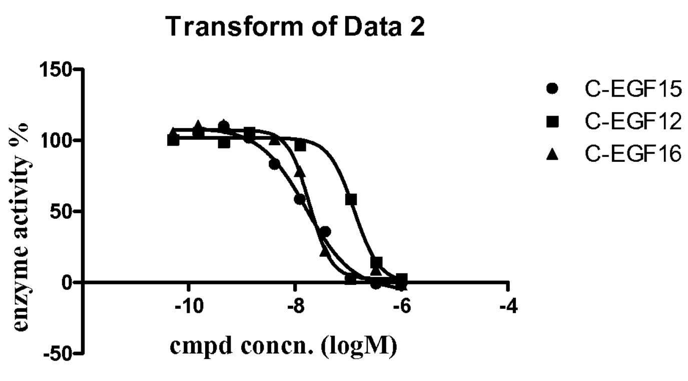


| | C-EGF15 | C-EGF12 | C-EGF16 |
|---|---|---|---|
| EC50 | 1.643e-008 | 1.301e-007 | 1.886e-008 |

Fig. 15

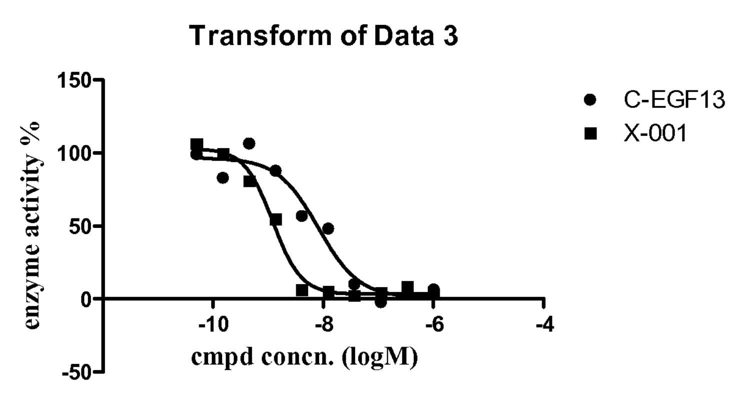


| | C-EGF13 | X-001 |
|---|---|---|
| EC50 | 8.133e-009 | 1.210e-009 |

Fig. 16

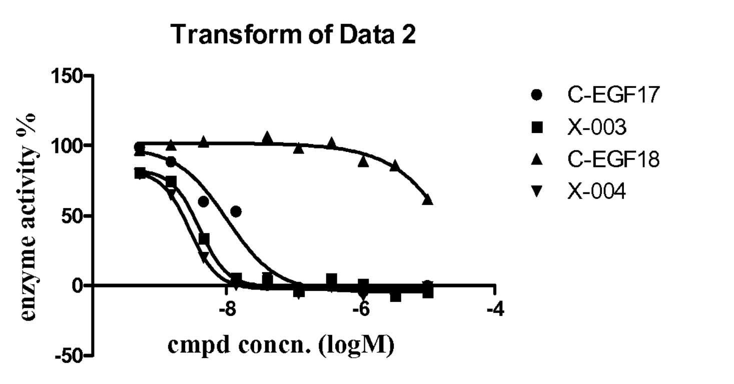


| | C-EGF17 | X-003 | C-EGF18 | X-004 |
|---|---|---|---|---|
| EC50 | 1.028e-008 | 3.982e-009 | ~ 0.0001160 | 2.839e-009 |

Fig. 17

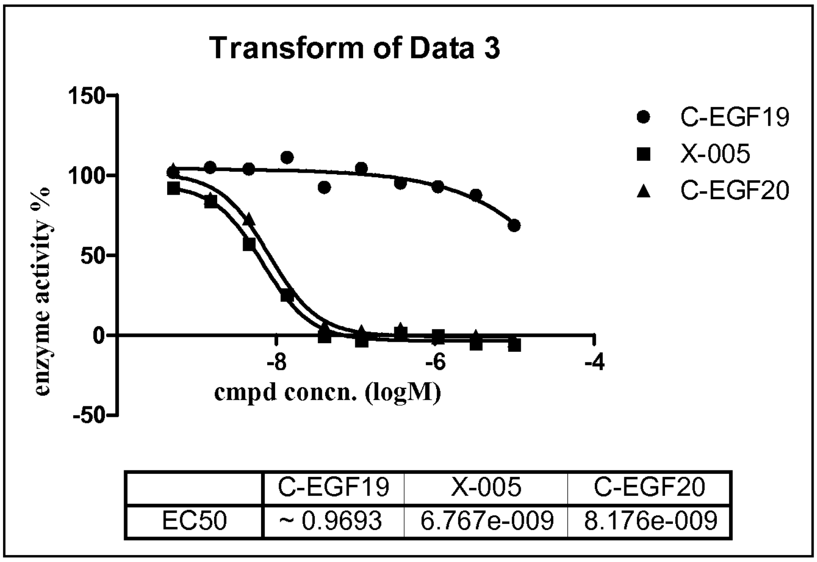


| | C-EGF19 | X-005 | C-EGF20 |
|---|---|---|---|
| EC50 | ~ 0.9693 | 6.767e-009 | 8.176e-009 |

Fig. 18

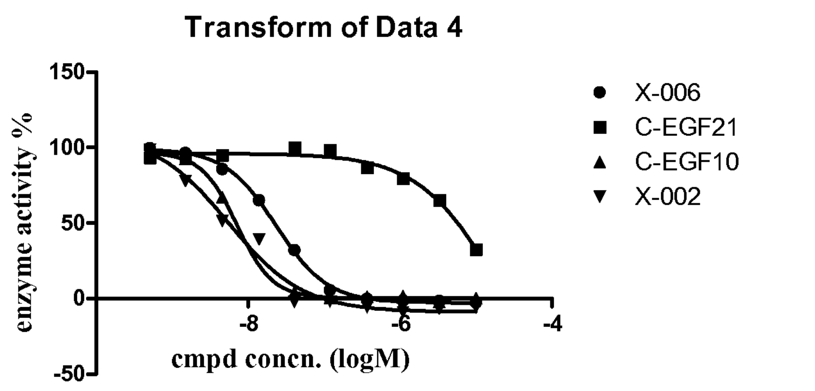


| | X-006 | C-EGF21 | C-EGF10 | X-002 |
|---|---|---|---|---|
| EC50 | 2.342e-008 | 1.934e-005 | 7.174e-009 | 5.503e-009 |

Fig. 19

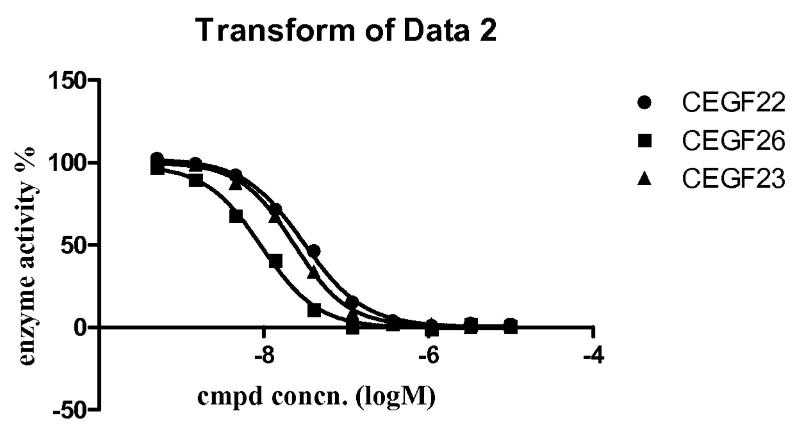


| | CEGF22 | CEGF26 | CEGF23 |
|---|---|---|---|
| EC50 | 3.082e-008 | 9.490e-009 | 2.355e-008 |

Fig. 20

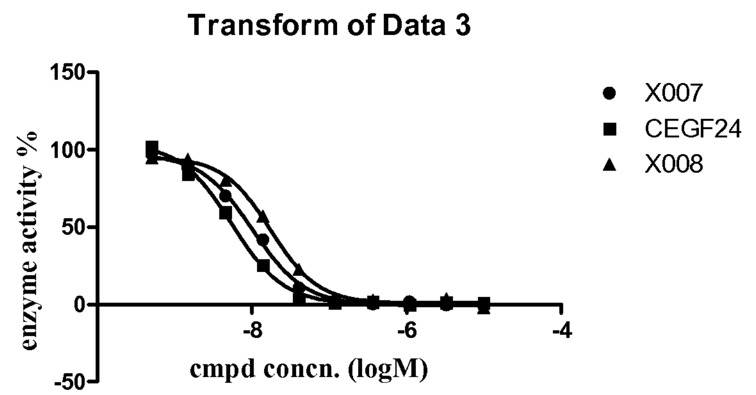


| | X007 | CEGF24 | X008 |
|---|---|---|---|
| EC50 | 1.025e-008 | 5.486e-009 | 1.759e-008 |

Fig. 21

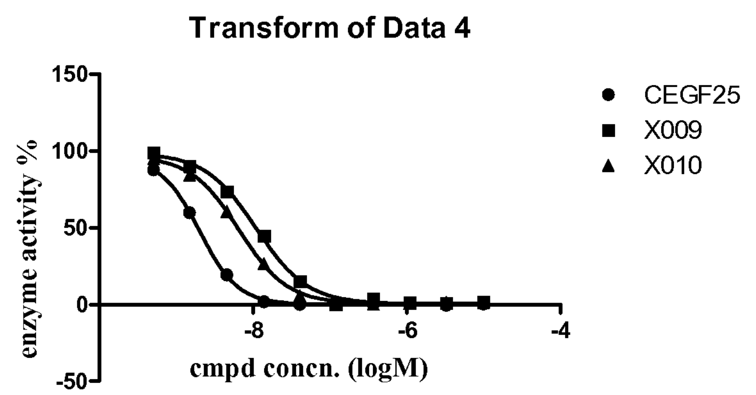


| | CEGF25 | X009 | X010 |
|---|---|---|---|
| EC50 | 2.040e-009 | 1.108e-008 | 6.613e-009 |

Fig. 22

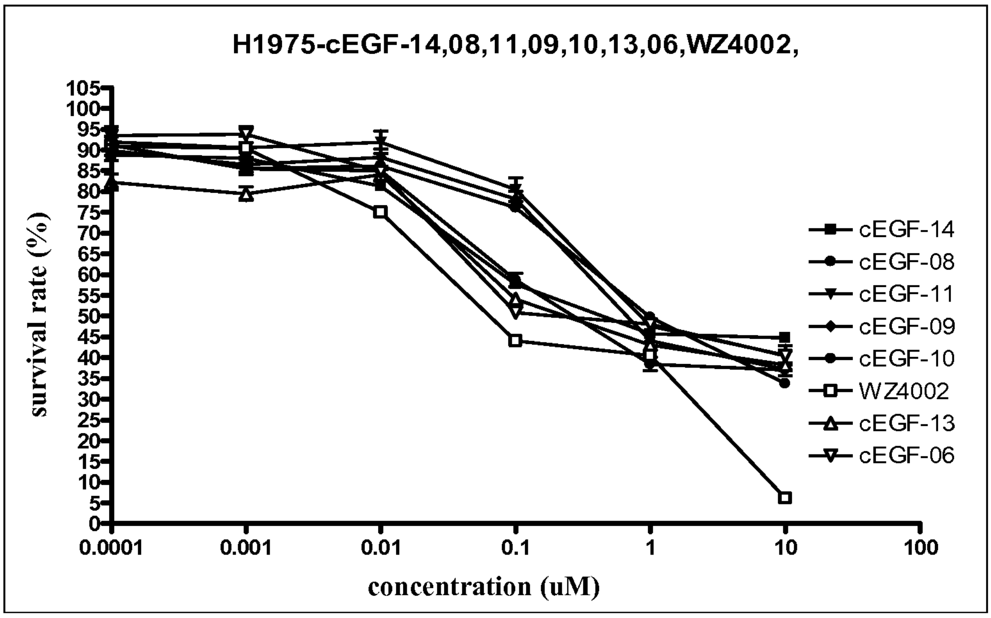
H1975-cEGF-14,08,11,09,10,13,06,WZ4002,
survival rate (%)
concentration (uM)
0.0001
0.001
0.01
0.1
1
10
100
105
100
95
90
85
80
75
70
65
60
55
50
45
40
35
30
25
20
15
10
5
0
cEGF-14
cEGF-08
cEGF-11
cEGF-09
cEGF-10
WZ4002
cEGF-13
cEGF-06

Fig. 23

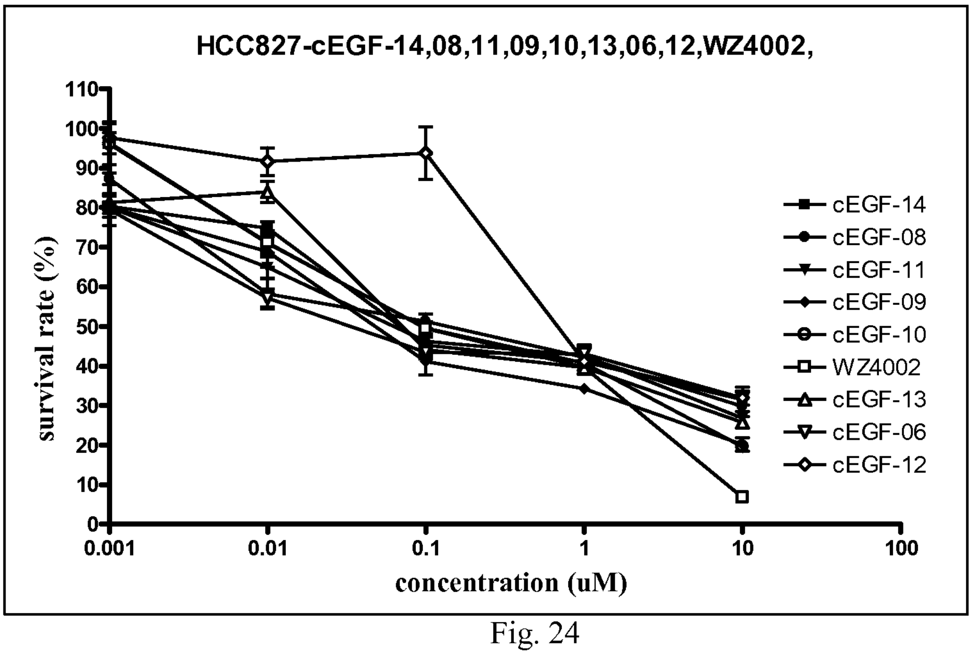
HCC827-cEGF-14,08,11,09,10,13,06,12,WZ4002,
survival rate (%)
concentration (uM)
0.001
0.01
0.1
1
10
100
110
100
90
80
70
60
50
40
30
20
10
0
cEGF-14
cEGF-08
cEGF-11
cEGF-09
cEGF-10
WZ4002
cEGF-13
cEGF-06
cEGF-12

Fig. 24

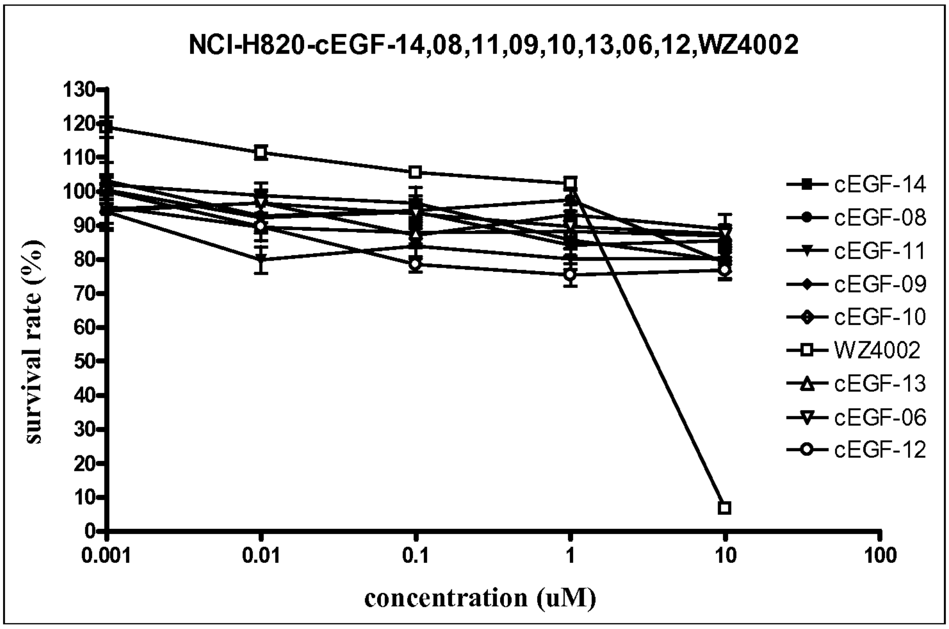
NCI-H820-cEGF-14,08,11,09,10,13,06,12,WZ4002
survival rate (%)
concentration (uM)
0.001
0.01
0.1
1
10
100
cEGF-14
cEGF-08
cEGF-11
cEGF-09
cEGF-10
WZ4002
cEGF-13
cEGF-06
cEGF-12

Fig. 25

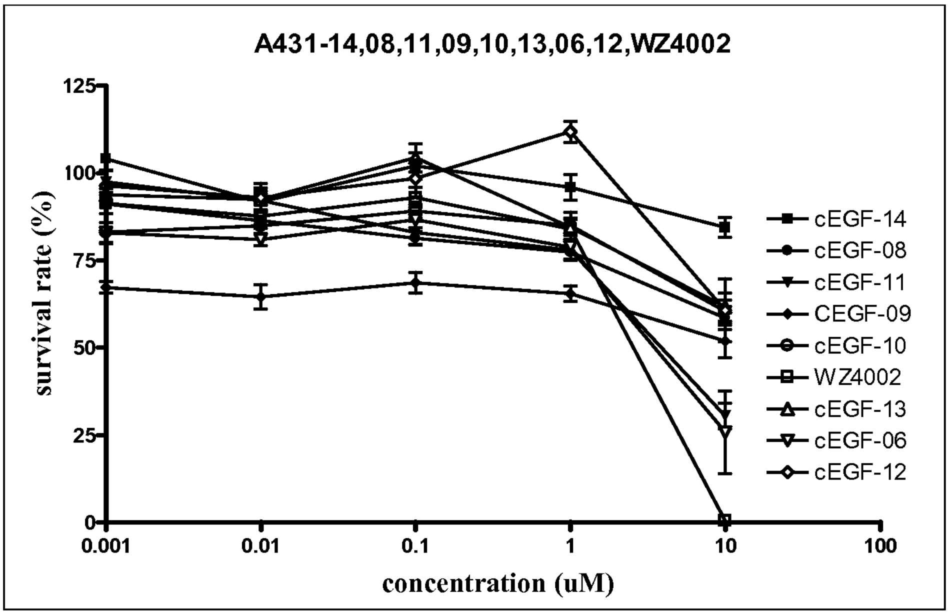
A431-14,08,11,09,10,13,06,12,WZ4002
survival rate (%)
concentration (uM)
0.001
0.01
0.1
1
10
100
cEGF-14
cEGF-08
cEGF-11
CEGF-09
cEGF-10
WZ4002
cEGF-13
cEGF-06
cEGF-12

Fig. 26

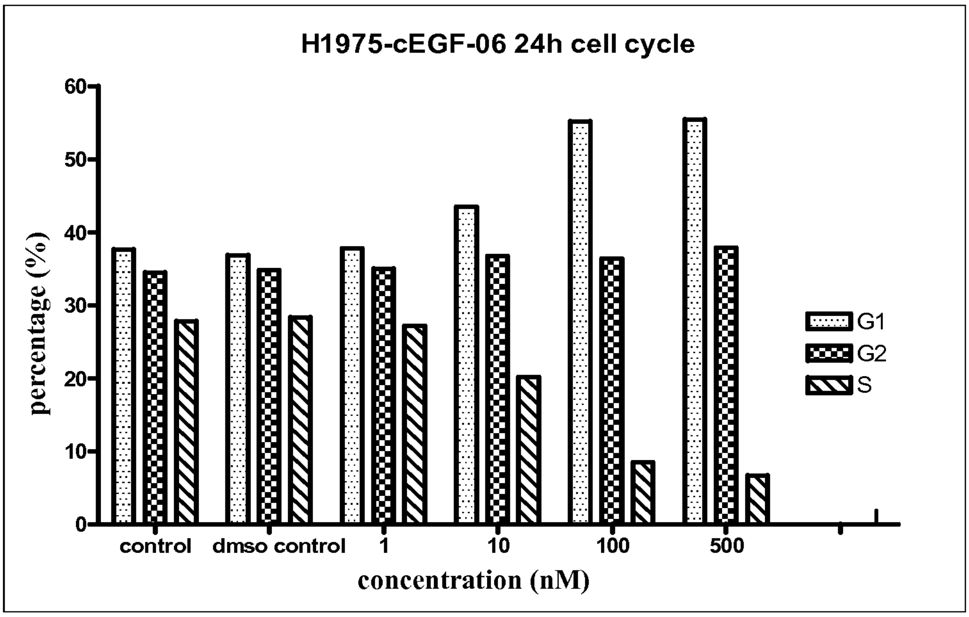


Fig. 27

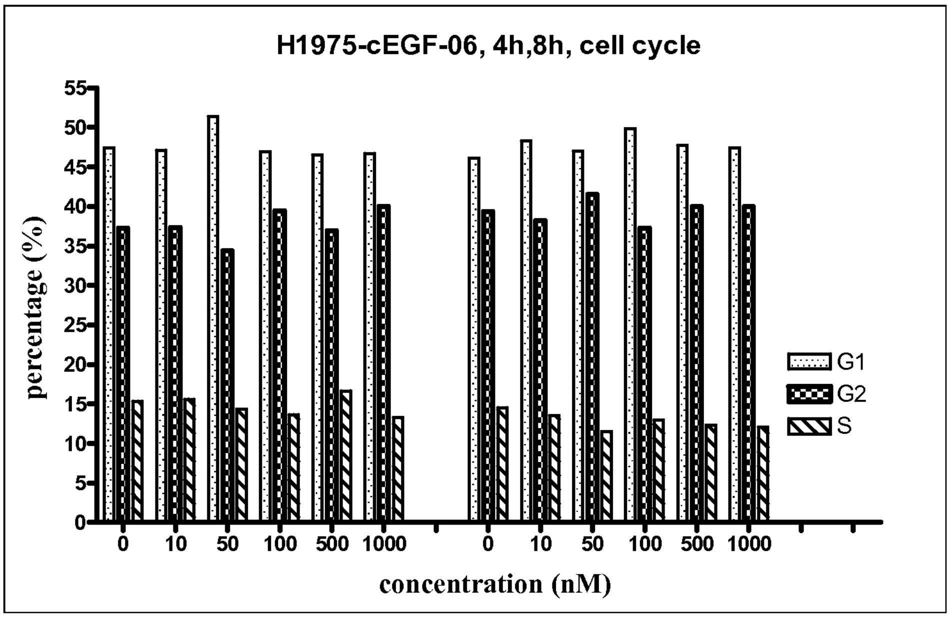


Fig. 28-1

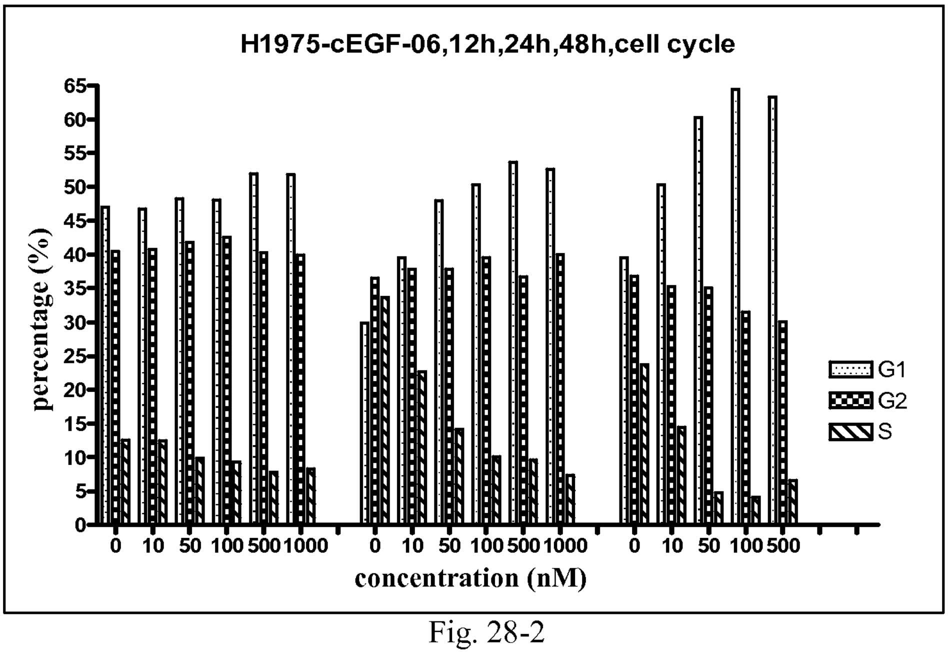


Fig. 28-2

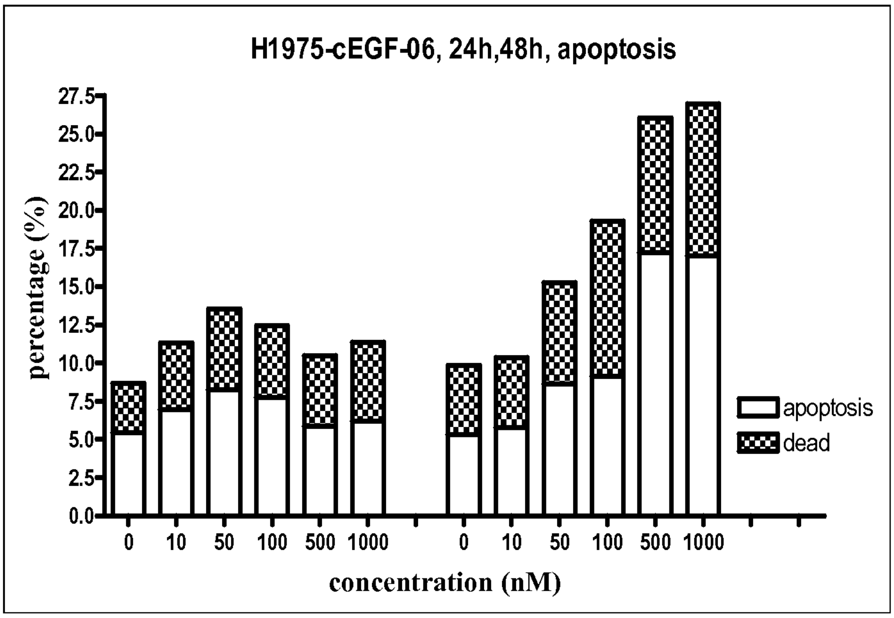


Fig. 29-1

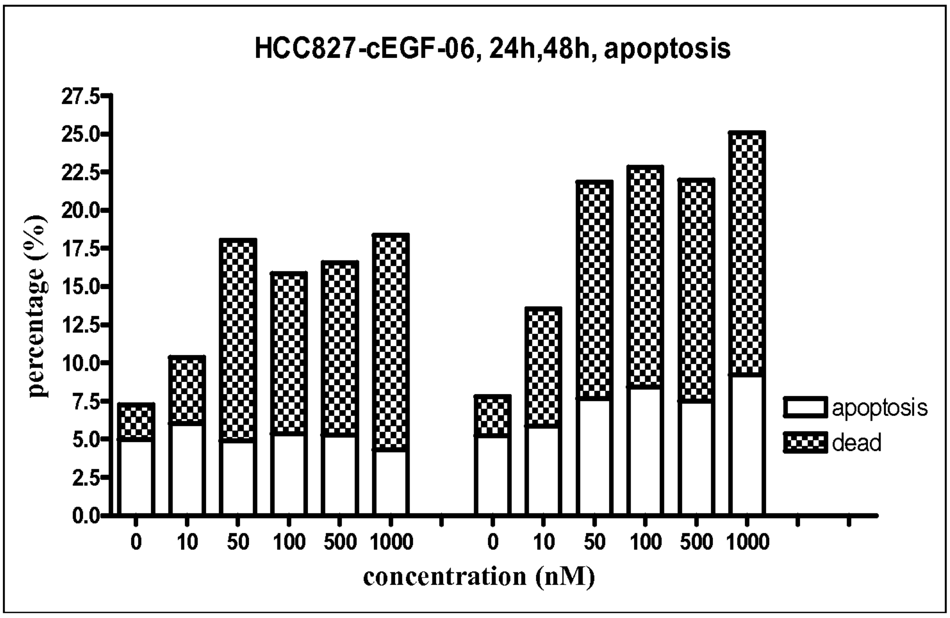


Fig. 29-2

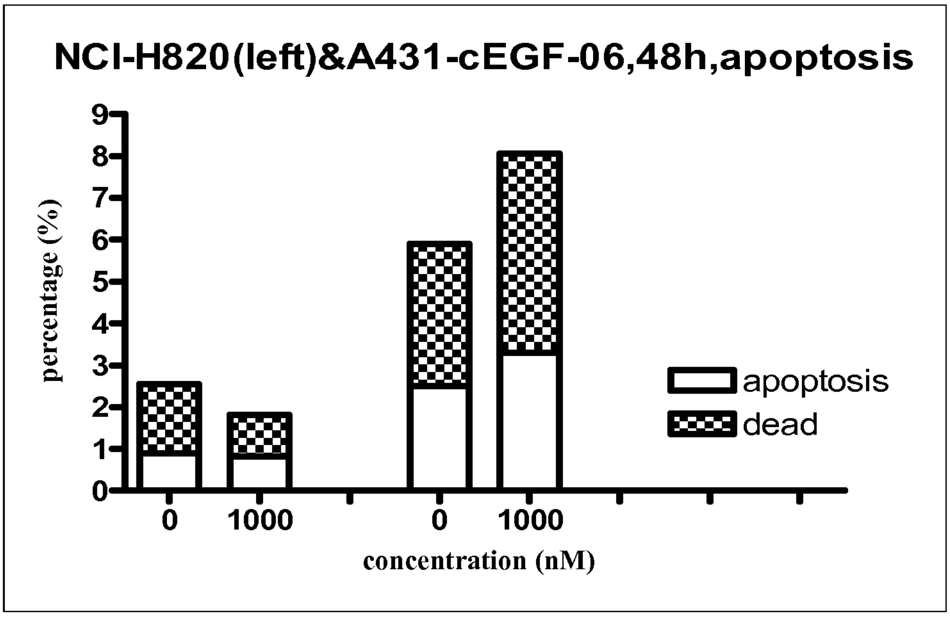


Fig. 29-3

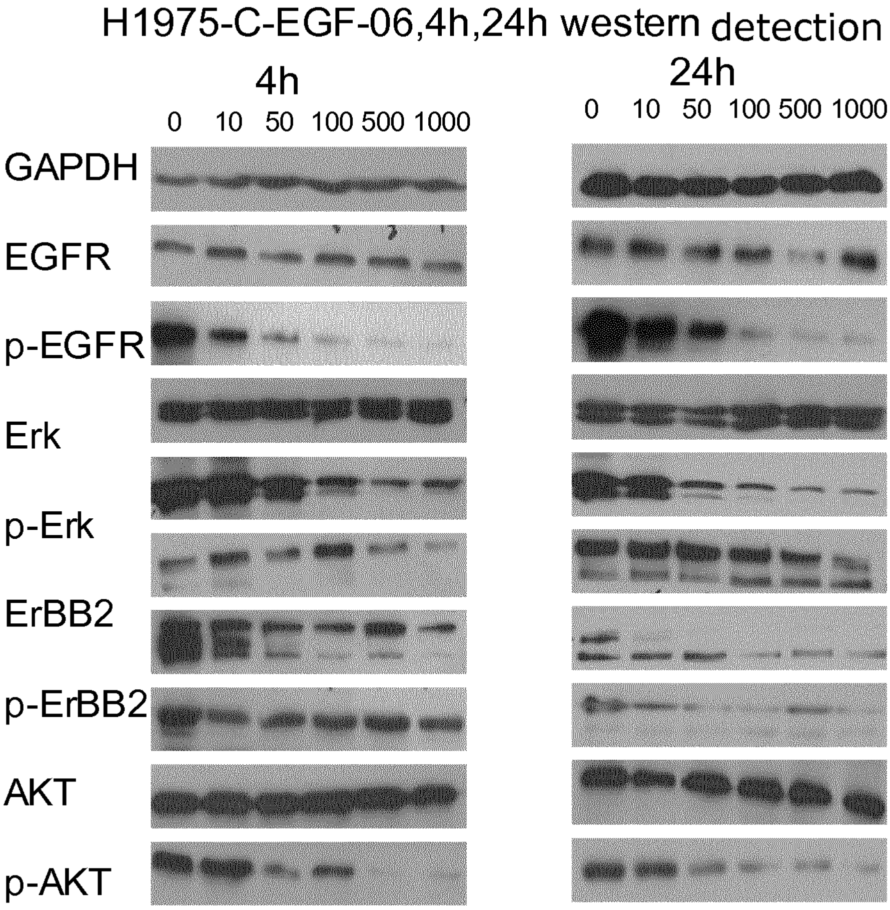
H1975-C-EGF-06,4h,24h western detection
4h
24h
0 10 50 100 500 1000
0 10 50 100 500 1000
GAPDH
EGFR
p-EGFR
Erk
p-Erk
ErBB2
p-ErBB2
AKT
p-AKT

Fig. 30

# INTERNATIONAL SEARCH REPORT

International application No.
PCT/CN2011/002152

**A. CLASSIFICATION OF SUBJECT MATTER**

See the extra sheet

According to International Patent Classification (IPC) or to both national classification and IPC

B. FIELDS SEARCHED

Minimum documentation searched (classification system followed by classification symbols)

IPC：C07D487/-

Documentation searched other than minimum documentation to the extent that such documents are included in the fields searched

Electronic data base consulted during the international search (name of data base and, where practicable, search terms used)

WPI, EPODOC, CNABS, CNKI, REGISTRY, CAPLUS, pyrimidine, pyrimidone, pyridine, EGFR, cancer, tumour, tumor, substructure search according to formulae (I) and (II)

C. DOCUMENTS CONSIDERED TO BE RELEVANT

| Category* | Citation of document, with indication, where appropriate, of the relevant passages | Relevant to claim No. |
|---|---|---|
| X | EP0163599A2(NIPPON ZOKI PHARMACEUTICAL CO.LTD.), 04 Dec. 1985(04.12.1985), see claims, page 1, lines 5-10 and page 4, line 19, the compound | 1，5 |
| A | | 2-4，6-7 |
| X | Kosaku HIROTA et al., Novel ring transformations of 5-cyanouracils into 2-thiocytosines, 2,4-diaminopyrimidines, and pyrimido[4,5-d]pyrimidines by the reaction with thioureas and guanidines, Journal of the Chemical Society, Perkin Transactions 1, January 1990，No.1, pages 123-128, see abstract and formulae 9a, 9b of page 125 | 1 |
| A | | 2-7 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 09 Mar. 2012(09.03.2012) | 29 Mar. 2012(29.03.2012) |

Name and mailing address of the ISA
State Intellectual Property Office of the P. R. China
No. 6, Xitucheng Road, Jimenqiao
Haidian District, Beijing 100088, China
Facsimile No. (86-10)62019451

Authorized officer
WANG, Shaohua
Telephone No. (86-10) **62086353**

Form PCT/ISA /210 (second sheet) (July 2009)

# INTERNATIONAL SEARCH REPORT

International application No.
PCT/CN2011/002152

C (Continuation). DOCUMENTS CONSIDERED TO BE RELEVANT

| Category* | Citation of document, with indication, where appropriate, of the relevant passages | Relevant to claim No. |
|---|---|---|
| A | WO01/64679A1(SMITHKLINE BEECHAM CORPORATION) , 07 Sep. 2001 （07.09.2001）, see the whole document | 1-7 |

**INTERNATIONAL SEARCH REPORT**
Information on patent family members

International application No.
PCT/CN2011/002152

| Patent Documents referred in the Report | Publication Date | Patent Family | Publication Date |
|---|---|---|---|
| EP0163599A2 | 04.12.1985 | US4886807A | 12.12.1989 |
| | | EP0163599B1 | 21.03.1990 |
| | | DE3576690D1 | 26.04.1990 |
| | | JP60226882A | 12.11.1985 |
| | | JP5059118B | 30.08.1993 |
| WO0164679A1 | 07.09.2001 | CZ20022933A3 | 12.02.2003 |
| | | KR20020082858A | 31.10.2002 |
| | | AU2001239992B2 | 01.09.2005 |
| | | EP1265900B1 | 16.05.2007 |
| | | DE60128457T2 | 17.01.2008 |
| | | NO326409B1 | 01.12.2008 |
| | | NO20024134A | 24.10.2002 |
| | | IN200201125P3 | 04.03.2005 |
| | | KR100806978B1 | 25.02.2008 |
| | | US7700768B2 | 20.04.2010 |
| | | JP2003525295A | 26.08.2003 |
| | | MX2002008588A1 | 01.02.2003 |
| | | NZ520914A | 26.03.2004 |
| | | EP1842851A2 | 10.10.2007 |
| | | MX247976B | 10.08.2007 |
| | | IL151426A | 31.01.2011 |
| | | EP1265900A1 | 18.12.2002 |
| | | CN1422273A | 04.06.2003 |
| | | BR0108715A | 27.04.2004 |
| | | US7235551B2 | 26.06.2007 |
| | | TWI290926B | 11.12.2007 |
| | | IN206224B | 13.07.2007 |
| | | CN100482664C | 29.04.2009 |
| | | HU200204431A2 | 28.04.2003 |
| | | ZA200207017A | 30.06.2004 |
| | | US2003100756A1 | 29.05.2003 |
| | | US2007167467A1 | 19.07.2007 |
| | | EP1842851A3 | 27.02.2008 |
| | | US2009048442A1 | 19.02.2009 |

Form PCT/ISA /210 (patent family annex) (July 2009)

**INTERNATIONAL SEARCH REPORT**
Information on patent family members

International application No.
PCT/CN2011/002152

| Patent Documents referred in the Report | Publication Date | Patent Family | Publication Date |
|---|---|---|---|
| | | AU3999201A | 12.09.2001 |
| | | DE60128457D1 | 28.06.2007 |
| | | US2007238743A1 | 11.10.2007 |
| | | US7629462B2 | 08.12.2009 |
| | | MX278732B | 06.09.2010 |
| | | MY141144A | 15.03.2010 |
| | | PH1200100470B1 | 19.11.2010 |
| | | ES2287107T3 | 16.12.2007 |
| | | CA2402092C | 24.01.2012 |

Form PCT/ISA /210 (patent family annex) (July 2009)

## INTERNATIONAL SEARCH REPORT

International application No.
PCT/CN2011/002152

**A. CLASSIFICATION OF SUBJECT MATTER**

According to International Patent Classification (IPC) or to both national classification and IPC

C07D 487/04(2006.01)i
A61K 31/519(2006.01) i
A61K 31/5377(2006.01) i
A61K 31/551(2006.01) i
A61K 31/541(2006.01) i
A61P 35/00(2006.01) i
A61P 35/02(2006.01) i

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Pharmaceutical Salts, J. Pharm. Sci.,* 1977, vol. 66, 1-19